(19) **Europäisches Patentamt**
**European Patent Office**
**Office européen des brevets**

(11) **EP 1 776 057 B1**

(12) **EUROPEAN PATENT SPECIFICATION**

(45) Date of publication and mention
of the grant of the patent:
**18.11.2009 Bulletin 2009/47**

(51) Int Cl.:
*A61B 19/00* *(2006.01)*    *A61M 25/01* *(2006.01)*

(21) Application number: **05786497.7**

(22) Date of filing: **12.08.2005**

(86) International application number:
**PCT/US2005/028877**

(87) International publication number:
**WO 2006/020943 (23.02.2006 Gazette 2006/08)**

(54) **ROBOTICALLY CONTROLLED INTRAVASCULAR TISSUE INJECTION SYSTEM**

ROBOTERGESTEUERTES INTRAVASKULÄRES GEWEBEINJEKTIONSSYSTEM

SYSTEME D'INJECTION INTRAVASCULAIRE DANS UN TISSU COMMANDE PAR ROBOT

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.08.2004  US 600869 P**
**13.01.2005  US 644505 P**

(43) Date of publication of application:
**25.04.2007  Bulletin 2007/17**

(73) Proprietor: **Hansen Medical, Inc.**
**Mountain View, California 94043-5207 (US)**

(72) Inventors:
• **MOLL, Frederic, H.**
**Woodside, CA 94062 (US)**
• **HLAVKA, Edwin, J.**
**Palo Alto, CA 94306 (US)**

(74) Representative: **Dorschner, David et al**
**Dennemeyer & Associates S.A.**
**55, rue des Bruyères**
**1274 Howald (LU)**

(56) References cited:
**WO-A-02/065933      US-A- 5 389 076**
**US-A- 6 123 665      US-A1- 2002 087 169**
**US-B1- 6 673 041**

Printed by Jouve, 75001 PARIS (FR)

## Description

WO02/065933 discloses a remotely controllable surgical instrument comprising an instrument shaft having an elongated disposable shaft removably interconnected to the drive unit via a coupling mechanism and a distal instrument drivable via cables.

FIELD OF INVENTION

The invention relates generally to robotically controlled systems, such as telerobotic surgical systems, and more particularly to a robotic catheter system for performing minimally invasive diagnostic and therapeutic procedures.

BACKGROUND

Robotic surgical systems and devices are well suited for use in performing minimally invasive medical procedures, as opposed to conventional techniques wherein the patient's body cavity is open to permit the surgeon's hands access to internal organs. For example, there is a need for a highly controllable yet minimally sized system to facilitate imaging, diagnosis, and treatment of tissues which may lie deep within a patient, and which may be preferably accessed only via naturally-occurring pathways such as blood vessels or the gastrointestinal tract.

DETAILED DESCRIPTION OF THE ILLUSTRATED EMBODIMENTS

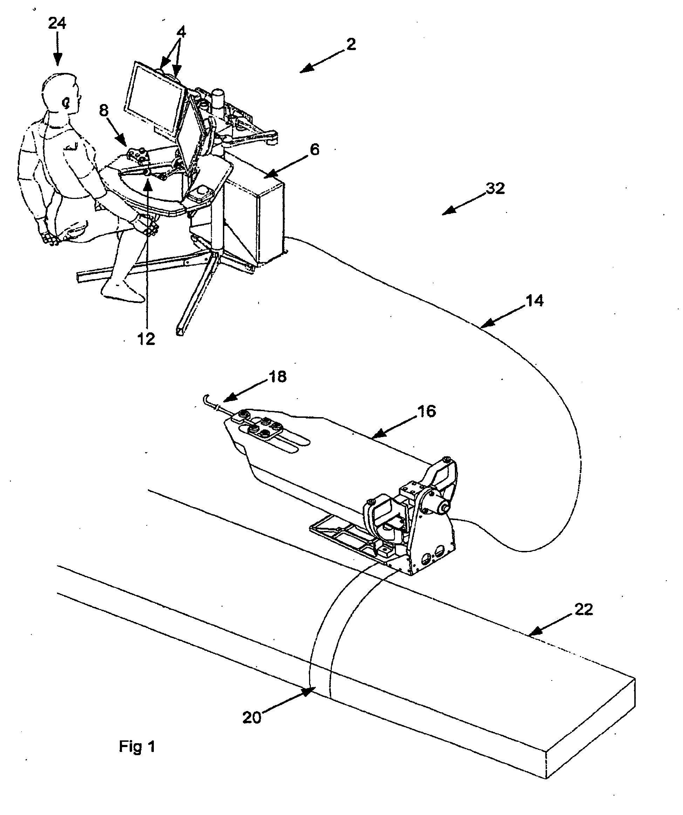

Referring to Fig. 1, one embodiment of a robotic catheter system 32, includes an operator control station 2 located remotely from an operating table 22, to which a instrument driver 16 and instrument 18 are coupled by a instrument driver mounting brace 20. A communication link 14 transfers signals between the operator control station 2 and instrument driver 16. The instrument driver mounting brace 20 of the depicted embodiment is a relatively simple, arcuate-shaped structural member configured to position the instrument driver 16 above a patient (not shown) lying on the table 22.

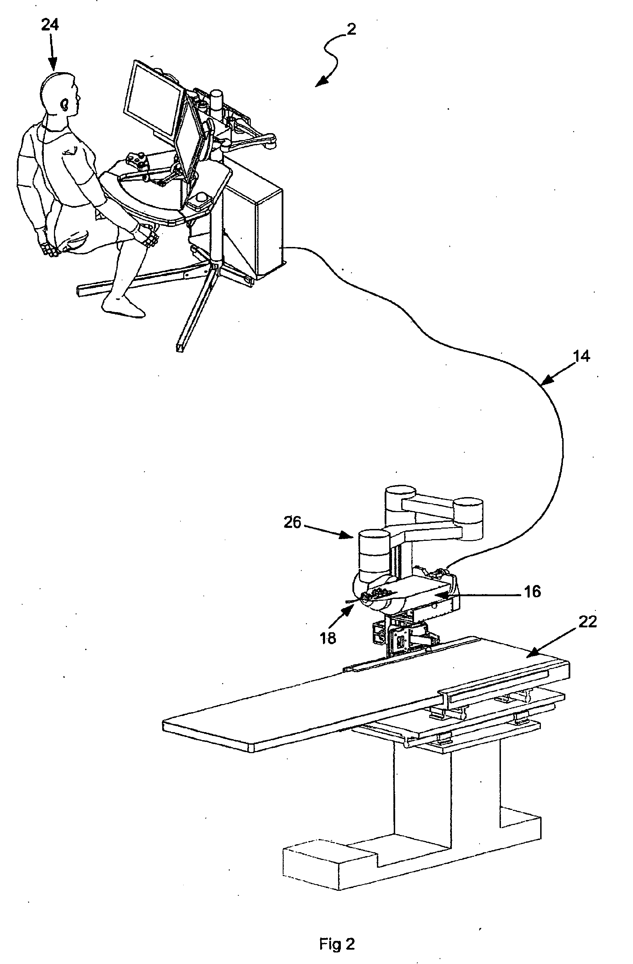

In Fig. 2, another embodiment of a robotic catheter system is depicted, wherein the arcuate-shaped member 20 is replaced by a movable support-arm assembly 26. The support assembly 26 is configured to movably support the instrument driver 16 above the operating table 22 in order to position the instrument driver 16 for convenient access into desired locations relative to a patient (not shown). The support assembly 26 in Fig. 2 is also contigured to lock the instrument driver 16 into position.

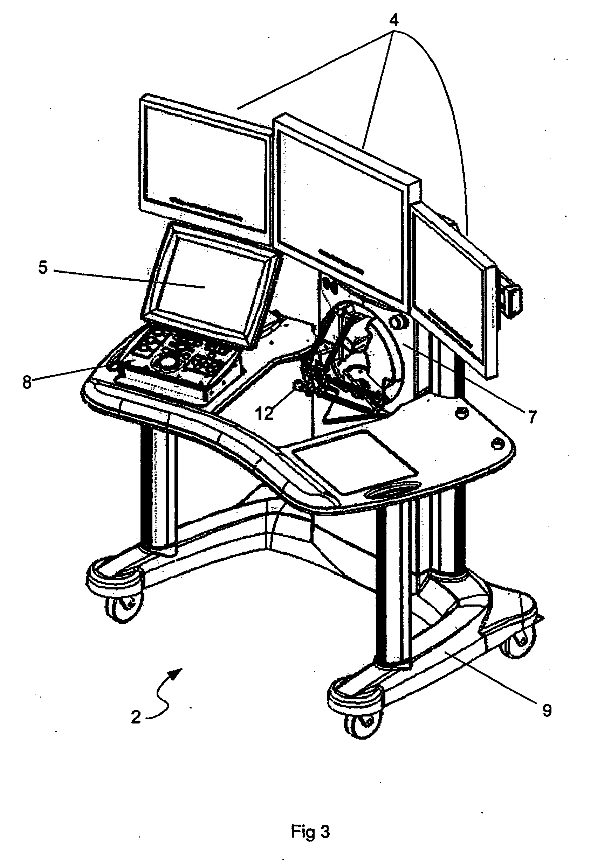

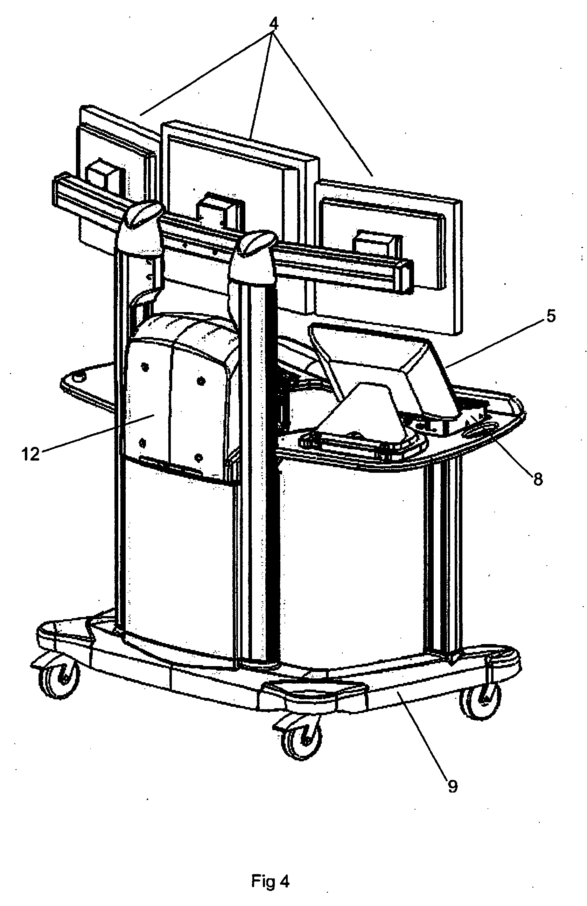

Referring to Figure 3, a view of another variation of an operator control station (2) is depicted having three displays (4), a touchscreen user interface (5), and a control button console (8). The master input device (12) depicted in the embodiment of Figure 3 is depicted and described in further detail in reference to Figure 105B. Also depicted in the embodiment of Figure 3 is a device disabling switch (7) configured to disable activity of the instrument temporarily. The cart (9) depicted in Figure 3 is configured for easy movability within the operating room or catheter lab, one advantage of which is location of the operator control station (2) away from radiation sources, thereby decreasing radiation dosage to the operator. Figure 4 depicts a reverse view of the embodiment depicted in Figure 3.

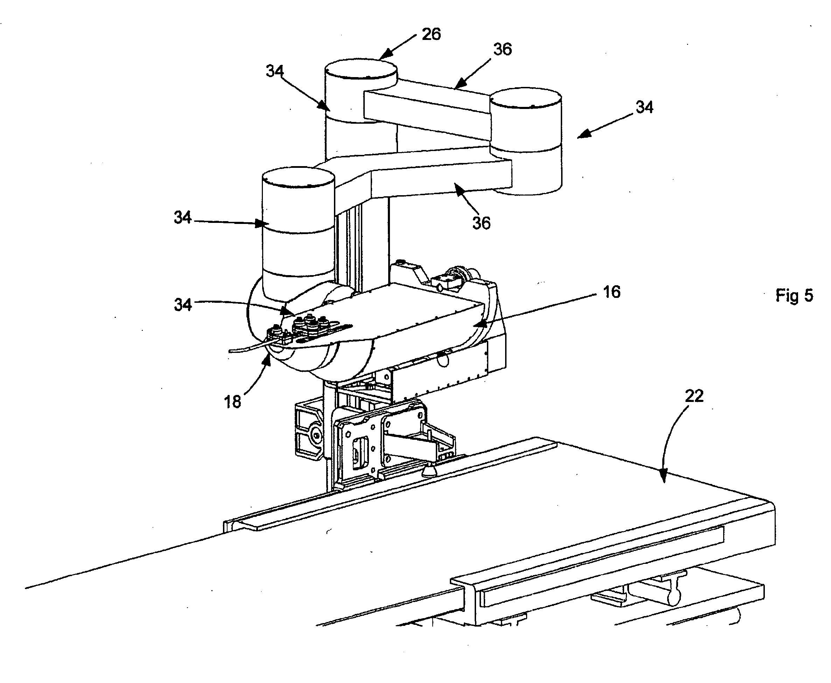

Figure 5 provides a closer view of the support assembly 26 depicted in the embodiments of Figure 2. The support assembly 26 comprises a series of rigid links 36 coupled by electronically braked joints 34. The joints 34 allow motion of the links 36 when energized by a control system (not shown), but otherwise prevent motion of the links. The control system may be activated by a switch (e.g., a footswitch or thumbswitch), or computer interface. In another embodiment, the rigid links 36 may be coupled by mechanically lockable joints, which may be locked and unlocked manually using, for example, locking pins, screws, or clamps. The rigid links 36 preferably comprise a light but strong material, such as high-gage aluminum, shaped to withstand the stresses and strains associated with precisely maintaining a three-dimensional position of the approximately ten pound weight of a typical embodiment of the instrument driver 16 once the position of the link 36 is fixed.

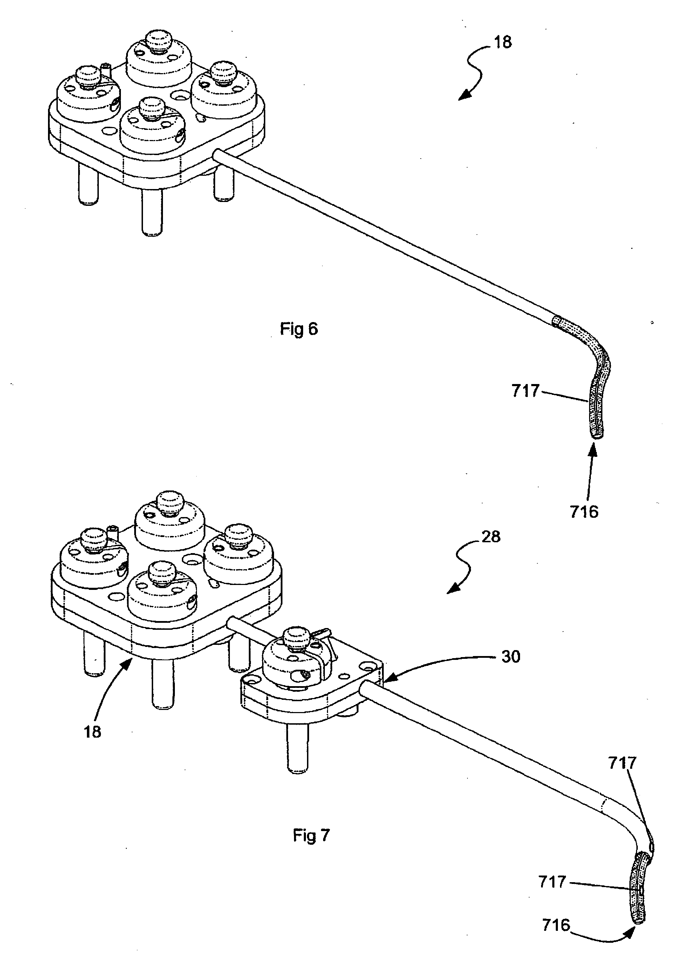

Figures 6 and 7 depict isometric views of respective embodiments of instruments configured for use with an embodiment of the instrument driver (16), such as that depicted in Figures 1-3. Figure 6 depicts an instrument (18) embodiment without an associated coaxial sheath coupled at its midsection. Figure 7 depicts a set of two instruments (28), combining an embodiment like that of Figure 6 with a coaxially coupled and independently controllable sheath instrument (30). To distinguish the non-sheath instrument (18) from the sheath instrument (30) in the context of this disclosure, the "non-sheath" instrument may also be termed the "guide" instrument (18). The guide instrument (18) defines an inner lumen (716) through which a tool, such as an ablation catheter, tissue grasper, endoscope, injection needle, or tissue-traversing needle may be deployed. Localization sensors (717), such as those available from Biosense Webster, Endocardial Solutions, Medtronic, and others, may be coupled to or integrated within the sheath instrument (30), guide instrument (18), and any coaxially-associated tools, to provide accurate three-dimensional location information to the computerized-robotic system utilized to operate the subject instruments.

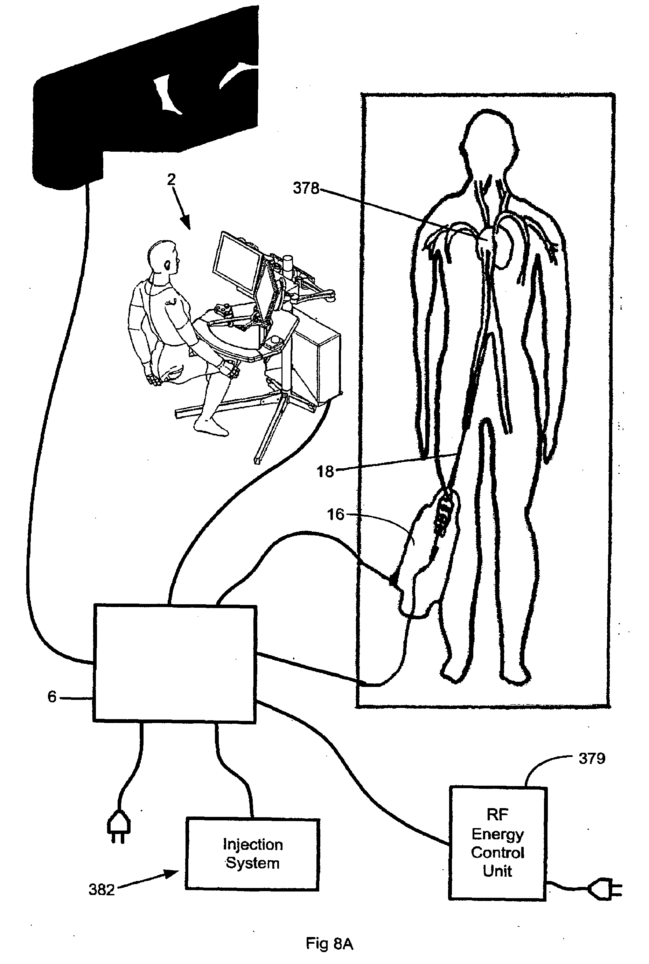

Referring to Figure 8A, a system comprising an operator control station (2), an instrument driver (16), a computer or processor (6), a display monitor (4), an elongate instrument (18) coupled to an electrode (378), and an RF energy control unit (379) is depicted. Such a system may be utilized for an embodiment wherein both RF ablation and injection may be conducted - either sequentially or simultaneously. In alternative embodiments, other ablation tools or modalities may be utilized, such as ultrasound or microwave radiation or cryo-ablation, or heated fluids such as hot saline, to facilitate creation of ablative lesions with the distal end of the elongate instrument (18).

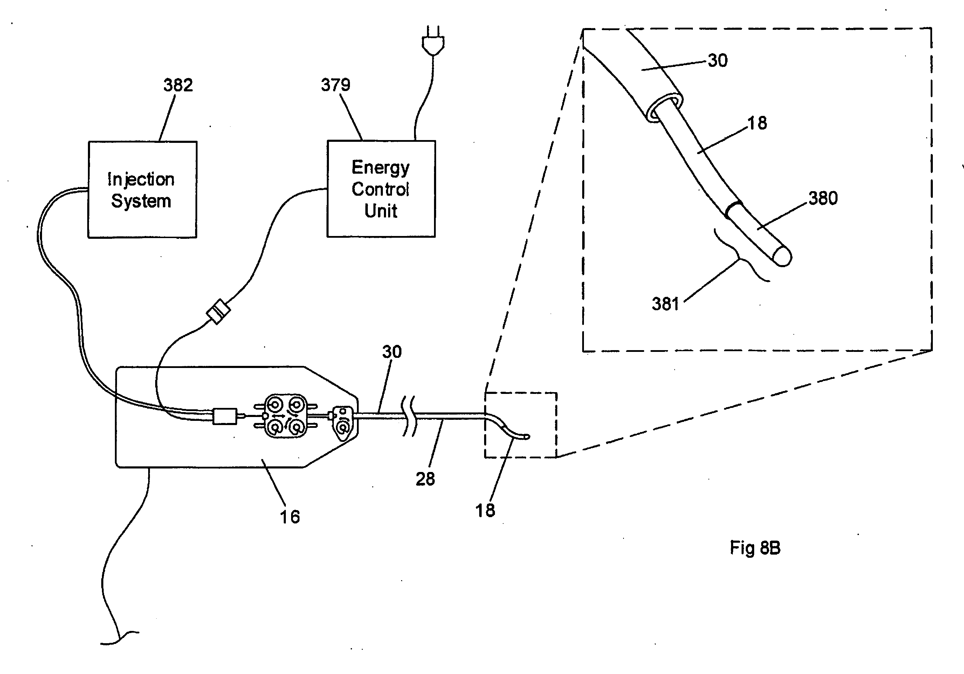

Referring to Figure 8B, a system similar to that depicted in Figure 8A is depicted comprising an instrument driver (16) interfaced to an instrument set (28) comprising coaxially-interfaced sheath (30) and guide (18) instruments. The guide instrument (18) is coaxially interfaced, through its inner lumen, with an elongate probe (380) which may comprise a heating and/or injecting tool: at its distal tip (381). In an embodiment comprising an injecting tip, an injection system (382) may be coupled to the instrument set (28) and configured to controllably deliver fluids, solutions, etc. - such as a biologically-compatible fixative formulation such as genepin, cells, such as stem cells, skeletal myoblasts, cardiac muscle cells or myoblasts, hormones, medicines, vascular endothelial growth factor, etc., through the injecting tip distally.

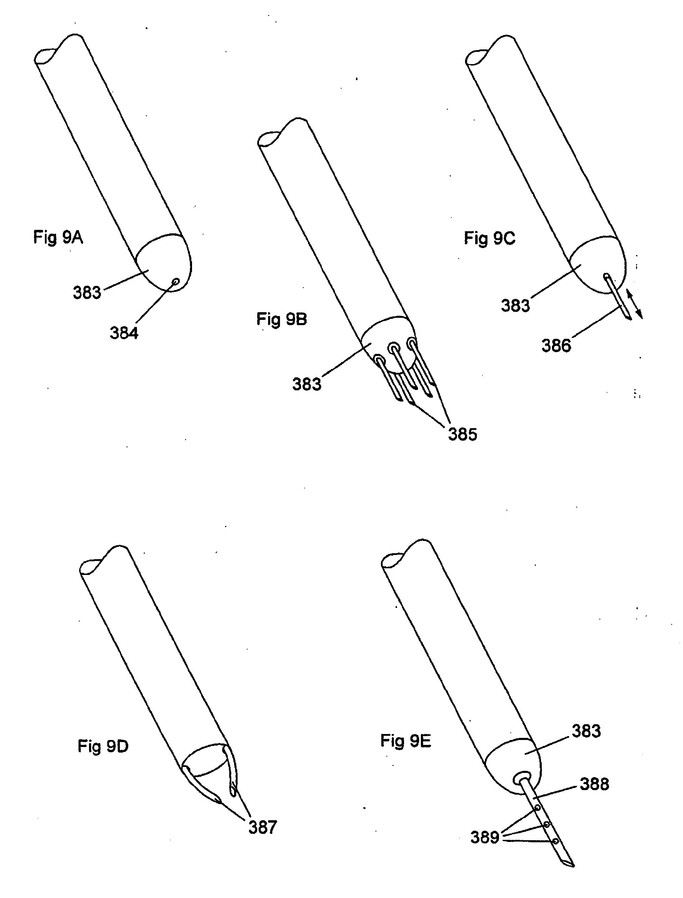

Referring to Figures 9A-H, various hybrid distal tip structures for an elongate instrument configured to both inject a chemical solution, such as a genepin solution or solution of another fixative, and also apply RF energy to induce localized denaturation are depicted. Figure 9A depicts a needle-less injection port (384) positioned-through-the center of a monopolar RF electrode (383). Figure 9B depicts a series of needle injection ports (385) located upon an RF electrode (383) for a volumic injection into a broader volume that would be practicable with a single needle.

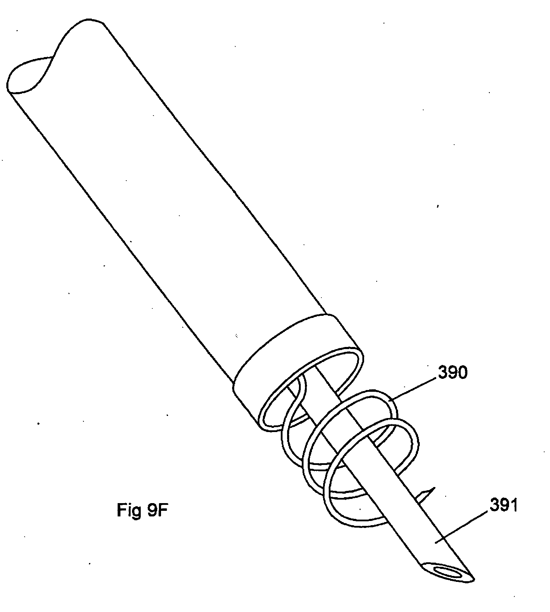

Figure 9C depicts an extensible/retractable needle (386) injection port through the center of an RF electrode (383). Figure 9D depicts bipolar electrode configuration wherein each of two distal elements (387) comprises both an electrode and an injection tip. Figure 9E depicts a single injection needle through the center of an RF electrode (383), the needle (388) comprising multiple fluid pathways (389) along its length to facilitate a distributed injection through a depth of targeted tissue. The needle (388) maybe extensible/retractable, as with each of the distal tip needle structures depicted herein. Figure 9F depicts an embodiment wherein an injection needle (391) is oriented through the center of a helical structure (390), and wherein any of the distal elements may be an RF electrode - in other words, the injection needle (391) or helical structure (390) may be a monopolar electrode, or each may be an electrode in a bipolar configuration.

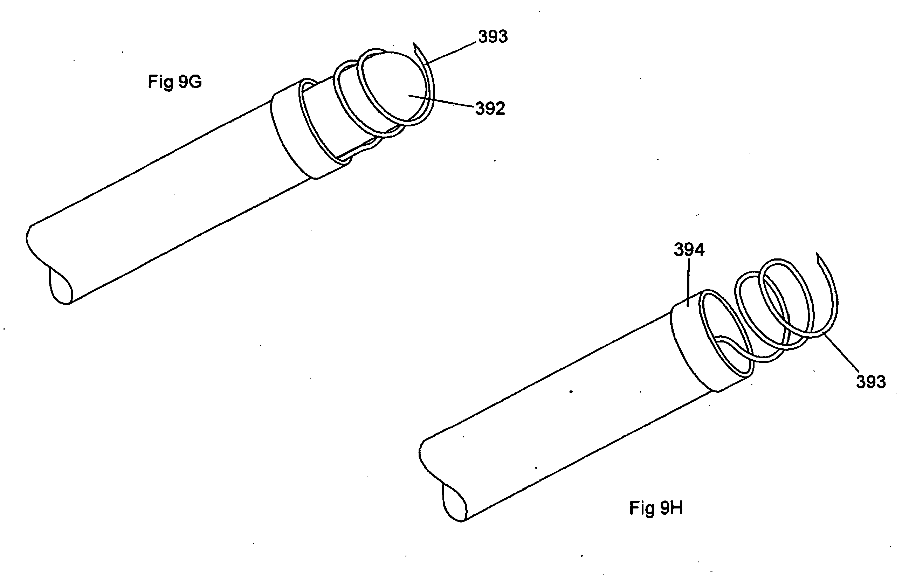

Figure 9G depicts an embodiment wherein a bullet-shaped electrode (392) is positioned through at least a portion of a helical injection needle (393). Figure 9H depicts an embodiment similar to that of Figure 9G with the exception that a distal ring (394) comprises the electrode as opposed to the bullet-shaped electrode of Figure 9G. The helical injection needles of the embodiments depicted in Figures 9G and 9H may have side ports (not shown) as depicted in the embodiment of Figure 9E, and may comprise an electrode form a bipolar electrode configuration in association with the bullet-shaped electrode (392) or ring electrode (394). In other variations configured for injection without RF energy delivery, geometries such as those depicted in Figures 9A-H may be utilized, absent the RF electrode hardware at the distal tip. Conventional injection needle tips may also be utilized for precision injection without RF delivery through the working lumen of the subject robotic catheter instruments.

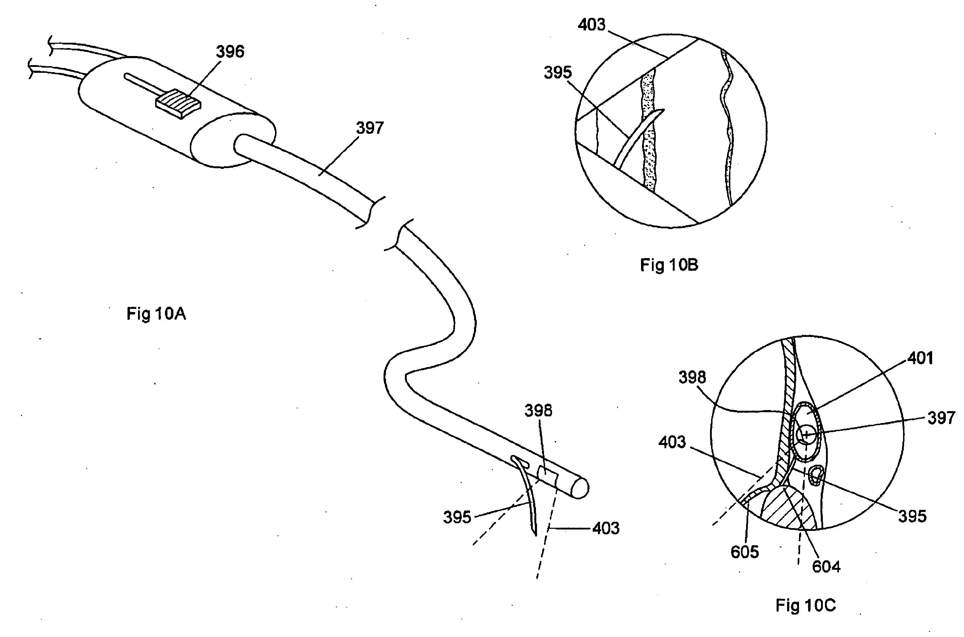

Referring to Figures 10A-C, a retractable injection needle (395) may be retractably extended from the side of an elongate probe (397) to provide access to tissue structures located to the periphery of a given probe orientation, such as the mitral annulus as oriented from the coronary sinus, as depicted in Figure 10C. The injection needle (395) may be advanced and/or retracted utilizing a simple proximal mechanical lever (396), as depicted in Figure 10A, or may be associated with an electromechanical configuration for precisely actuating advancement and/or retraction.

To facilitate accurate positioning of a side-extending injector, or injector which also comprises an electrode in another embodiment, an imaging device (398), such as an ultrasound array, CCD device, or more conventional optical camera, in one embodiment comprising a mirror for side-oriented field of view (403), may be coupled to the probe (397) to provide a field of view (403) configured to capture images of at least a portion of the needle or needle/electrode as it is advanced out of the probe (397), as depicted in Figure 10B. Referring to Figure 10C, a partial cross sectional view of a system such as that depicted in Figures 10A and 10B is depicted with the probe (397) threaded down a coronary sinus (401) of a human heart, and an injection needle (395), in this embodiment also comprising an electrode, directed out of the coronary sinus (401) lumen and into the collagenous mitral valve annulus (604). The field of view (403) of the imaging device (398), in the depicted embodiment comprising an ultrasound transducer, is oriented to capture images of at least a portion of the needle (395), and preferably portions of surrounding identifiable tissues, such as the mitral annulus (604) or mitral valve leaflet (605).

Utilizing instruments such as those depicted in Figures 8-10, precision injection therapy may be conducted. For example, in one embodiment, a system such as that depicted in Figures 8A-B, absent the RF electrode and energy system, may be utilized to navigate an injection tip from the inferior vena cava or superior vena cava into the right atrium of the heart where a precision injection may be conducted. Crossing the tricuspid valve, a precision injection may be conducted in the right ventricle. Alternatively, a robotically-operated guide instrument may be navigated to the left heart via a trans-septal puncture, or via a retrograde approach through the aorta and into the left ventricle. Precision robotic control of the guide and sheath instruments facilitates precision placement of injection tips to controllably deliver fluids, solutions, etc - such as a biologically-compatible fixative formulation such as genepin, cells, such as stem cells, skeletal myoblasts, cardiac muscle cells or myoblasts, hormones, medicines, vascular.endothelial growth factor, etc.

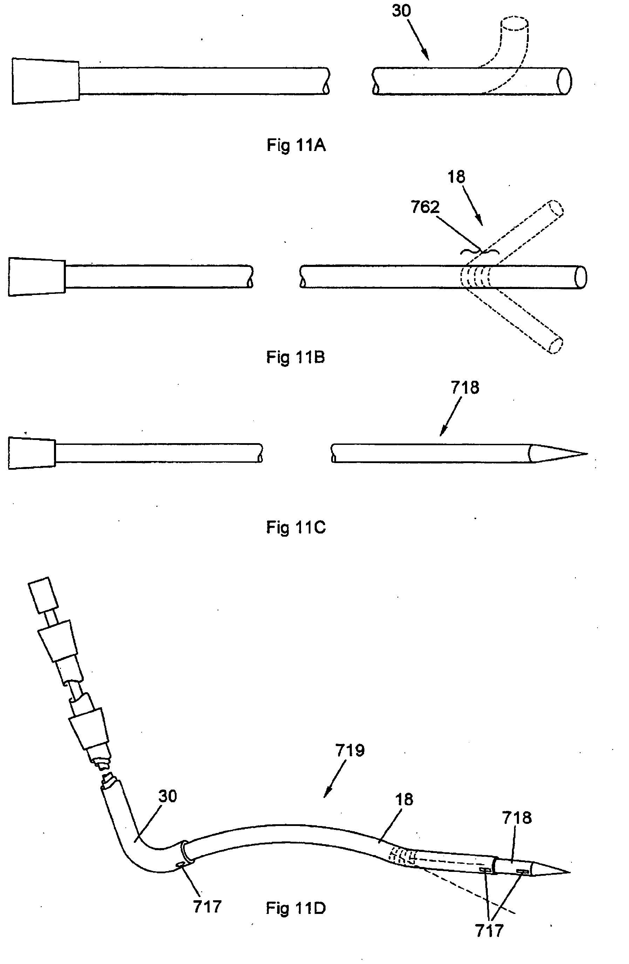

Referring to Figure 11A, a steerable sheath instrument (30) is depicted. Referring to Figure 11B, a steerable guide instrument (18) is depicted, this embodiment having a discrete zone (762) which may be biased to bend more than the adjacent zones through the use of a more flexible structural cross section in such zone. Referring to Figure 11C, a needle instrument or tool (718) is depicted that is configured for traversing soft tissue such as cardiac muscle tissue when advanced forward. Referring to Figure 11D, a coaxial assembly (719) of the instruments depicted in Figures 11A-C is depicted. In the depicted embodiment, localization sensors (717) are coupled to each of the instruments for accurate determination of relative and absolute positioning.

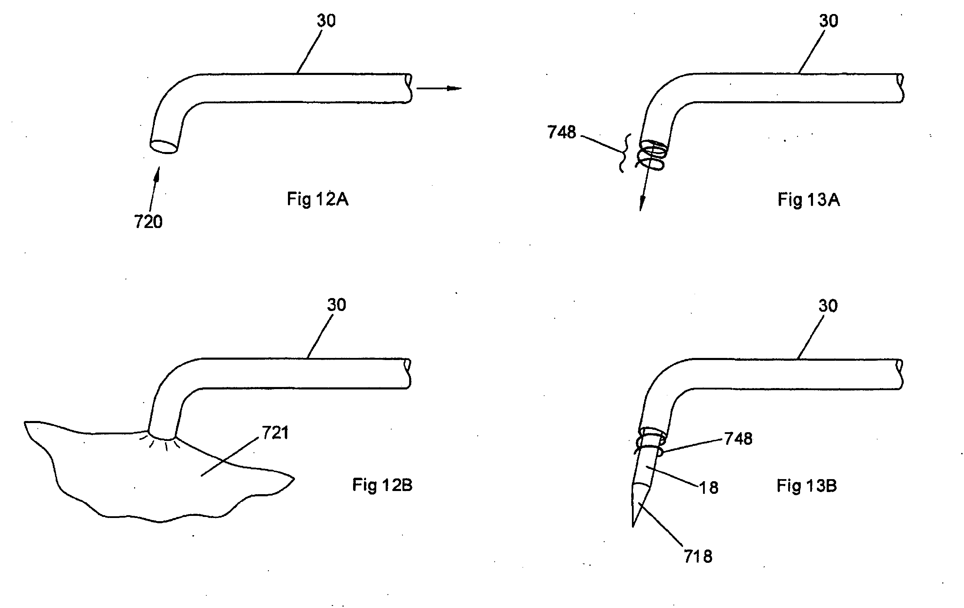

Referring to Figures 12A and 12B, vacuum (720) may be utilized to engage a sheath or guide instrument adjacent a tissue mass or wall (721). Referring to Figures 13A and 13B, a helical coil (748) tip may be utilized to rotatably engage an adjacent tissue mass or wall as a needle tool or instrument (718) is forwarded through the working lumen of the associated sheath (30) or guide (18) instrument.

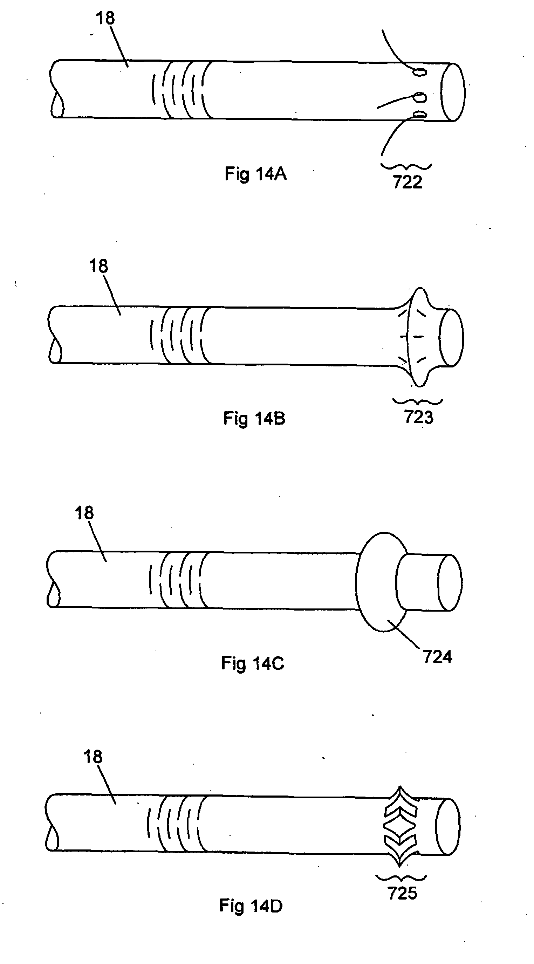

Referring to Figures 14A-D, various anchoring mechanisms may be utilized to engage adjacent tissue masses or walls, such as the outwardly-angled barbs (722) depicted in the embodiment of Figure 14A, the outwardly-expandable circumferential geometry (723) of the embodiment of Figure 14B, and the expandable balloon (724) of the embodiment of Figure 14C. Figure 14D depicts an embodiment similar to that depicted in Figure 14B with the exception that stress relief cutouts are defined in the expandable geometry (725).

Referring to Figure 15, a guide (18), sheath (not shown), or needle (not shown) instrument may be outfitted with ultrasound transducers (726) to image adjacent tissue structures and/or other instruments. Referring to Figures 16A-C, a circumferential array (727) of ultrasound transducers, such as that available from Volcano Therapeutics, Inc., may be utilized to provide images (728) around the circumference of an instrument. Referring to Figures 17A-C, a subset of a full circumferential array may also be utilized to provide a partial circumferential ultrasound image view (729). Referring to Figures 18A-D, imaging transducers (727) may be positioned upon a guide instrument, sheath instrument, or both, in addition to upon a needle instrument.

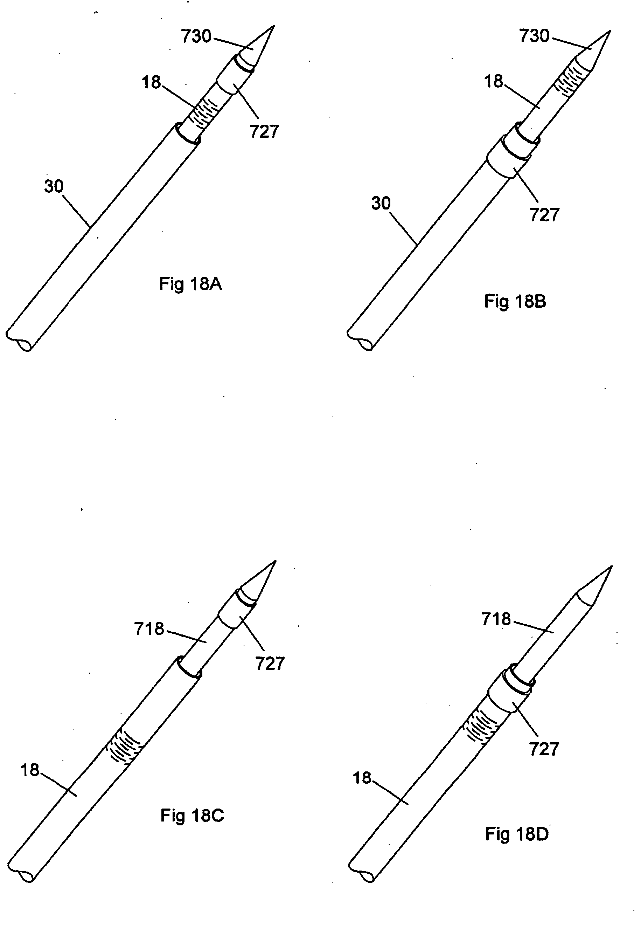

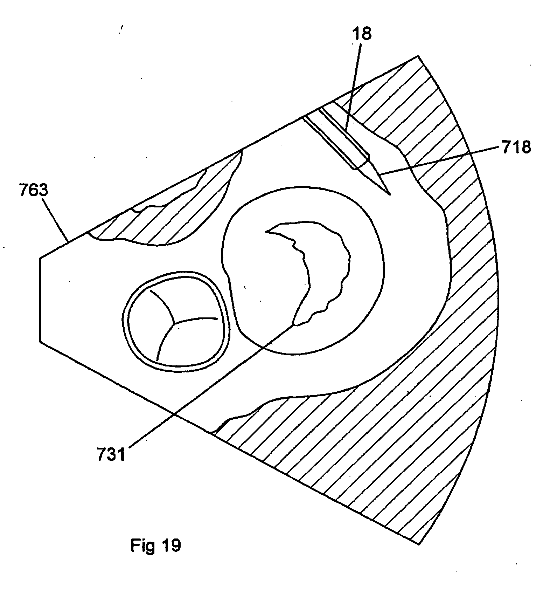

Figure 18A depicts an embodiment wherein an imaging transducer is coupled to a guide instrument (18) which has an integrated needle tip (730). Figure 18B depicts an embodiment similar to that of Figure 18A with the exception that the imaging transducer (727) is coupled to an associated sheath instrument (30). Figure 18C depicts an embodiment wherein an imaging transducer (727) is coupled to a needle instrument (718) deployed through the working lumen of a guide instrument (18). Figure 18D depicts an embodiment similar to that of Figure 18C with the exception that an imaging transducer (727) is coupled to the steerable guide instrument (I8). Referring to Figure 19, conventional intracardiac echo ("ICE") or intravascular ("IVUS") ultrasound also may be utilized to image (763) pertinent tissue structures (731) and instruments (718, 18).

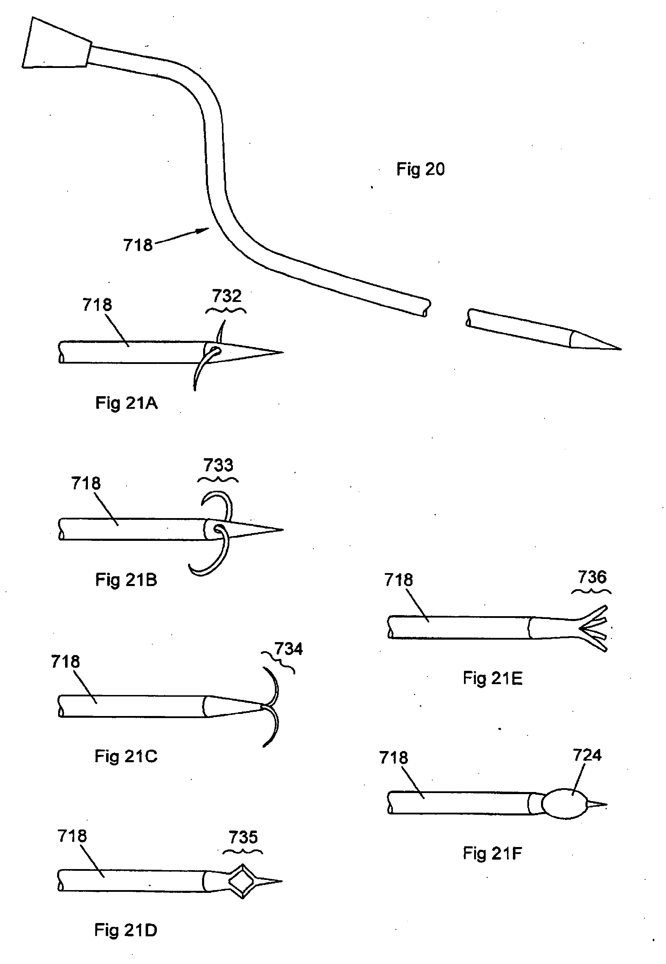

Referring to Figures 20 and 21A-F, various embodiments of suitable tissue-traversing needle instruments (718) are depicted. Figure 20 depicts a needle instrument (718) with a conventional sharp tip. Figures 21A-F depict needle instrument embodiments having controllable mechanisms for at least temporarily anchoring a needle distal tip relative to adjacent tissue structures. Figures 21A-C depict embodiments having deployable barbs (732, 733, 734). Figure 21D depicts circumferentially expandable portions (735) which expand outward as the very distal portion is pulled proximally with a tension member or spring biasing (not shown). Figure 21E depicts radially expandable members (736) deployed distally, while Figure 21F depicts an expandable balloon (724) coupled to the distal end of a needle instrument tip.

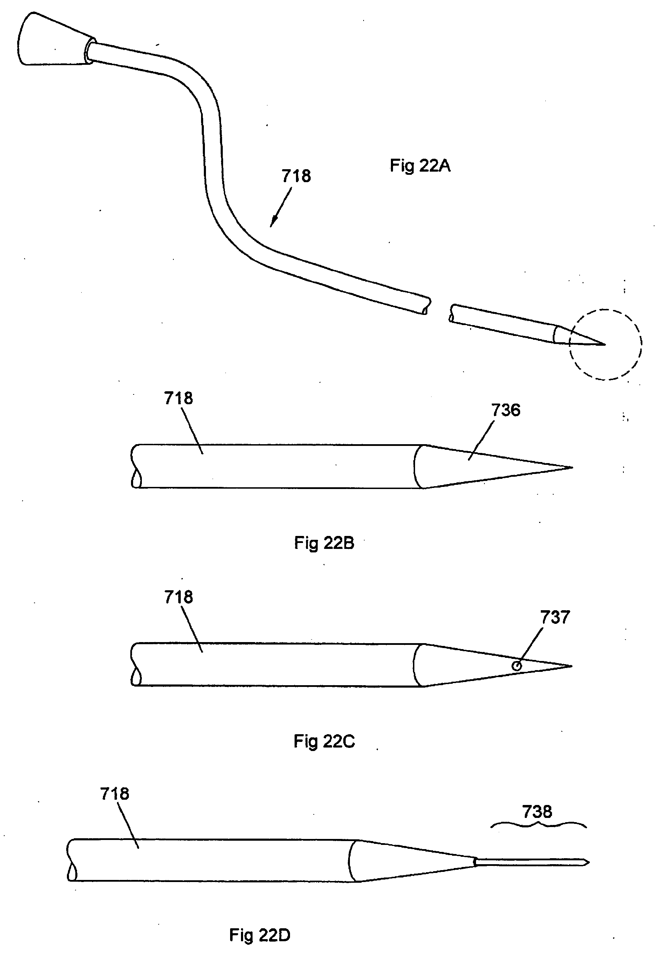

Referring to Figures 22A-22D, various injection tips are depicted for suitable tissue-traversing needle instrument (718) embodiments. Figure 22B depicts a sharp traversing tip such as that depicted in Figure 20 with the exception that the tip defines an injection lumen and port (736). The embodiment of Figure 22C is similar with the exception that the injection port (737) is positioned proximal of the distal tip. The embodiment of Figure 22D is similar with the exception that an additional smaller injection needle tip (738) is positioned distal of the distal tip of the tissue-traversing needle, for injections deeper than desired traversing.

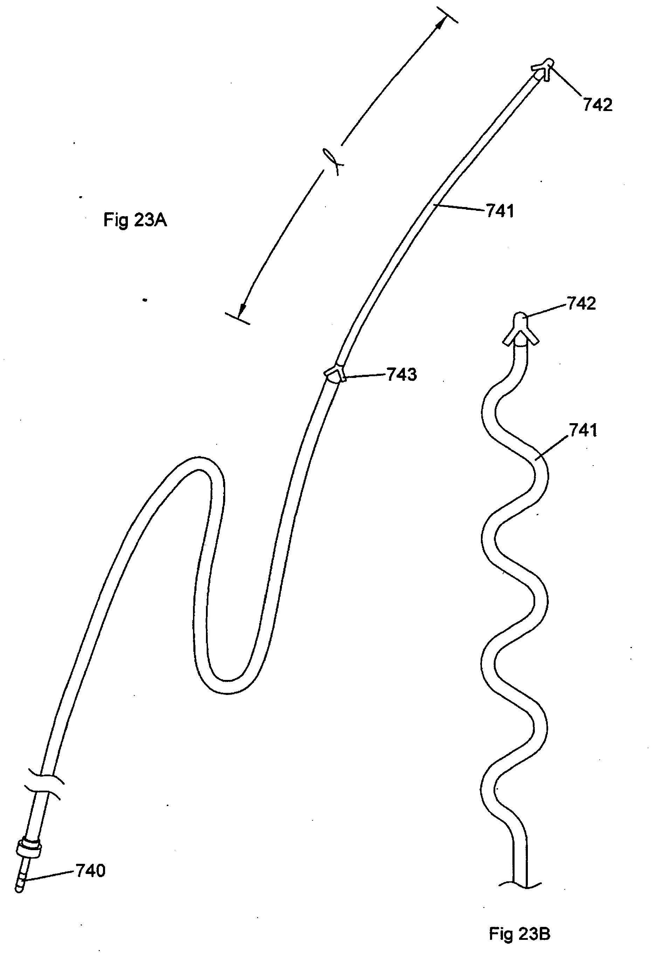

Referring to Figures 23A-28B, various aspects of various embodiments of deployable tension prostheses (741) are depicted. Referring to Figure 23A, a deployable tension prosthesis (741) with straight length "1" is depicted coupled to a flexible elongate instrument. The prosthesis has proximal and distal tissue anchors (743, 742). Figure 23B depicts the prosthesis (741) without the proximal instrument, and shows that the prosthesis preferably is highly flexible.

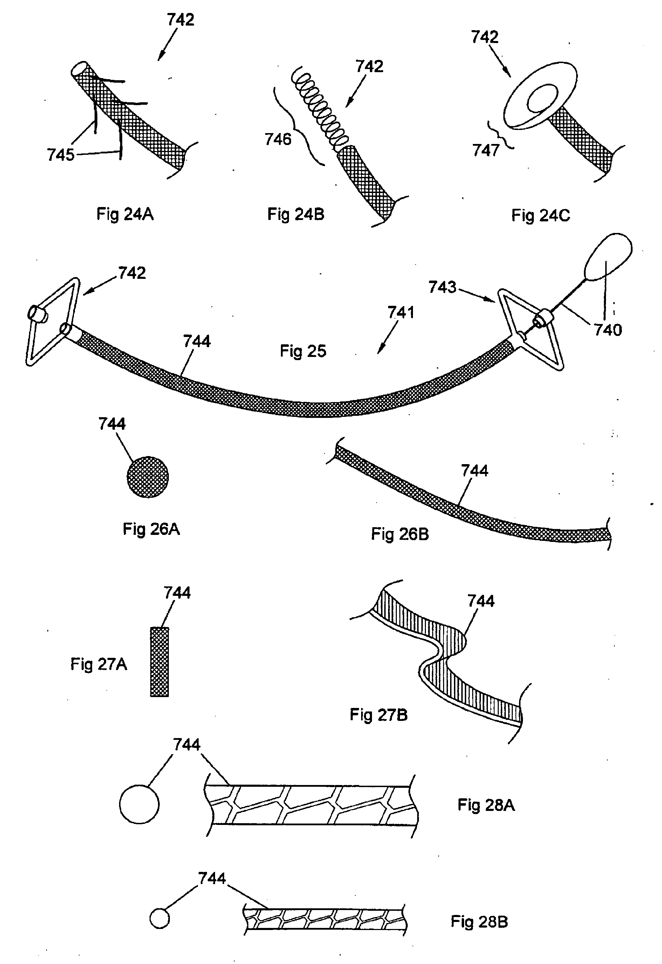

Figures 24A-C depict anchoring tip (742) embodiments for various deployable tension prostheses which may be utilized to retain the position of the distal tip of a deployable tension prosthesis relative to surrounding tissues as the more proximal portions of the deployable tension prosthesis are pulled into tension by a needle instrument proximally. Figure 24A depicts a barbed tip (745). Figure 24B depicts a helical tip (746) which may be wound into adjacent tissue structures. Figure 24C depicts a more broad helical tip (747) which may be utilized to wind into adjacent tissue structures or through holes, defects, or fossas in adjacent tissue structures. Figure 25 depicts a deployable tension prosthesis (741). When the proximal loop of the tension element (740) depicted in Figure 25 is pulled in tension relative to the slidable flexible housing (744), proximal (743) and distal (742) expandable members protrude outward anchoring the prosthesis distally and proximally, enabling application of compression to tissue structures positioned adjacent or between the distal and proximal expandable members. Referring to Figures 26A-B and 27A-B, the slidable flexible housing (744) may comprise a substantially round or substantially circular cross section. Referring to Figures 28A-B, the slidable flexible housing (744) may comprise a composite of various materials or pieces of material, such as PTFE or other preferably biocompatible polymers or metals.

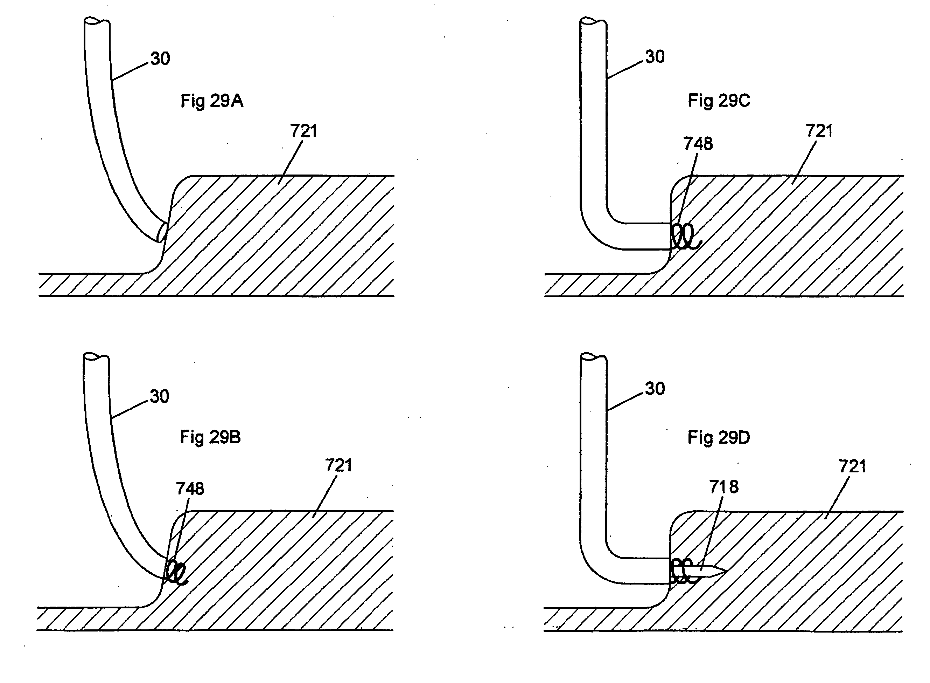

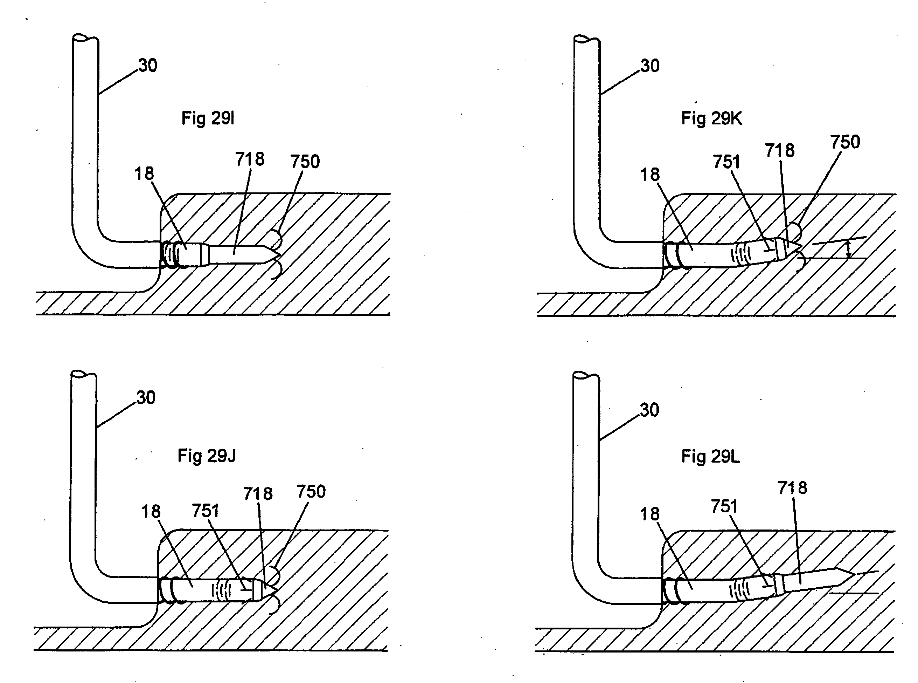

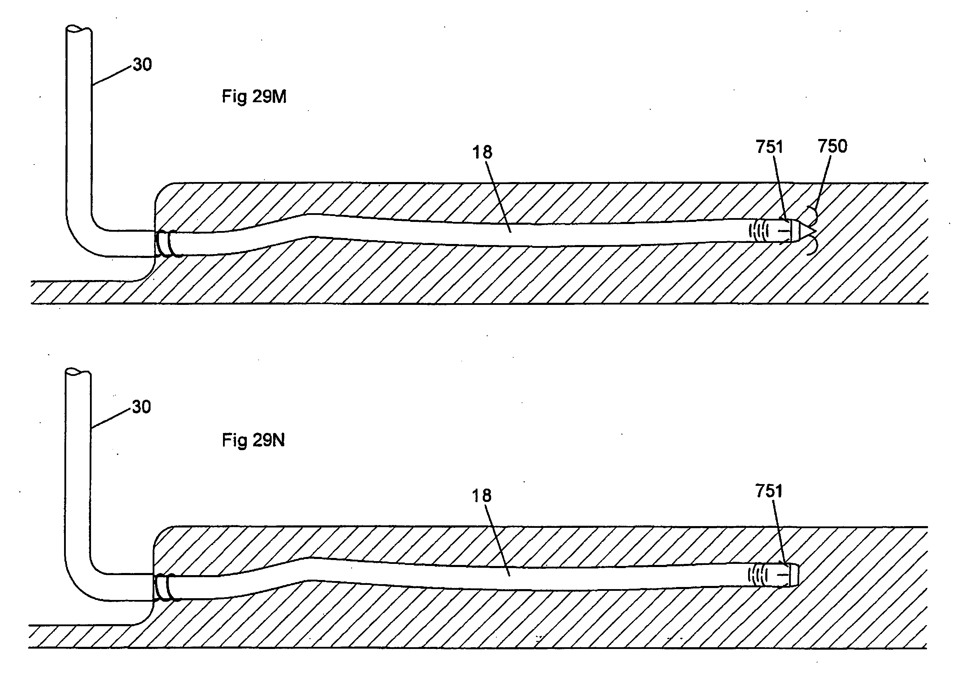

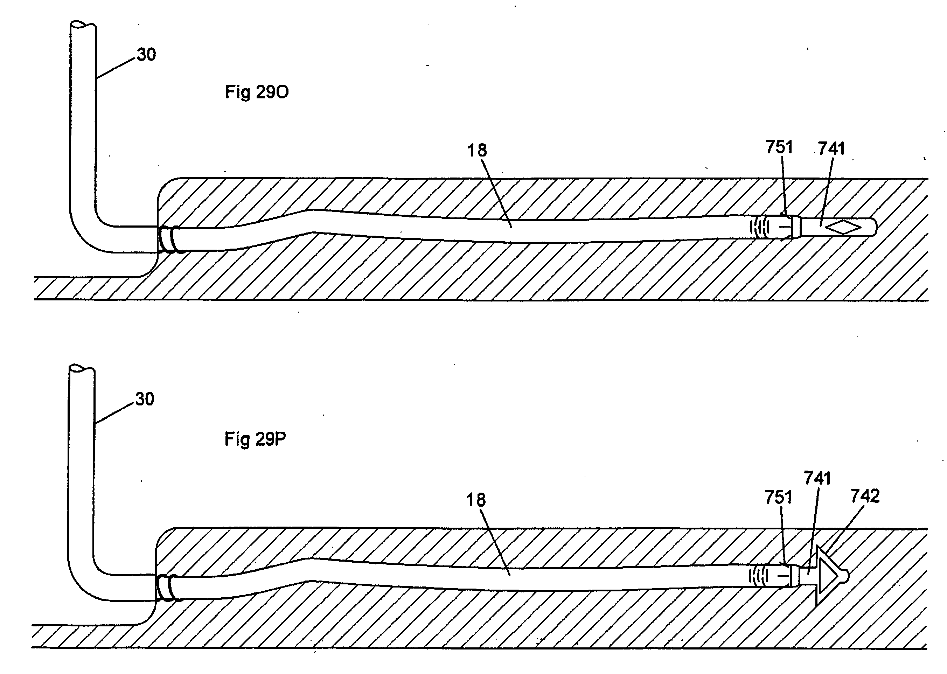

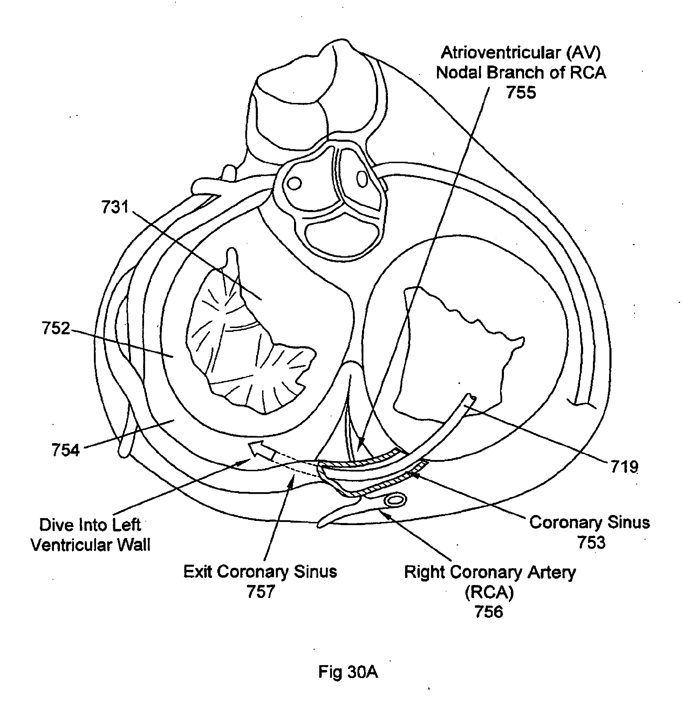

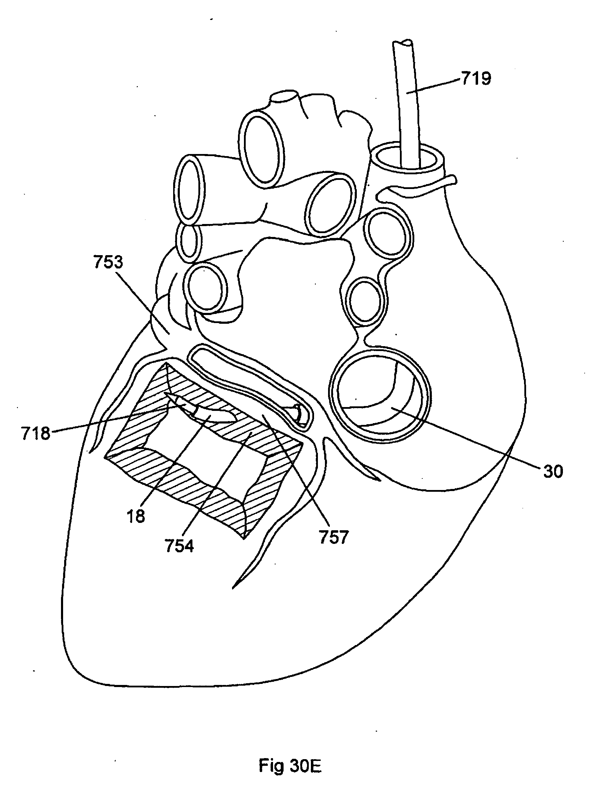

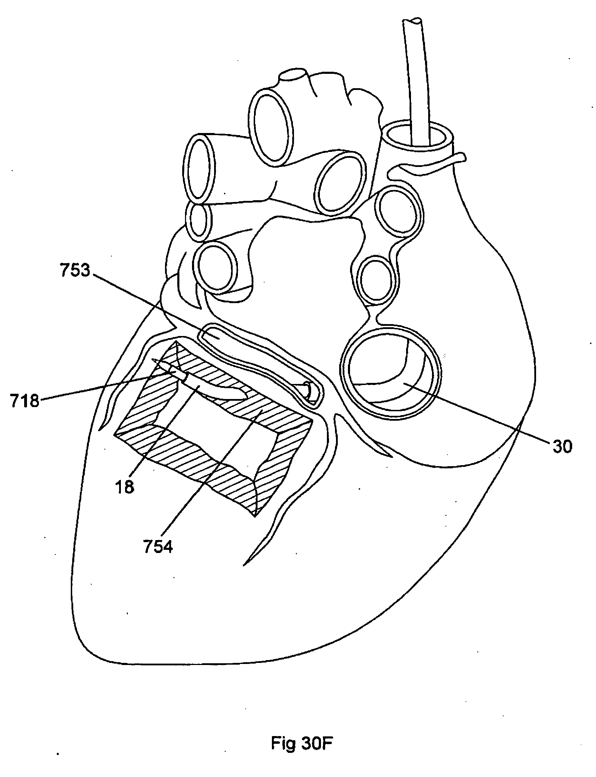

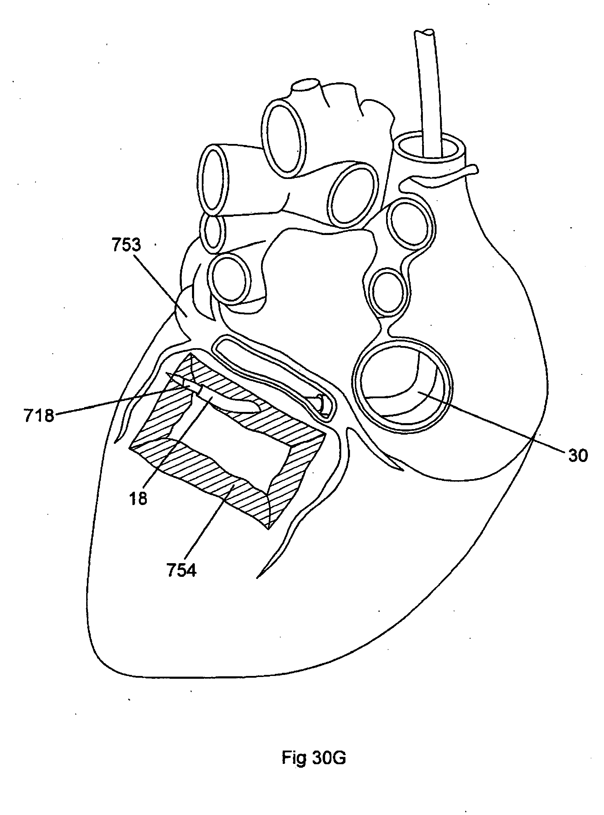

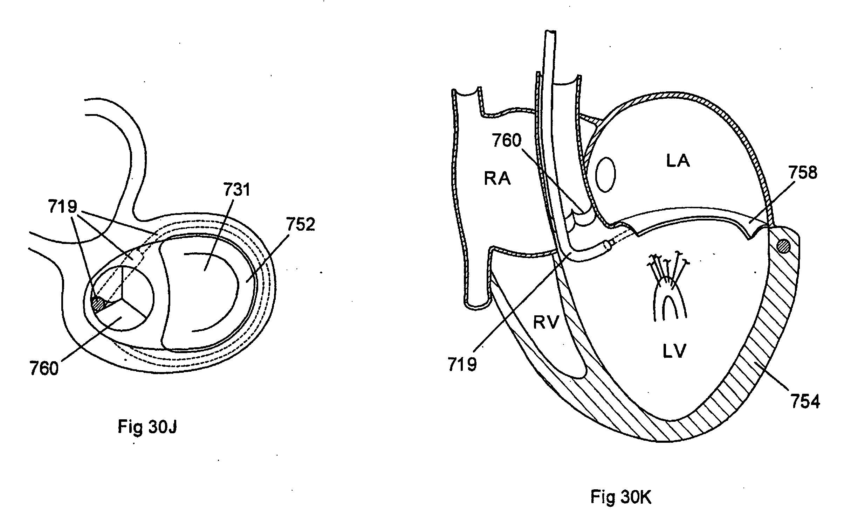

Referring to Figures 29A-R, an assembly and process for utilizing a tension prosthesis to generate compression along and adjacent the length of the tension prosthesis is depicted. A straight line embodiment is depicted, but similar technique may be utilized to generate compression along a curve to, for example, generate hoop stress or a "purse stringing" effect, as depicted in the mitral valve geometry modification embodiment of Figures 30A-M.

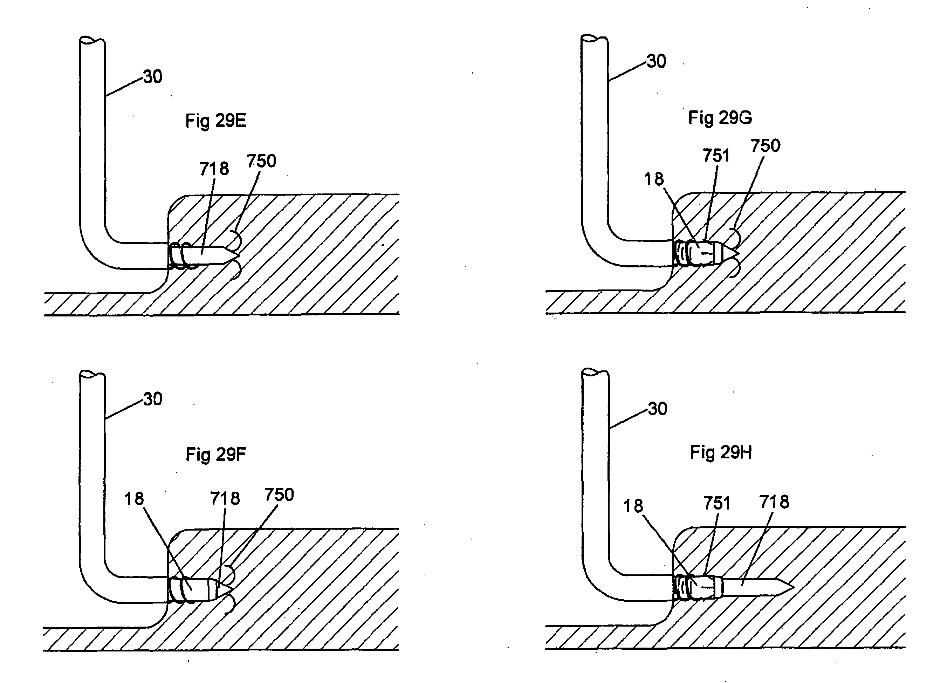

Referring to Figure 29A, a sheath instrument (30) is navigated to a targeted tissue mass (721). As shown in Figures 29B-C, a helical portion (748) may be utilized to engage the tissue mass, subsequent to which a needle instrument (718) may be forwarded into the subject tissue mass, as depicted in Figure 29D. After the needle (718) is advanced forward, the position may be retained by deploying an anchoring mechanism (750), such as barbs, as depicted in Figure 29E. With the needle instrument anchored in position, a guide instrument (18) may be forwarded, coaxially in this embodiment, over the needle instrument (718), as depicted in Figure 29F. With the guide instrument advanced forward, the guide position may be retained by deploying an anchoring mechanism (751), such as barbs as depicted in Figure 29G, while the needle instrument (718) is advanced yet forward, as depicted in Figure 29H.

This subprocess of sequentially forwarding and anchoring the needle instrument (718) and guide instrument (18) may be repeated to incrementally move the guide/needle assembly forward through the tissue mass, as depicted in Figures 29I-N. Between or during incremental forwarding/anchoring moves, the trajectory of the assembly may be controllably modified as depicted in Figures 29K-L by utilizing the remote steerability of the robotic guide (18) instrument. Referring to Figures 29O-P, once the needle (718) and guide (18) instruments are advanced to a preferred distal position, a tension prosthesis (741) may be threaded through the guide instrument (18) and the distal anchor (742) of the tension prosthesis (741) may be expanded into place for form an anchor for the tension prosthesis. The tension prosthesis may be coupled, for example, to the distal end of the needle instrument or other elongate instrument suitable for deploying the prosthesis, and may be decoupled using a simple releasable mechanical linkage, electrolytically erodable linkage, male/female threaded interface, etc.

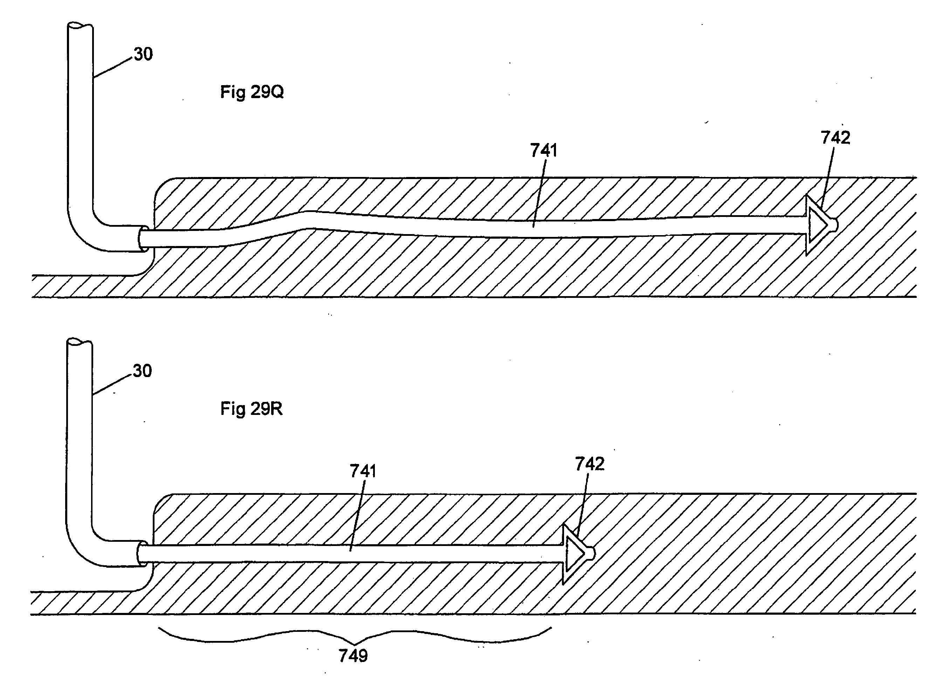

Referring to Figure 29Q, with the tension prosthesis anchored, the guide instrument may be removed by pulling it proximally into the sheath instrument and out of the tissue mass. Finally, the tension prosthesis may be placed into tension with a proximally-applied load to place the adjacent tissue into compression (749) and/or shear stress, as depicted in Figure 29R. A proximal anchor (not shown) may be utilized to lock the proximal end of the prosthesis into position and retain the tensile load.

Referring to Figures 30A-M, a tension prosthesis is utilized to create a hoop stress around the posterior aspect of a malformed mitral valve to modify the valve geometry and preferably decrease valve leakage by bringing the leaflets into better coaption. One of the problems commonly associated with mitral valve leakage is deformation of the posterior aspect of the mitral valve annulus away from the center of the heart (or away from the position of the tricuspid valve, for example).

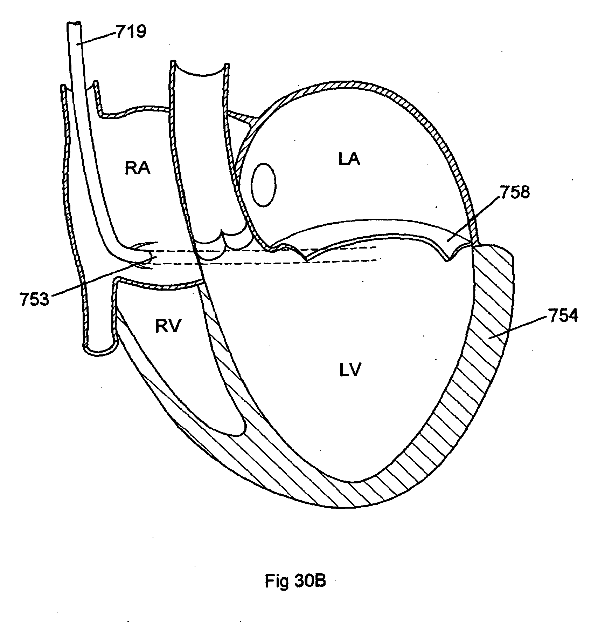

Referring to Figure 30A, the coronary sinus is entered with a steerable guide/sheath instrument assembly from the inferior vena cava. After the instrument assembly (719) is advanced just past the position of the atrioventricular nodal branch (755) of the right coronary artery (756), a needle instrument is protruded out the distal end of the guide instrument and steered by the steerable guide instrument to exit the coronary sinus (753) and dive into the left ventricular wall myocardium (754). An anterior heart view of this pathway around the mitral valve annulus (758) is depicted in Figure 30B.

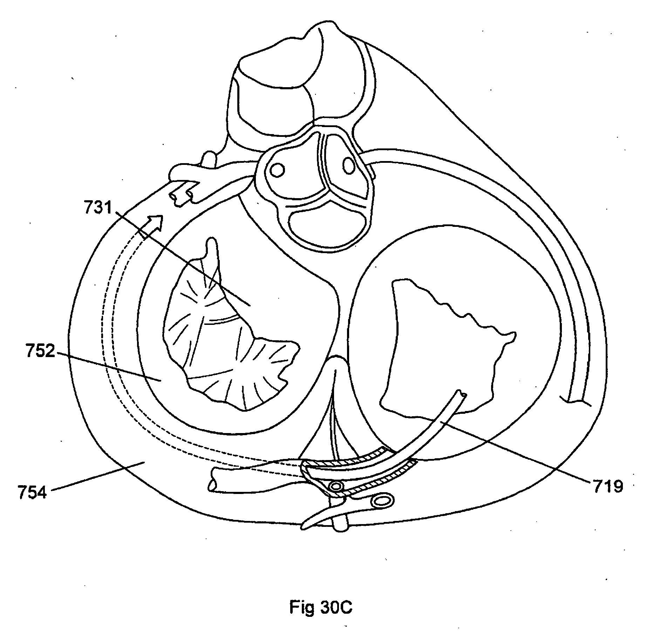

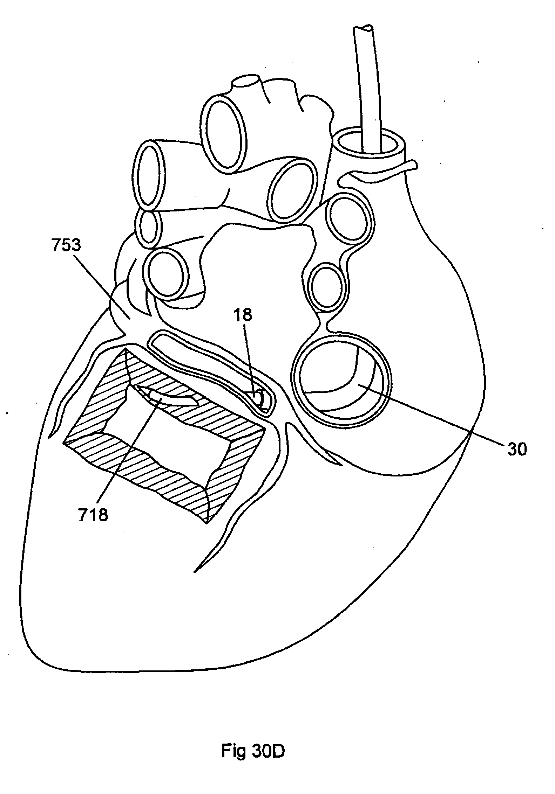

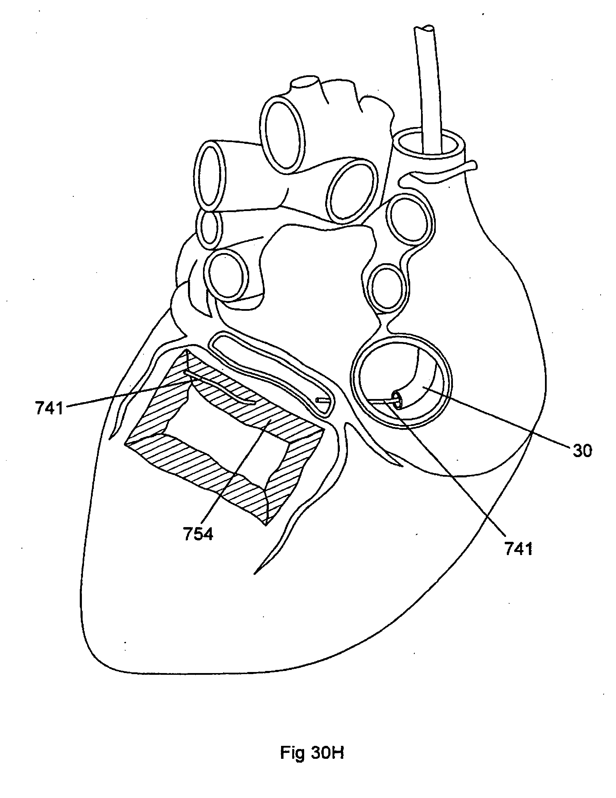

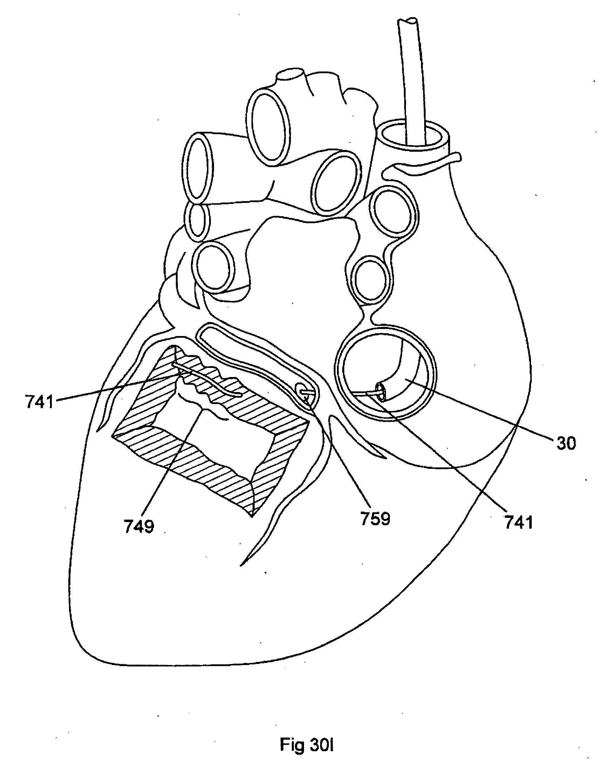

Referring to Figure 30C, using the an incremental forwarding/anchoring/steering process such as that depicted in reference to Figures 29A-R, a tension prosthesis may be deployed in the wall of the myocardium (754) around the position of the posterior aspect (752) of the mitral valve (731) annulus. Figures 30D-G depict a needle/guide instrument assembly diving out of the coronary sinus and into/through the left ventricular wall (windows of the coronary sinus 753 and of the left ventricular wall 754 are removed for illustration purposes). Figure 30H depicts a deployed tension prosthesis (741) after the needle and guide instruments and been pulled away proximally into the sheath instrument (30). Figure 30I depicts hoop stress pushing portions of the left ventricular myocardium adjacent the posterior mitral valve annulus into circumferential compression (749), or "hoop stress", which is desired to pull the posterior mitral valve annulus into a more desirable shape.

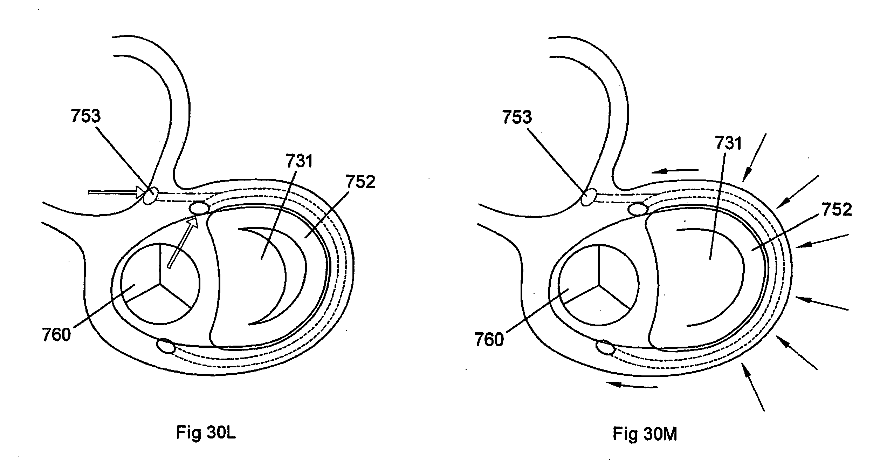

Figures 30J-K depict an embodiment wherein a similar result is achieved by navigating the instrument assembly (719) from a trans-aortic-valve (760) retrograde approach and diving into the left ventricular myocardium between the positions of the aortic (760) and mitral (731) valves. Figures 30L and 30M depict the preferred hoop stress geometric remodeling of the posterior mitral annulus (758) - both the trans-aortic-valve (760) retrograde and coronary sinus (753) approaches are depicted in dashed outlining.

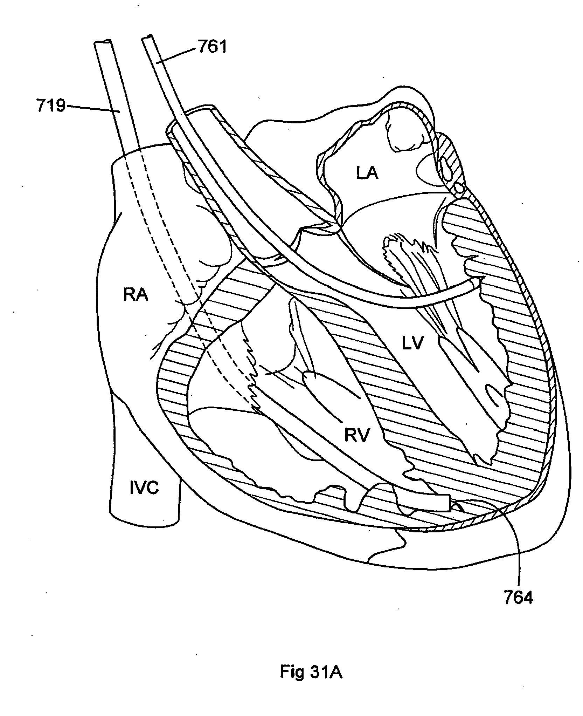

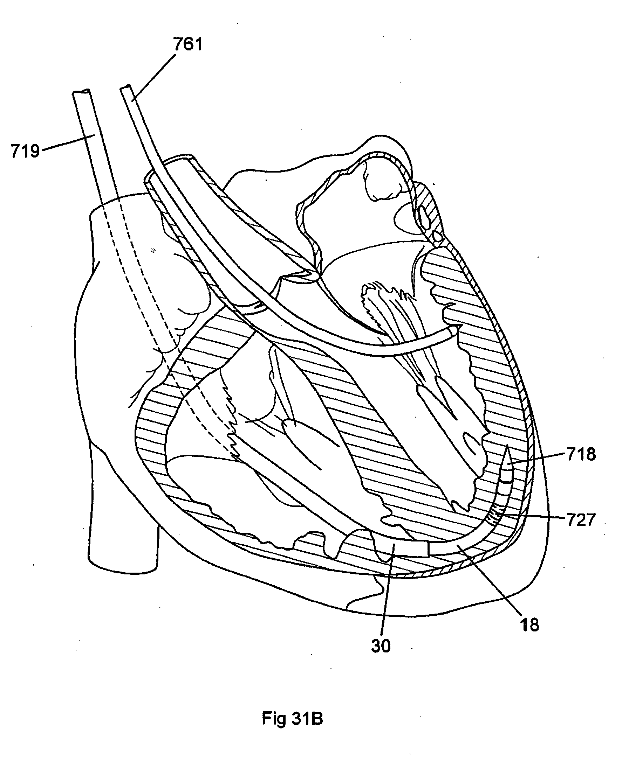

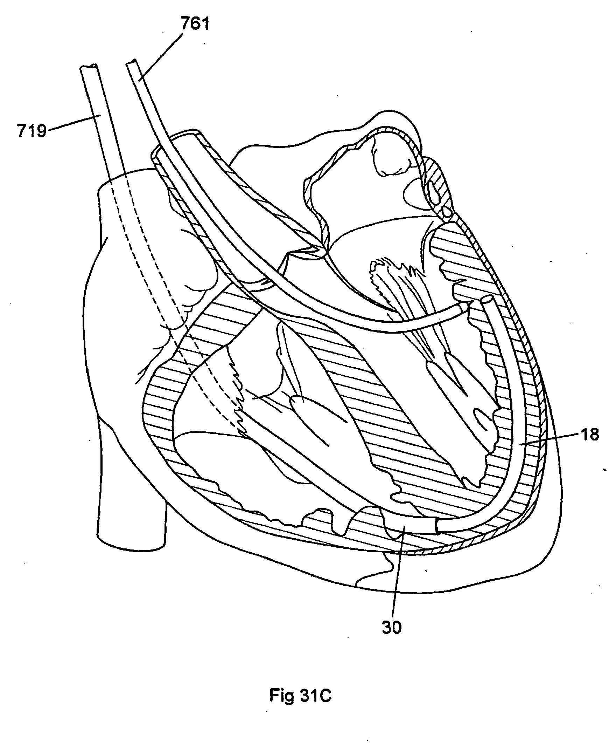

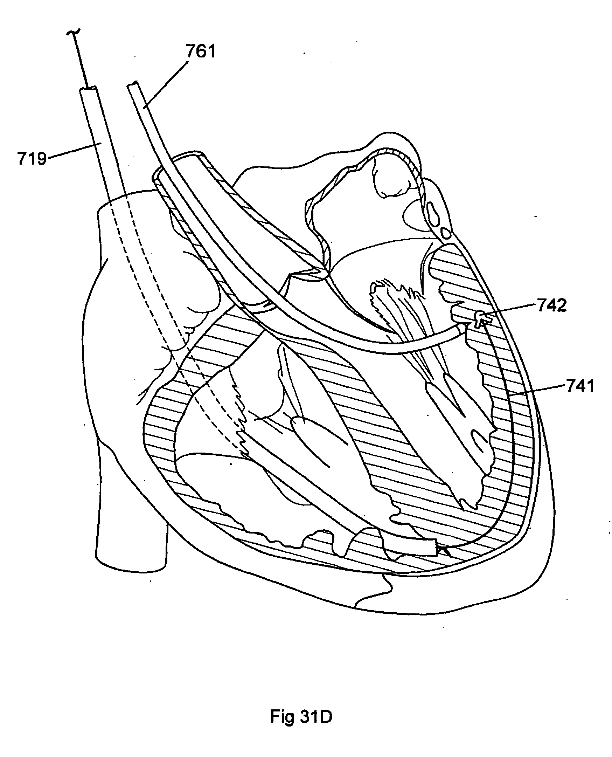

Referring to Figures 31A-D, placement of a tension prosthesis along the length of one aspect of the left ventricle is depicted. Such a tension prosthesis placement may be utilized to take up slack which may be the result of a myocardial infarction, congestive heart failure, etc. As shown in Figure 31A, an assembly (719) comprising sheath, guide, and needle instruments may be navigated from the inferior vena cava, across the tricuspid valve, and toward the right ventricular apex (764). Referring to Figure 31B, the needle instrument (718) and guide instrument (18) may then be deployed forward into the myocardium and steerably incrementally advanced using the aforementioned techniques. Referring to Figures 31C-D, upon arrival to a preferred target location in the myocardium, a tension prosthesis (741) may be deployed and the guide instrument (18) pulled away proximally.

Each of Figures 31A-D also depicts a second catheter instrument (761) placed transaortically into the left ventricle. This second catheter instrument may be utilized to monitor the position of needle/guide instrument assembly (719) within the ventricular wall utilizing ultrasound, localization (magnetic, conduction based, voltage based, etc), and other techniques to increase safety and precision of the deployment of the tension prosthesis. Similarly, a second, instrument may be utilized, for example, in the circumflex artery and/or coronary sinus to carefully monitor the position of adjacent tissue structures and incrementally moving instruments in procedures such as those depicted in Figures 30A-30M.

The remainder of the specification provides additional technical details for assembling and using embodiments of the robotic system disclosed herein.

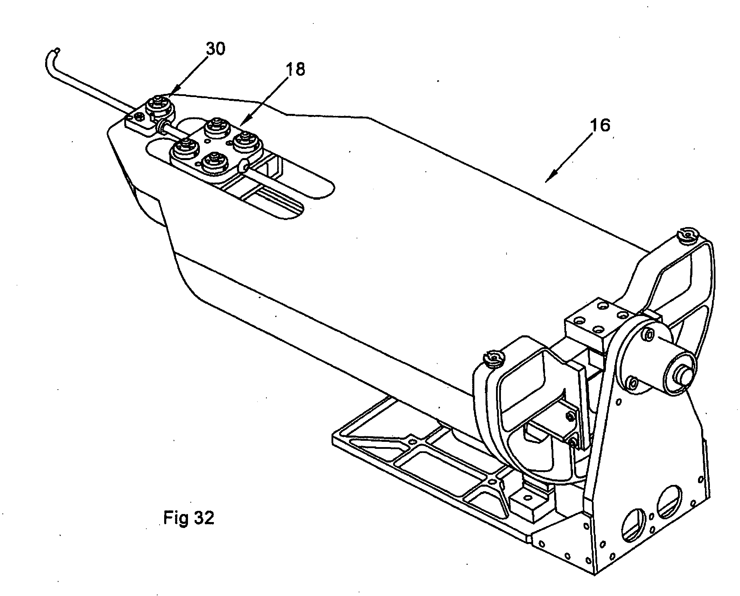

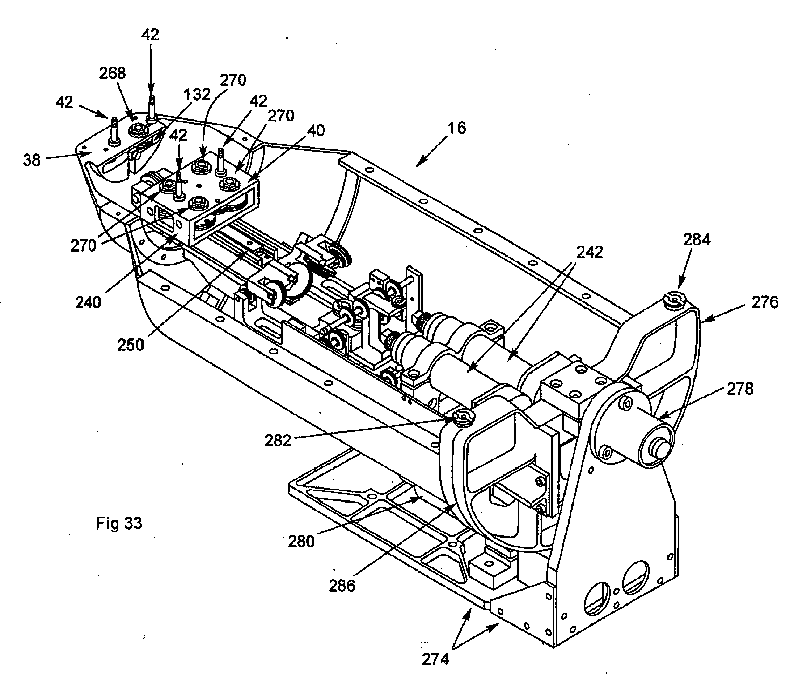

Figures 32-38 illustrate various embodiments of the instrument driver. In Figure 32, an instrument driver (16) is depicted as interfaced with a steerable guide instrument (18) and a steerable sheath instrument (30). Figure 33 depicts an embodiment of the instrument driver (16), in which the sheath instrument interface surface (38) remains stationary, and requires only a simple motor actuation in order for a sheath to be steered using an interfaced control element via a control element interface assembly (132). This may be accomplished with a simple cable loop about a sheath socket drive pulley (272) and a capstan pulley (not shown), which is fastened to a motor, similar to the two upper motors (242) (visible in Figure 33). The drive motor for the sheath socket drive schema is hidden under the linear bearing interface assembly.

The drive schema for the four guide instrument interface sockets (270) is more complicated, due in part to the fact that they are coupled to a carriage (240) configured to move linearly along a linear bearing interface (250) to provide for motor-driven insertion of a guide instrument toward the patient relative to the instrument driver, hospital table, and sheath instrument. The cabling and motor schema moves the carriage (240) along the linear bearing interface (250). Various conventional cable termination and routing techniques are utilized to accomplish a preferably high-density instrument driver structure with the carriage (240) mounted forward of the motors for a lower profile patient-side interface.

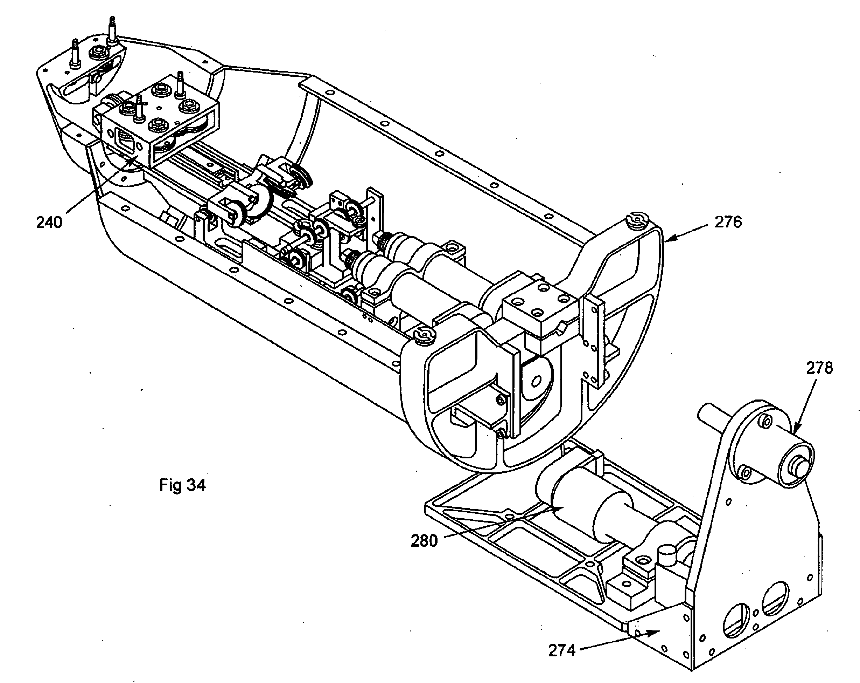

Still referring to Figure 33, the instrument driver (16) is rotatably mounted to an instrument driver base (274), which is configured to interface with an instrument driver mounting brace (not shown), such as that depicted in Figure 1, or a movable setup joint construct (not shown), such as that depicted in Figure 2. Rotation between the instrument driver base (274) and an instrument driver base plate (276) to which it is coupled is facilitated by a heavy-duty flanged bearing structure (278). The flanged bearing structure (278) is configured to allow rotation of the body of the instrument driver (16) about an axis approximately coincident with the longitudinal axis of a guide instrument (not shown) when the guide instrument is mounted upon the instrument driver (16) in a neutral position. This rotation preferably is automated or powered by a roll motor (280) and a simple roll cable loop (286), which extends around portions of the instrument driver base plate and terminates as depicted (282, 284). Alternatively, roll rotation may be manually actuated and locked into place with a conventional clamping mechanism. The roll motor (280) position is more easily visible in Figure 34.

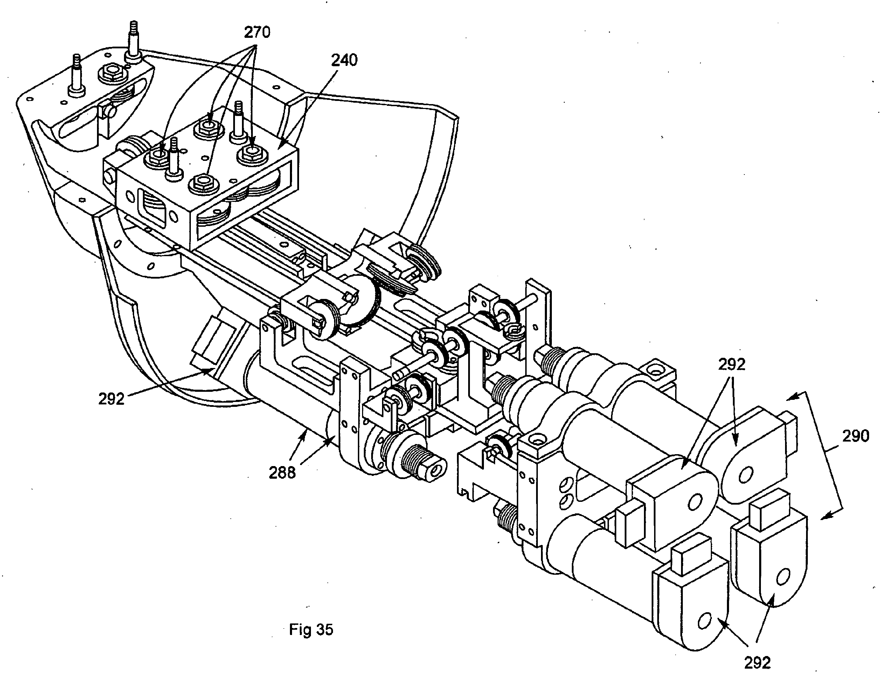

Figure 35 illustrates another embodiment of an instrument driver, including a group of four motors (290). Each motor (290) has an associated high-precision encoder for controls purposes and being configured to drive one of the four guide instrument interface sockets (270), at one end of the instrument driver. Another group of two motors (one hidden, one visible - 288) with encoders (292) are configured to drive insertion of the carriage (240) and the sheath instrument interface socket (268).

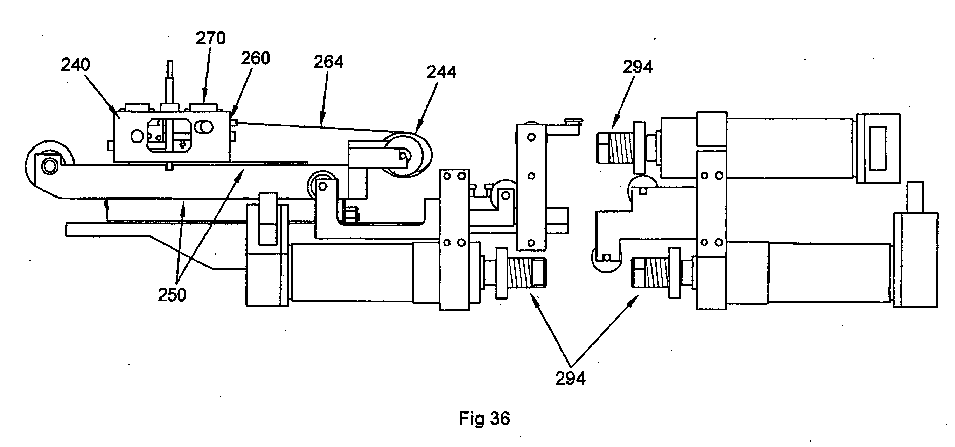

Referring to Figure 36, a further embodiment of an instrument driver is depicted to show the position of the carriage (240) relative to the linear bearing interfaces (250). Also shown is the interfacing of a portion of a instrument interface cable (264) as it bends around a pulley (244) and completes part of its loop to an instrument interface pulley (260) rotatably coupled to the carriage (240) and coupled to a guide instrument interface socket (270), around the instrument interface pulley (260), and back to a motor capstan pulley (294). To facilitate adjustment and installation of such cable loops, and due to the fact that there is generally no requirement to have a loop operating for a long period of time in one direction, thereby perhaps requiring a true unterminated loop, two ends of a cut cable loop preferably are terminated at each capstan (294).

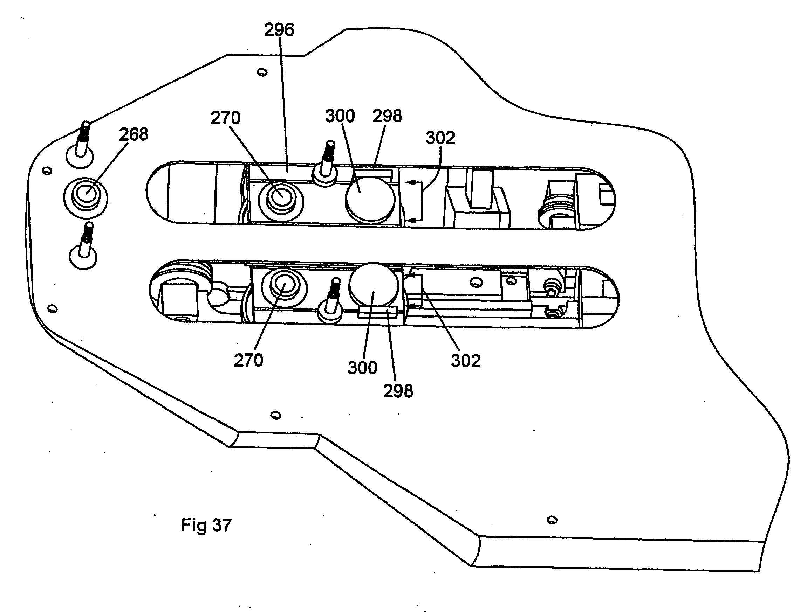

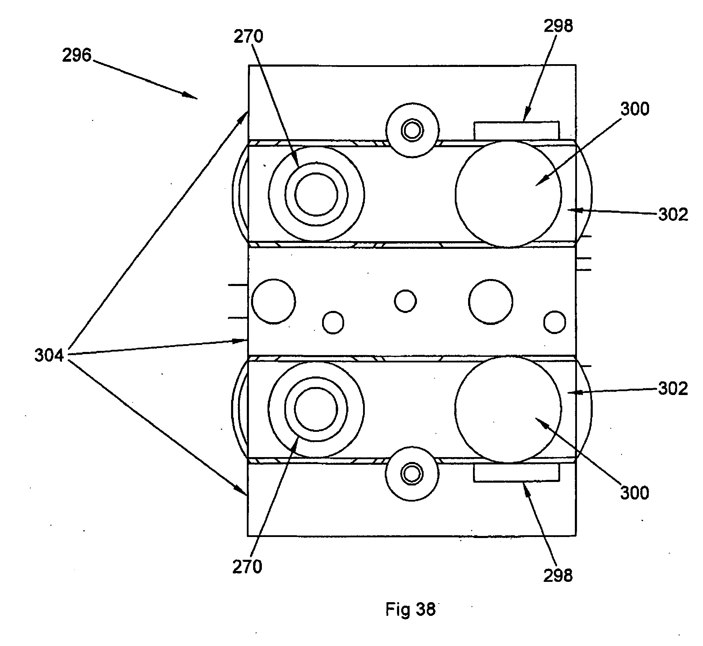

The carriage (240) depicted in the embodiments of Figures 32-36 generally comprises a structural box configured to house the instrument interface sockets and associated instrument interface pulleys. Referring to Figures 37 and 38, a split carriage (296) is depicted, comprising a main carriage body (304) similar to that of the non split carriage depicted in previous embodiments (240), and either one or two linearly movable portions (302), which are configured to slide relative to the main carriage body (304) when driven along either forward or backward relative to the main carriage body by a gear (300) placed into one of the guide instrument interface sockets, the gear (300) configured to interface with a rack (298) mounted upon the main carriage body (304) adjacent the gear (300). In an alternate embodiment, the carriage need not be split on both sides, but may have one split side and one non-split side. Further, while a carriage with four guide instrument interface sockets is suitable for driving a guide instrument with anywhere from one to four control element interface assemblies, the additional hardware required for all four control element interface assemblies may be undesirable if an instrument only requires only one or two.

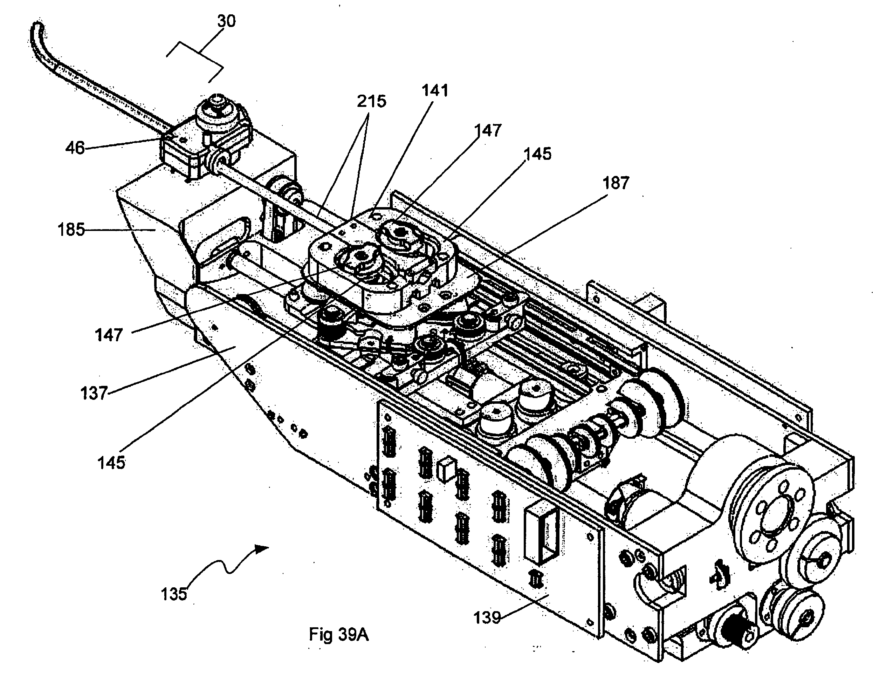

Referring to Figures 39A-39K, another variation of an instrument driver is depicted, comprising a variation of a split carriage design, such as that depicted in Figure 38. As opposed to the embodiment of Figure 38, wherein each instrument base interface is moved straight along a slot, or rotated, or both (independently), the embodiment of Figures 39A-39K provides rotation and/or arcuate slot motion by a "winged" split carriage design, wherein the tension member pulleys and axles may be rotated about the axle axis, or moved along an arcuate pathway, independently.

Referring to Figure 39A, a winged split carriage instrument driver (135) is depicted coupled to a guide instrument (215) configured for the winged split carriage with a specialized guide instrument base (141) having two arcuate slots (145) as opposed to straight slots in other embodiments. One or more electronics boards (139) preferably are coupled to the main housing structure (137) of the winged split carriage instrument driver (135). The depicted assembly also comprises a sheath instrument (30) movably threaded over at least a portion of the guide instrument (215) and coupled to the sheath frame block (185) which is coupled to the main housing structure (137) when the depicted assembly is fully assembled.

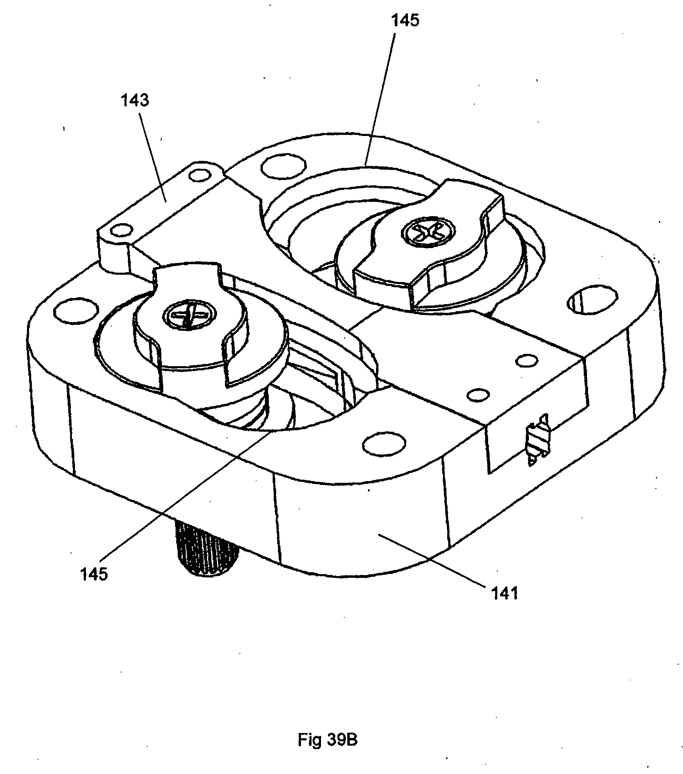

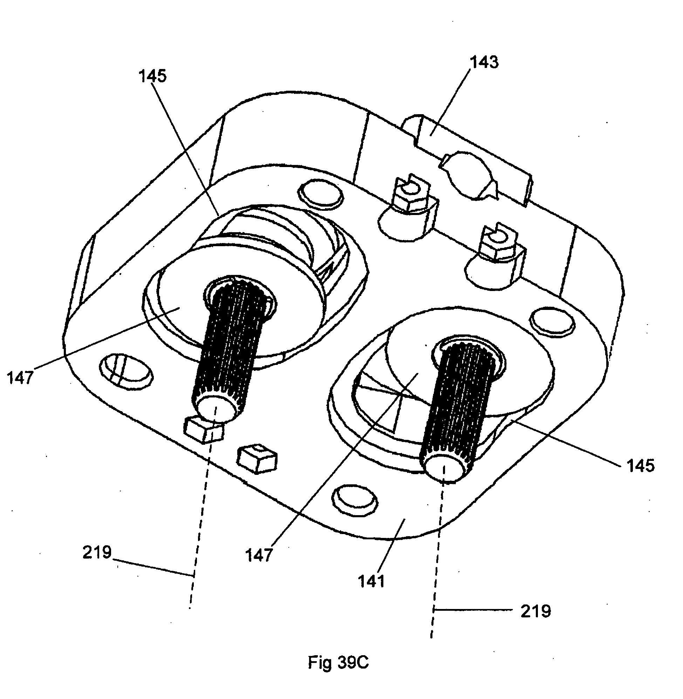

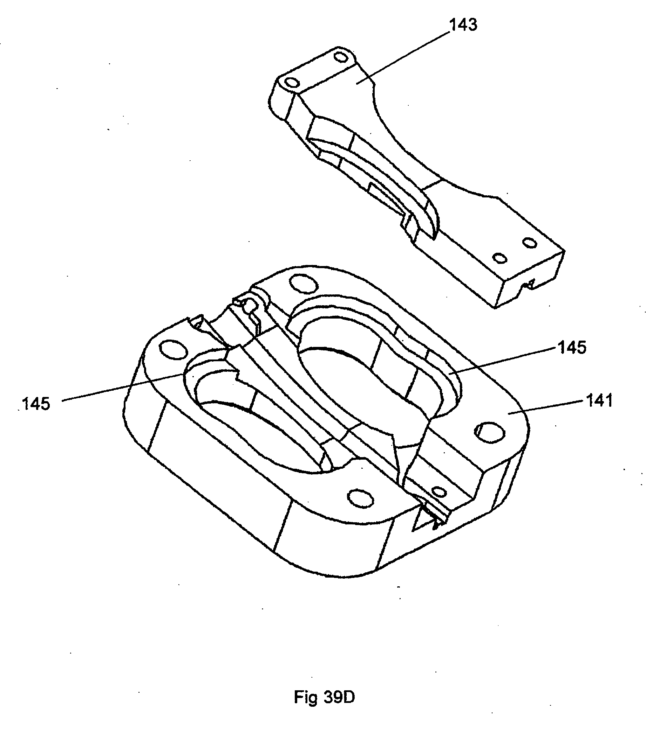

Referring to Figure 39B, a winged instrument driver guide instrument base (141) is depicted showing the arcuate slots (145) in greater detail, as well as a winged instrument driver guide instrument base top plate (143), which is configured to be fitted down upon the proximal tubular portion of a guide instrument catheter member (not shown) to maintain the relative positioning of the catheter member (not shown) relative to the winged instrument driver guide instrument base (141). An underside isometric view of the same structures depicted in Figure 39B is depicted in Figure 39C. In the depicted embodiment, a low-profile control element interface assembly (147) is configured to rotate about the longitudinal axis of the interface assembly (219) while also slidably translating through the associated arcuate slot (145). Figure 39D depicts an exploded view of the winged instrument driver guide instrument base top plate (143) and winged instrument driver guide instrument base (141) depicted in Figure 39B, also showing the arcuate slots (145) defined therein.

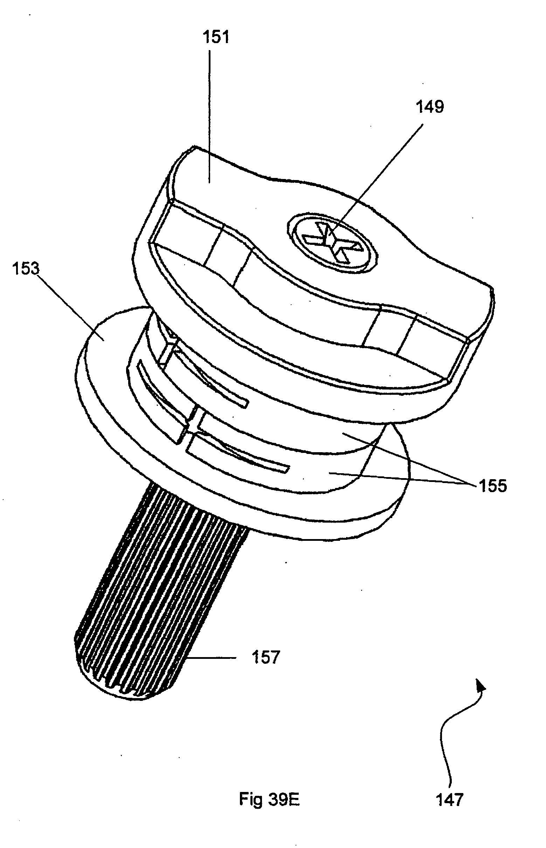

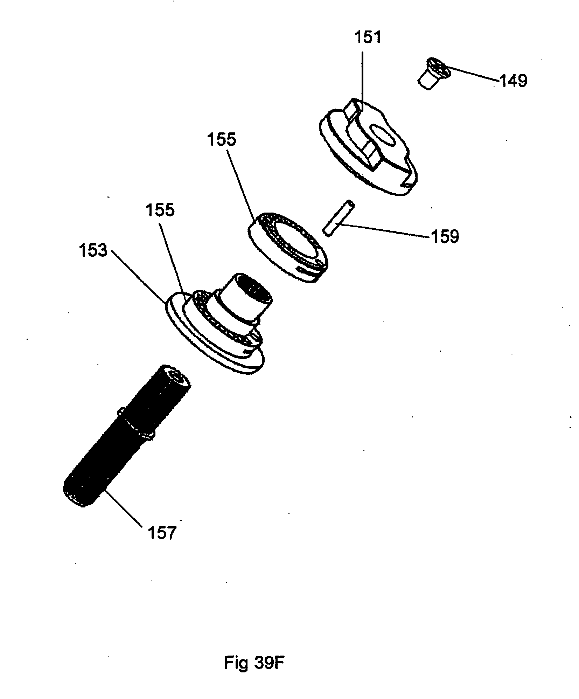

Referring to Figure 39E, a low-profile control element interface assembly (147) is shown in isometric view comprising a splined axle (157) coupled to a pulley flange (153), and also coupled to a set of control element pulleys (155) which are compressed between a low-profile manual adjustment knob (151) and the pulley flange (153) with a retaining fastener (149), such as a screw. An exploded view of the same structures is depicted in Figure 39F. Also shown in Figure 39F is a pin (159) configured to prevent relative rotational displacement between the two control element pulleys (155) when the low-profile control element interface assembly (147) is assembled. The depicted embodiment of low-profile control element interface assembly (147) may be utilized with any of the aforementioned instrument base and instrument driver assemblies, subject to the requirement that the instrument interface sockets are also geometrically matched for a splined interface between socket and axle facilitating highly-efficient transfer of loads between the matched socket and axle. The low-profile control element interface assembly (147) preferably comprises polymers or metals which may be formed or machined into very high precision subassemblies or parts which are low in weight, high in hardness, and low in fracture toughness. In one embodiment, each of the components of the low-profile control element interface assembly (147) comprises polycarbonate or ultra-high-molecular-weight polyethylene.

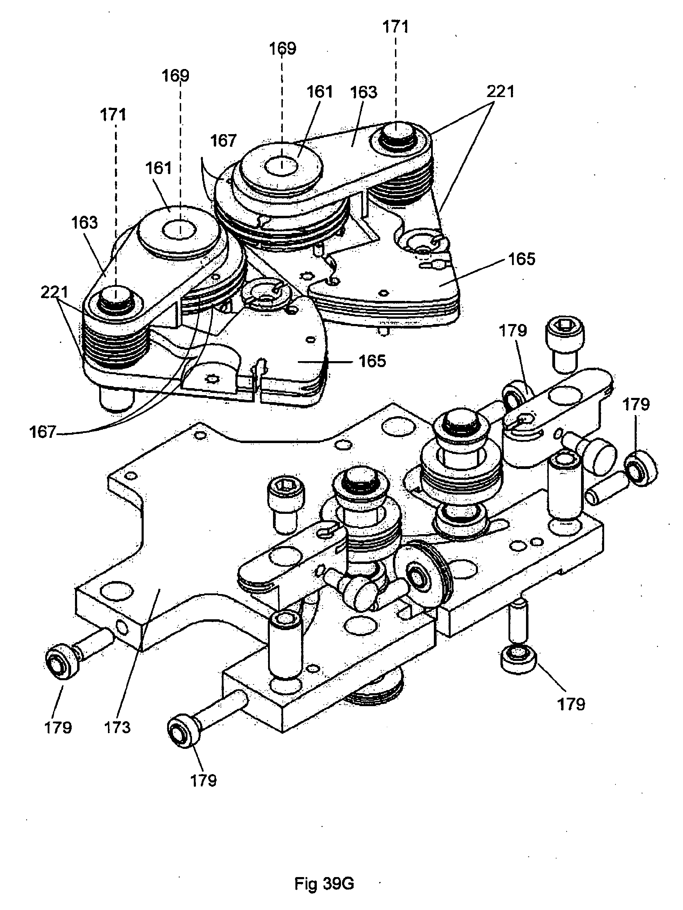

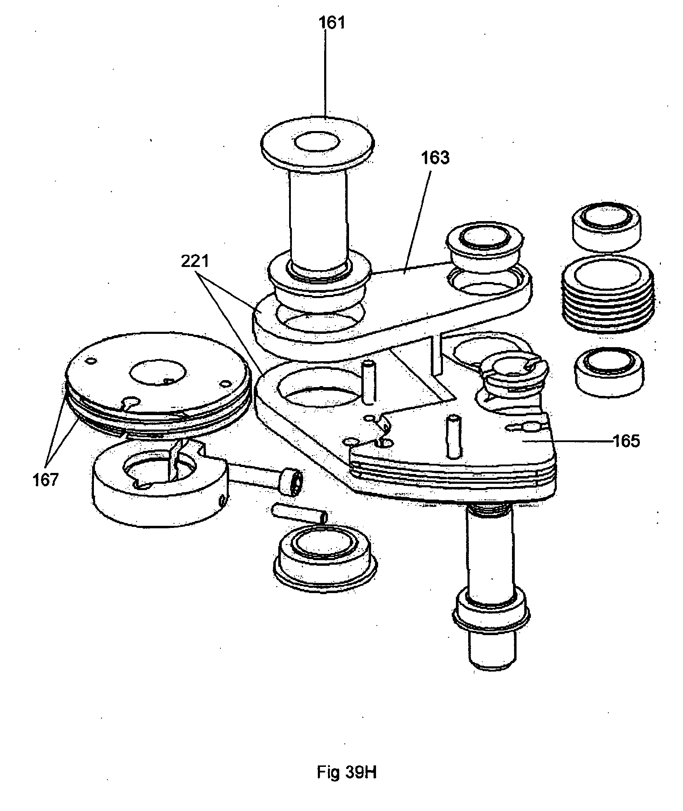

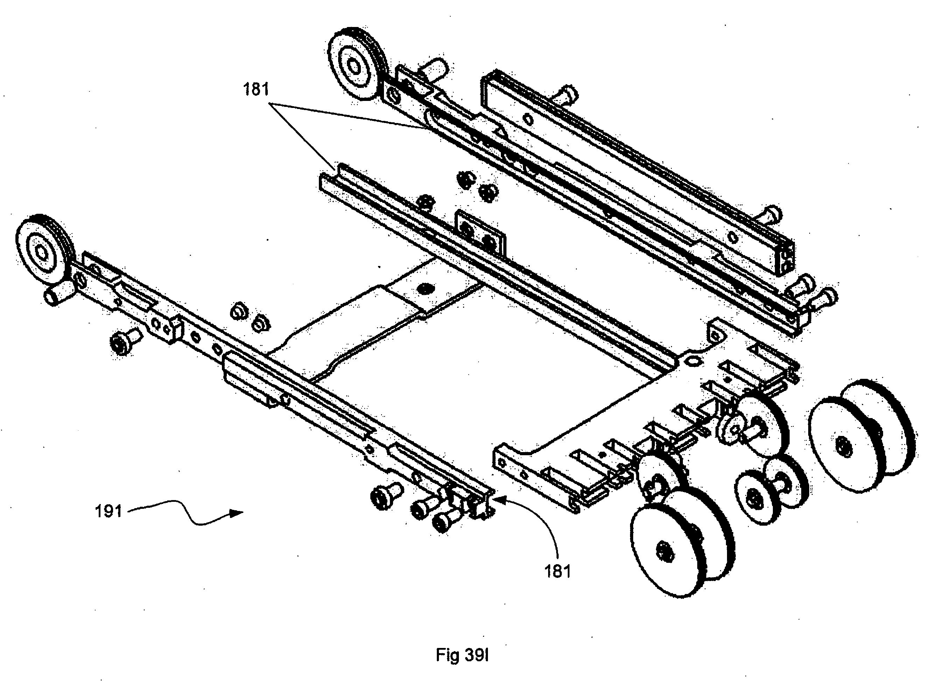

Referring to Figure 39G, a winged split carriage assembly is depicted in semi-exploded view. The winged carriage base (173) is configured to rotatably support two independently rotatable wing structures (221), each comprising a bottom portion (165) and a top portion (163). A further exploded view of the wing structures (221) and associated members are depicted in Figure 39H. Rotatably coupled to the rotatable wing structures (221) is a set of control element pulleys (167) to which a splined instrument interface socket (161) is coupled. The winged carriage base (173) is configured to slidably couple to a carriage interface frame (not shown) with bearings (179). As shown in Figure 39I, slots (181) constrain the motion of the winged carriage base (173) relative to the carriage interface frame (191) to linear motion. Shafts and bearings are utilized to rotatably couple the wing structures (221) to the winged carriage base and facilitate rotational motion of the wing structures (221) about the axis of the pertinent coupling shaft (171). Similar shaft and bearing configurations are utilized to provide for rotation of the control element pulleys (167) relative to the wing structures (221). Thus, the winged split carriage design is configured to allow for independent motion of each of two wing structures (221), while also allowing for independent rotational motion of two sets of control element pulleys (167) and thereby instrument interface sockets (161). In other words, with a winged guide instrument (215) such as that depicted in Figure 39A coupled to an arcuate slot instrument mounting base (187), and two control element interface assemblies (147) coupled to two instrument interface sockets positioned below the mounting base (187) in the configuration depicted in Figure 39A, each of the control element interface assemblies (147) may be rotated about their longitudinal axis, and also arcuately translated through the arcuate slot formed in the instrument base (141), to provide for tensioning and control of two control elements, one around each of the control element pulleys (167) on each of the control element interface assemblies (147), with actuation of a single control element interface assembly (147). Thus four control elements may be driven with the actuation of only two control element interface assemblies (147).

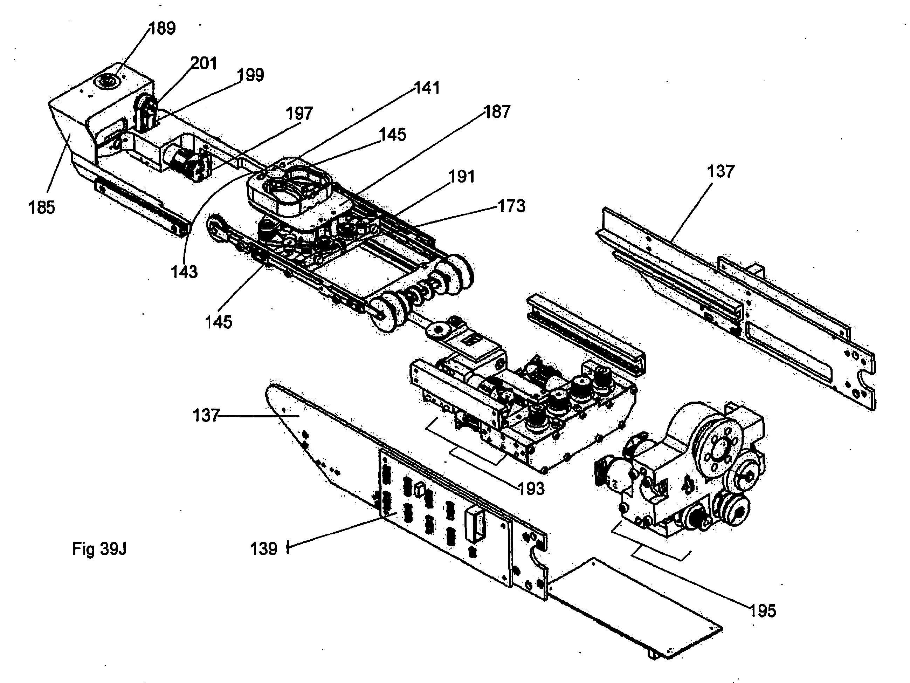

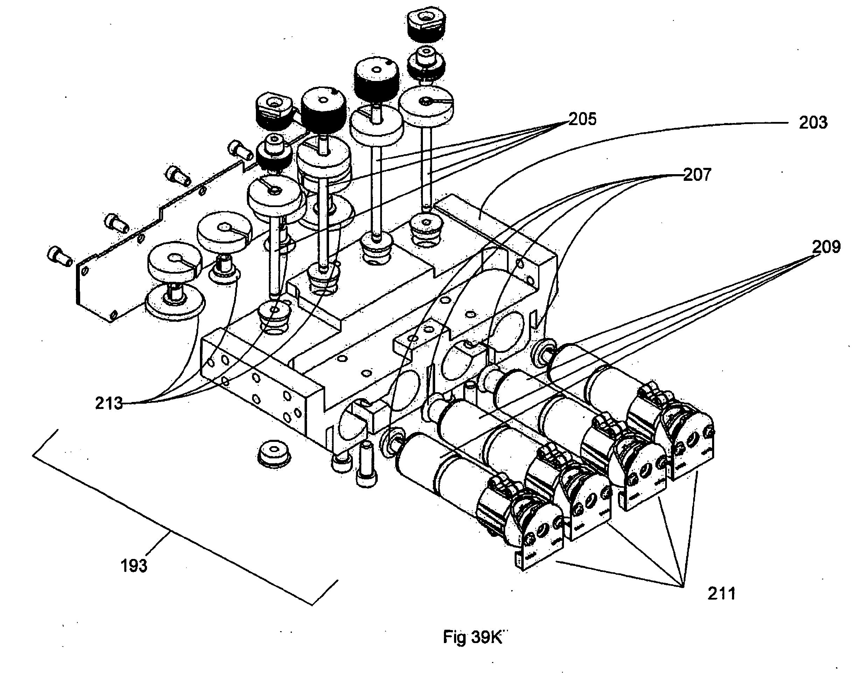

Referring to Figure 39J, an exploded view of an assembly similar to that depicted in Figure 39A is depicted. Neither the sheath instrument, the two control element interface assemblies, nor the guide instrument catheter member are depicted in Figure 39J. As with aforementioned embodiments, the instrument driver roll assembly (195) and instrument driver motor/gear assembly (193) are coupled to the main frame (137) of the instrument driver. As shown in Figure 39K, redundant encoder readers (211) associated with each of four control element drive motors (209) of this embodiment facilitate high precision rotational position readings of the motor shafts and prevent position read errors. The motor output shafts are coupled to bevel gears (207) which are interfaced with another set of bevel gears (213) and thereby configured to drive the depicted vertical output shafts (205). The motor/gear interface block (203) is utilized to couple the motors, gears, and shafts into positions relative to each other and the main frame of the instrument driver (not shown), while constraining motions generally to rotational motions of shafts, motors, gears, and bearings. The rotation and arcuate translation of the winged structure instrument interface sockets (161) relative to the winged carriage base (173) and wing structures (221) is a key difference between the winged split carriage instrument driver and the non-winged embodiments described herein.

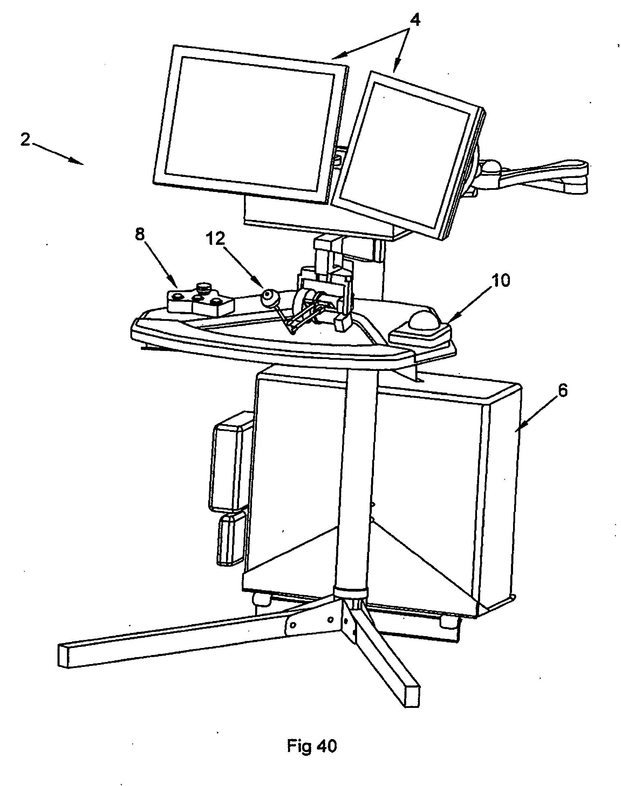

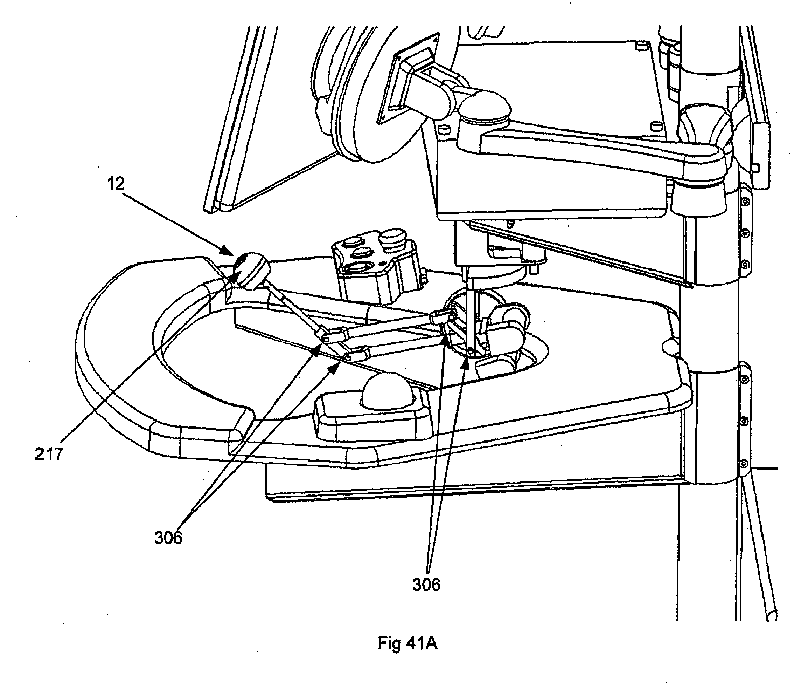

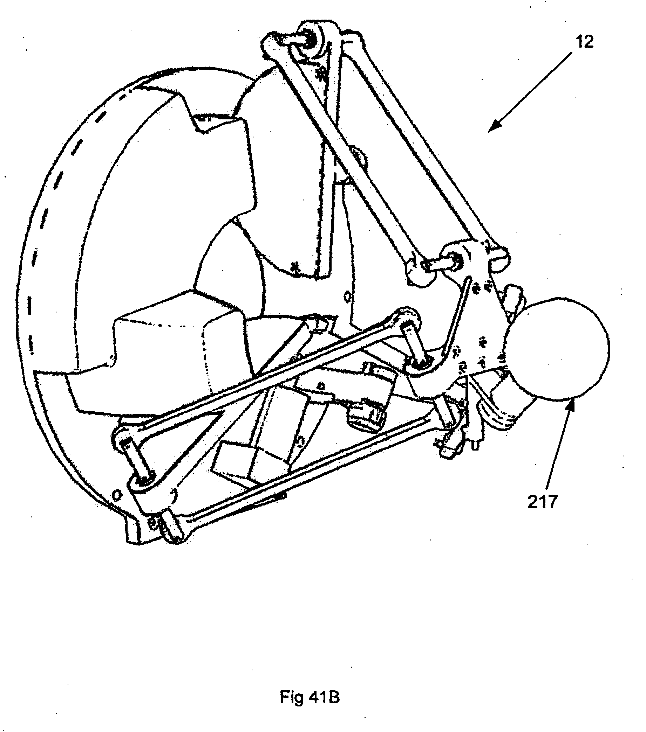

Referring to Figure 40, an operator control station is depicted showing a control button console (8), a computer (6), a computer control interface (10), such as a mouse, a visual display system (4) and a master input device (12). In addition to "buttons" on the button console (8) footswitches and other known user control interfaces may be utilized to provide an operator interface with the system controls. Referring to Figure 41A, in one embodiment, the master input device (12) is a multi-degree-of-freedom device having multiple joints and associated encoders (306). An operator interface (217) is configured for comfortable interfacing with the human fingers. The depicted embodiment of the operator interface (217) is substantially spherical. Further, the master input device may have integrated haptics capability for providing tactile feedback to the user. Another embodiment of a master input device (12) is depicted in Figure 41B having a similarly-shaped operator interface (217). Suitable master input devices are available from manufacturers such as Sensible Devices Corporation under the trade name "Phantom™", or Force Dimension under the trade name "Omega™". In one embodiment featuring an Omega-type master input device, the motors of the master input device are utilized for gravity compensation. In other words, when the operator lets go of the master input device with his hands, the master input device is configured to stay in position, or hover around the point at which is was left, or another predetermined point, without gravity taking the handle of the master input device to the portion of the master input device's range of motion closest to the center of the earth. In another embodiment, haptic feedback is utilized to provide feedback to the operator that he has reached the limits of the pertinent instrument workspace. In another embodiment, haptic feedback is utilized to provide feedback to the operator that he has reached the limits of the subject tissue workspace when such workspace has been registered to the workspace of the instrument (i.e., should the operator be navigating a tool such as an ablation tip with a guide instrument through a 3-D model of a heart imported, for example, from CT data of an actual heart, the master input device is configured to provide haptic feedback to the operator that he has reached a wall or other structure of the heart as per the data of the 3-D model, and therefore help prevent the operator from driving the tool through such wall or structure without at least feeling the wall or structure through the master input device). In another embodiment, contact sensing technologies configured to detect contact between an instrument and tissue may be utilized in conjunction with the haptic capability of the master input device to signal the operator that the instrument is indeed in contact with tissue.

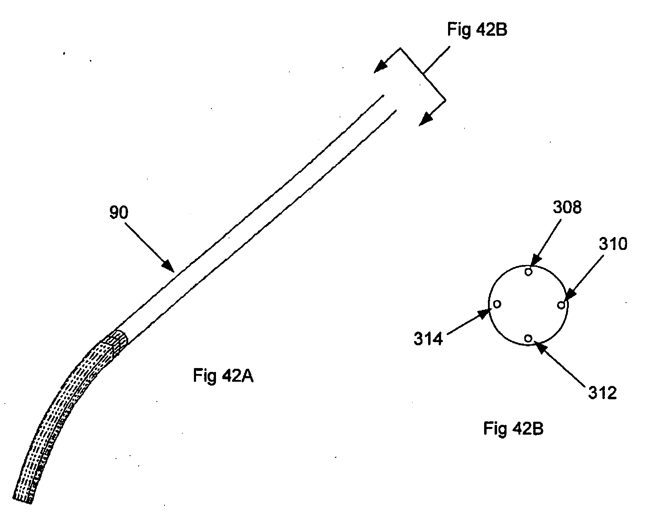

Referring to Figures 42-45, the basic kinematics of a catheter with four control elements is reviewed.

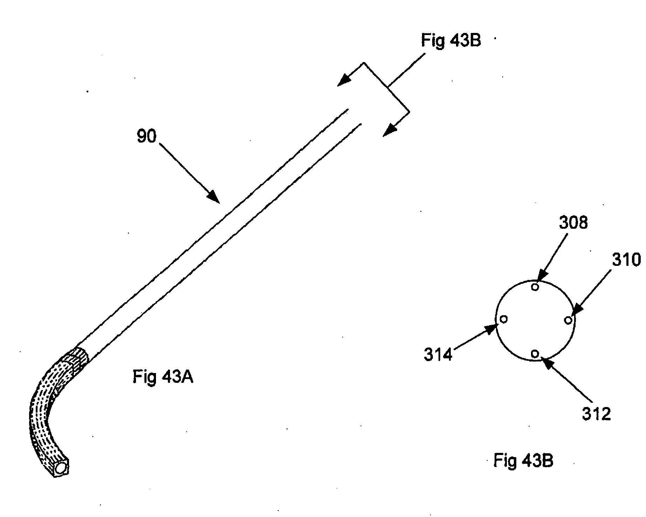

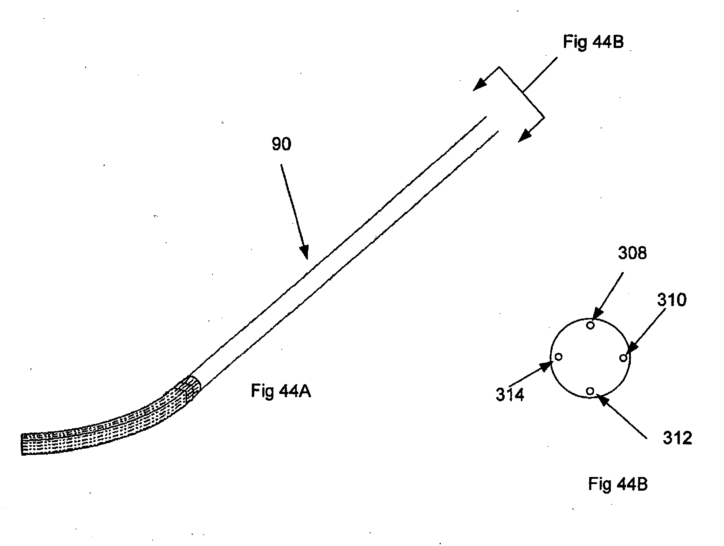

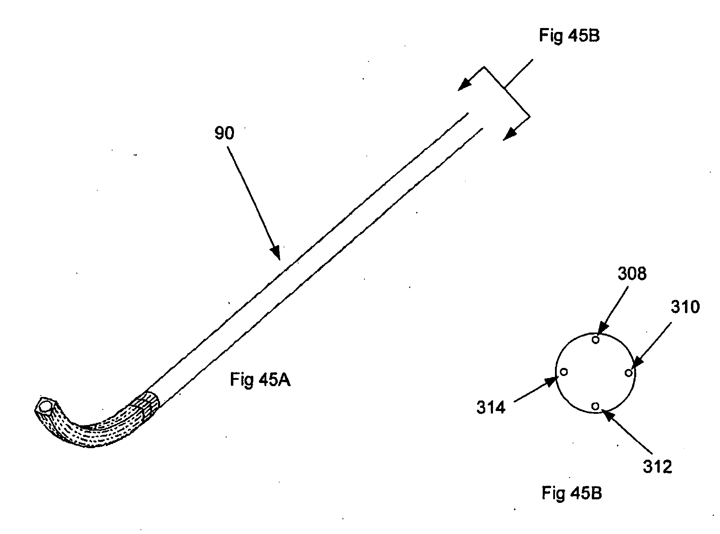

Referring to Figures 42 A-B, as tension is placed only upon the bottom control element (312), the catheter bends downward, as shown in Figure 42A. Similarly, pulling the left control element (314) in Figures 43 A-B bends the catheter left, pulling the right control element (310) in Figures 44 A-B bends the catheter right, and pulling the top control element (308) in Figures 45 A-B bends the catheter up. As will be apparent to those skilled in the art, well-known combinations of applied tension about the various control elements results in a variety of bending configurations at the tip of the catheter member (90). One of the challenges in accurately controlling a catheter or similar elongate member with tension control elements is the retention of tension in control elements, which may not be the subject of the majority of the tension loading applied in a particular desired bending configuration. If a system or instrument is controlled with various levels of tension, then losing tension, or having a control element in a slack configuration, can result in an unfavorable control scenario.

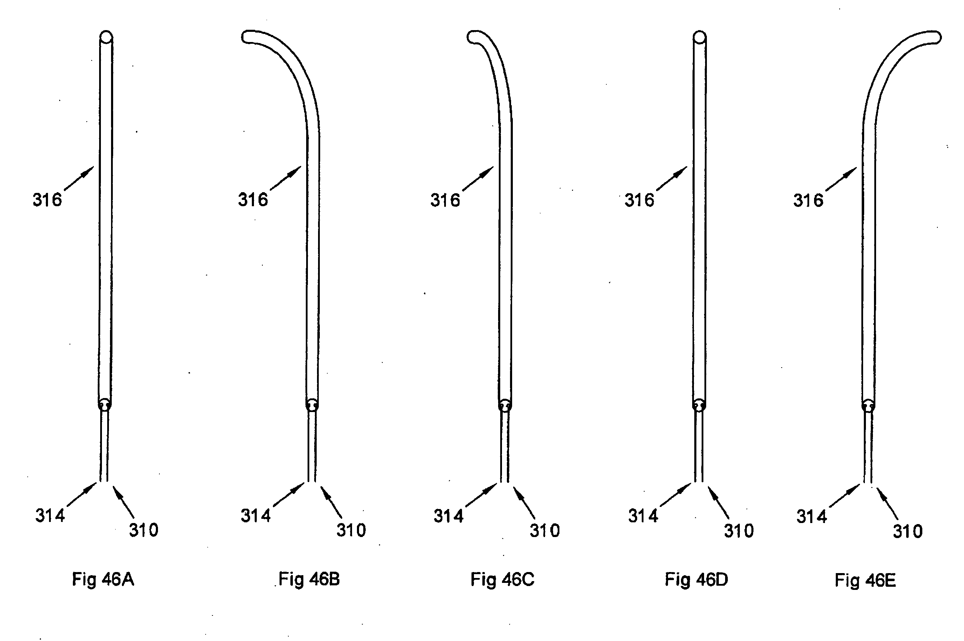

Referring to Figures 46A-E, a simple scenario is useful in demonstrating this notion. As shown in Figure 46A, a simple catheter (316) steered with two control elements (314, 310) is depicted in a neutral position. If the left control element (314) is placed into tension greater than the tension, if any, which the right control element (310) experiences, the catheter (316) bends to the left, as shown in Figure 46B. If a change of direction is desired, this paradigm needs to reverse, and the tension in the right control element (310) needs to overcome that in the left control element (314). At the point of a reversal of direction like this, where the tension balance changes from left to right, without slack or tension control, the right most control element (314) may gather slack which needs to be taken up before precise control can be reestablished. Subsequent to a "reeling in" of slack which may be present, the catheter (316) may be may be pulled in the opposite direction, as depicted in Figures 46C-E, without another slack issue from a controls perspective until a subsequent change in direction.

The above-described instrument embodiments present various techniques for managing tension control in various guide instrument systems having between two and four control elements. For example, in one set of embodiments, tension may be controlled with active independent tensioning of each control element in the pertinent guide catheter via independent control element interface assemblies (132) associated with independently-controlled guide instrument interface sockets (270) on the instrument driver (16). Thus, tension may be managed by independently actuating each of the control element interface assemblies (132) in a four-control-element embodiment, a three-control-element embodiment, or a two-control-element embodiment.

In another set of embodiments, tension may be controlled with active independent tensioning with a split carriage design, as described in reference to Figures 37 and 38. For example, a split carriage with two independent linearly movable portions, such as that depicted in Figure 38, may be utilized to actively and independently tension each of two control element interface assemblies, each of which is associated with two dimensions of a given degree of freedom. For example, there can be + and - pitch on one interface assembly, + and - yaw on the other interface assembly, with slack or tension control provided for pitch by one of the linearly movable portions (302) of the split carriage (296), and slack or tension control provided for yaw by the other linearly movable portion (302) of the split carriage (296).

Similarly, slack or tension control for a single degree of freedom, such as yaw or pitch, may be provided by a single-sided split carriage design similar to that of Figure 38, with the exception that only one linearly movable portion would be required to actively tension the single control element interface assembly of an instrument.

In another set of embodiments, tensioning may be controlled with spring-loaded idlers configured to keep the associated control elements out of slack. The control elements preferably are pre-tensioned in each embodiment to prevent slack and provide predictable performance. Indeed, in yet another set of embodiments, pre-tensioning may form the main source of tension management. In the case of embodiments only having pre-tensioning or spring-loaded idler tensioning, the control system may need to be configured to reel in bits of slack at certain transition points in catheter bending, such as described above in relation to Figures 46A and 46B.

To accurately coordinate and control actuations of various motors within an instrument driver from a remote operator control station such as that depicted in Figure 1, an advanced computerized control and visualization system is preferred. While the control system embodiments that follow are described in reference to a particular control systems interface, namely the SimuLink™ and XPC™ control interfaces available from The Mathworks Inc., and PC-based computerized hardware configurations, many other configurations may be utilized, including various pieces of specialized hardware, in place of more flexible software controls running on PC-based systems.

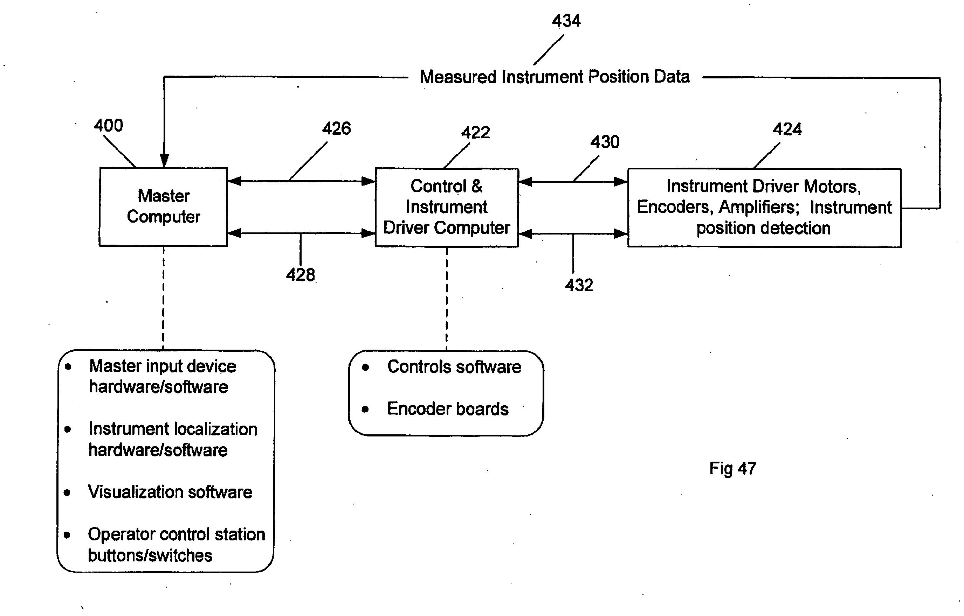

Referring to Figure 47, an overview of an embodiment of a controls system flow is depicted. A master computer (400) running master input device software, visualization software, instrument localization software, and software to interface with operator control station buttons and/or switches is depicted. In one embodiment, the master input device software is a proprietary module packaged with an off-the-shelf master input device system, such as the Phantom™ from Sensible Devices Corporation, which is configured to communicate with the Phantom™ hardware at a relatively high frequency as prescribed by the manufacturer. Other suitable master input devices, such as that (12) depicted in Figure 41B are available from suppliers such as Force Dimension of Lausanne, Switzerland. The master input device (12) may also have haptics capability to facilitate feedback to the operator, and the software modules pertinent to such functionality may also be operated on the master computer (400). Preferred embodiments of haptics feedback to the operator are discussed in further detail below.

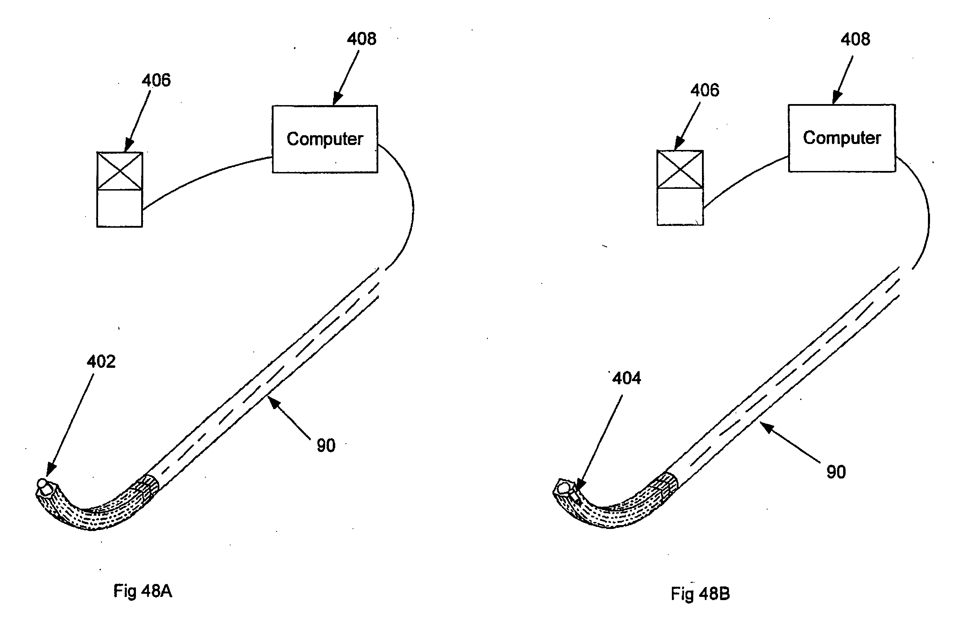

The term "localization" is used in the art in reference to systems for determining and/or monitoring the position of objects, such as medical instruments, in a reference coordinate system. In one embodiment, the instrument localization software is a proprietary module packaged with an off the-shelf or custom instrument position tracking system, such as those available from Ascension Technology Corporation, Biosense Webster, Inc., Endocardial Solutions, Inc., Boston Scientific (EP Technologies), Medtronic, Inc., and others. Such systems may be capable of providing not only real-time or near real-time positional information, such as X-Y-Z coordinates in a Cartesian coordinate system, but also orientation information relative to a given coordinate axis or system. Some of the commercially-available localization systems use electromagnetic relationships to determine position and/or orientation, while others, such as some of those available from Endocardial Solutions, Inc. -St Jude Medical, utilize potential difference or voltage, as measured between a conductive sensor located on the pertinent instrument and conductive portions of sets of patches placed against the skin, to determine position and/or orientation. Referring to Figures 48A and 48B, various localization sensing systems may be utilized with the various embodiments of the robotic catheter system disclosed herein. In other embodiments not comprising a localization system to determine the position of various components, kinematic and/or geometric relationships between various components of the system may be utilized to predict the position of one component relative to the position of another. Some embodiments may utilize both localization data and kinematic and/or geometric relationships to determine the positions of various components.

As shown in Figure 48A, one preferred localization system comprises an electromagnetic field transmitter (406) and an electromagnetic field receiver (402) positioned within the central lumen of a guide catheter (90). The transmitter (406) and receiver (402) are interfaced with a computer operating software configured to detect the position of the detector relative to the coordinate system of the transmitter (406) in real or near-real time with high degrees of accuracy. Referring to Figure 48B, a similar embodiment is depicted with a receiver (404) embedded within the guide catheter (90) construction. Preferred receiver structures may comprise three or more sets of very small coils spatially configured to sense orthogonal aspects of magnetic fields emitted by a transmitter. Such coils may be embedded in a custom configuration within or around the walls of a preferred catheter construct. For example, in one embodiment, two orthogonal coils are embedded within a thin polymeric layer at two slightly flattened surfaces of a catheter (90) body approximately ninety degrees orthogonal to each other about the longitudinal axis of the catheter (90) body, and a third coil is embedded in a slight polymer-encapsulated protrusion from the outside of the catheter (90) body, perpendicular to the other two coils. Due to the very small size of the pertinent coils, the protrusion of the third coil may be minimized. Electronic leads for such coils may also be embedded in the catheter wall, down the length of the catheter body to a position, preferably adjacent an instrument driver, where they may be routed away from the instrument to a computer running localization software and interfaced with a pertinent transmitter.

In another similar embodiment (not shown), one or more conductive rings may be electronically connected to a potential-difference-based localization/orientation system, along with multiple sets, preferably three sets, of conductive skin patches, to provide localization and/or orientation data utilizing a system such as those available from Endocardial Solutions - St. Jude Medical. The one or more conductive rings may be integrated into the walls of the instrument at various longitudinal locations along the instrument, or set of instruments. For example, a guide instrument may have several conductive rings longitudinally displaced from each other toward the distal end of the guide instrument, while a coaxially-coupled sheath instrument may similarly have one or more conductive rings longitudinally displaced from each other toward the distal end of the sheath instrument - to provide precise data regarding the location and/or orientation of the distal ends of each of such instruments.

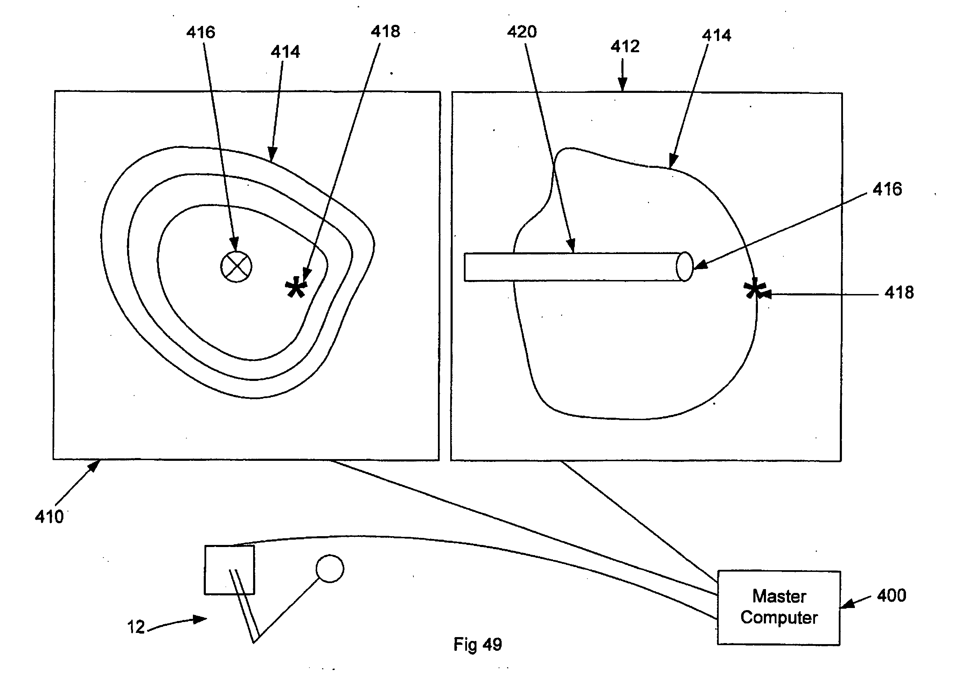

Referring back to Figure 47, in one embodiment, visualization software runs on the master computer (400) to facilitate real-time driving and navigation of one or more steerable instruments. In one embodiment, visualization software provides an operator at an operator control station, such as that depicted in Figure 1 (2), with a digitized "dashboard" or "windshield" display to enhance instinctive drivability of the pertinent instrumentation within the pertinent tissue structures. Referring to Figure 49, a simple illustration is useful to explain one embodiment of a preferred relationship between visualization and navigation with a master input device (12). In the depicted embodiment, two display views (410, 412) are shown. One preferably represents a primary (410) navigation view, and one may represent a secondary (412) navigation view. To facilitate instinctive operation of the system, it is preferable to have the master input device coordinate system at least approximately synchronized with the coordinate system of at least one of the two views. Further, it is preferable to provide the operator with one or more secondary views which may be helpful in navigating through challenging tissue structure pathways and geometries.

Using the operation of an automobile as an example, if the master input device is a steering wheel and the operator desires to drive a car in a forward direction using one or more views, his first priority is likely to have a view straight out the windshield, as opposed to a view out the back window, out one of the side windows, or from a car in front of the car that he is operating. The operator might prefer to have the forward windshield view as his primary display view, such that a right turn on the steering wheel takes him right as he observes his primary display, a left turn on the steering wheel takes him left, and so forth. If the operator of the automobile is trying to park the car adjacent another car parked directly in front of him, it might be preferable to also have a view from a camera positioned, for example, upon the sidewalk aimed perpendicularly through the space between the two cars (one driven by the operator and one parked in front of the driven car), so the operator can see the gap closing between his car and the car in front of him as he parks. While the driver might not prefer to have to completely operate his vehicle with the sidewalk perpendicular camera view as his sole visualization for navigation purposes, this view is helpful as a secondary view.

Referring still to Figure 49, if an operator is attempting to navigate a steerable catheter in order to, for example, contact a particular tissue location with the catheter's distal tip, a useful primary navigation view (410) may comprise a three dimensional digital model of the pertinent tissue structures (414) through which the operator is navigating the catheter with the master input device (12), along with a representation of the catheter distal tip location (416) as viewed along the longitudinal axis of the catheter near the distal tip. This embodiment illustrates a representation of a targeted tissue structure location (418), which may be desired in addition to the tissue digital model (414) information. A useful secondary view (412), displayed upon a different monitor, in a different window upon the same monitor, or within the same user interface window, for example, comprises an orthogonal view depicting the catheter tip representation (416), and also perhaps a catheter body representation (420), to facilitate the operator's driving of the catheter tip toward the desired targeted tissue location (418).

In one embodiment, subsequent to development and display of a digital model of pertinent tissue structures, an operator may select one primary and at least one secondary view to facilitate navigation of the instrumentation. By selecting which view is a primary view, the user can automatically toggle a master input device (12) coordinate system to synchronize with the selected primary view. In an embodiment with the leftmost depicted view (410) selected as the primary view, to navigate toward the targeted tissue site (418), the operator should manipulate the master input device (12) forward, to the right, and down. The right view will provide valued navigation information, but will not be as instinctive from a "driving" perspective.

To illustrate: if the operator wishes to insert the catheter tip toward the targeted tissue site (418) watching only the rightmost view (412) without the master input device (12) coordinate system synchronized with such view, the operator would have to remember that pushing straight ahead on the master input device will make the distal tip representation (416) move to the right on the rightmost display (412). Should the operator decide to toggle the system to use the rightmost view (412) as the primary navigation view, the coordinate system of the master input device (12) is then synchronized with that of the rightmost view (412), enabling the operator to move the catheter tip (416) closer to the desired targeted tissue location (4T8) by manipulating the master input device (12) down and to the right.

The synchronization of coordinate systems described herein may be conducted using fairly conventional mathematic relationships. For example, in one embodiment, the orientation of the distal tip of the catheter may be measured using a 6-axis position sensor system such as those available from Ascension Technology Corporation, Biosense Webster, Inc., Endocardial Solutions, Inc., Boston Scientific (EP Technologies), and others. A 3-axis coordinate frame, C, for locating the distal tip of the catheter, is constructed from this orientation information. The orientation information is used to construct the homogeneous transformation matrix, $T_{C0}^{G0}$, which transforms a vector in the Catheter coordinate frame "C" to the fixed Global coordinate frame "G" in which the sensor measurements are done (the subscript $C_0$ and superscript Go are used to represent the 0'th, or initial, step). As a registration step, the computer graphics view of the catheter is rotated until the master input and the computer graphics view of the catheter distal tip motion are coordinated and aligned with the camera view of the graphics scene. The 3-axis coordinate frame transformation matrix $T_{Gref}^{G0}$ for the camera position of this initial view is stored (subscripts $G_{ref}$ and superscript $C_{ref}$ stand for the global and camera "reference" views). The corresponding catheter "reference view" matrix for the catheter coordinates is obtained as:

$$T_{Cref}^{C0} = T_{G0}^{C0} T_{Gref}^{G0} T_{Cref}^{Gref} = (T_{C0}^{G0})^{-1} T_{Gref}^{G0} T_{C1}^{G1}$$

Also note that the catheter's coordinate frame is fixed in the global reference frame G, thus the transformation matrix between the global frame and the catheter frame is the same in all views, i.e., $T_{C0}^{G0} = T_{Cref}^{Gref} = T_{Ci}^{Gi}$ for any arbitrary view $i$.

The coordination between primary view and master input device coordinate systems is achieved by transforming the master input as follows: Given any arbitrary computer-graphics view of the representation, e.g. the $i$th view, the 3-axis coordinate frame transformation matrix $T_{Gi}^{G0}$ of the *camera* view of the computer graphics scene is obtained form the computer graphics software. The corresponding *catheter* transformation matrix is computed in a similar manner as above:

$$T_{Ci}^{C0} = T_{G0}^{C0} T_{Gi}^{G0} T_{Ci}^{Gi} = (T_{C0}^{G0})^{-1} T_{Gi}^{G0} T_{Ci}^{Gi}$$

The transformation that needs to be applied to the master input which achieves the view coordination is the one that transforms from the *reference* view that was registered above, to the current ith view, i.e., $T_{Cref}^{Ci}$. Using the previously computed quantities above, this transform is computed as:

$$T_{Cref}^{Ci} = T_{C0}^{Ci} T_{Cref}^{C0}$$

The master input is transformed into the commanded catheter input by application of the transformation $T_{Cref}^{Ci}$. Given a command input

$$r_{master} = \begin{bmatrix} x_{master} \\ y_{master} \\ y_{master} \end{bmatrix},$$

one may calculate:

$$r_{catheter} = \begin{bmatrix} x_{catheter} \\ y_{catheter} \\ y_{catheter} \end{bmatrix} = T_{Cref}^{Ci} \begin{bmatrix} x_{master} \\ y_{master} \\ y_{master} \end{bmatrix}.$$

Under such relationships, coordinate systems of the primary view and master input device may be aligned for instinctive operation.

Referring back to embodiment of Figure 111, the master computer (400) also comprises software and hardware interfaces to operator control station buttons, switches, and other input devices which may be utilized, for example, to "freeze" the system by functionally disengaging the master input device as a controls input, or provide toggling between various scaling ratios desired by the operator for manipulated inputs at the master input device (12). The master computer (400) has two separate functional connections with the control and instrument driver computer (422): one (426) for passing controls and visualization related commands, such as desired XYZ) in the catheter coordinate system) commands, and one (42.8) for passing safety signal commands. Similarly, the control and instrument driver computer (422) has two separate functional connections with the instrument and instrument driver hardware (424): one (430) for passing control and visualization related commands such as required-torque-related voltages to the amplifiers to drive the motors and encoders, and one (432) for passing safety signal commands.

In one embodiment, the safety signal commands represent a simple signal repeated at very short intervals, such as every 10 milliseconds, such signal chain being logically read as "system is ok, amplifiers stay active". If there is any interruption in the safety signal chain, the amplifiers are logically toggled to inactive status and the instrument cannot be moved by the control system until the safety signal chain is restored. Also shown in the signal flow overview of Figure 47 is a pathway (434) between the physical instrument and instrument driver hardware back to the master computer to depict a closed loop system embodiment wherein instrument localization technology, such as that described in reference to Figures 48A-B, is utilized to determine the actual position of the instrument to minimize navigation and control error, as described in further detail below.

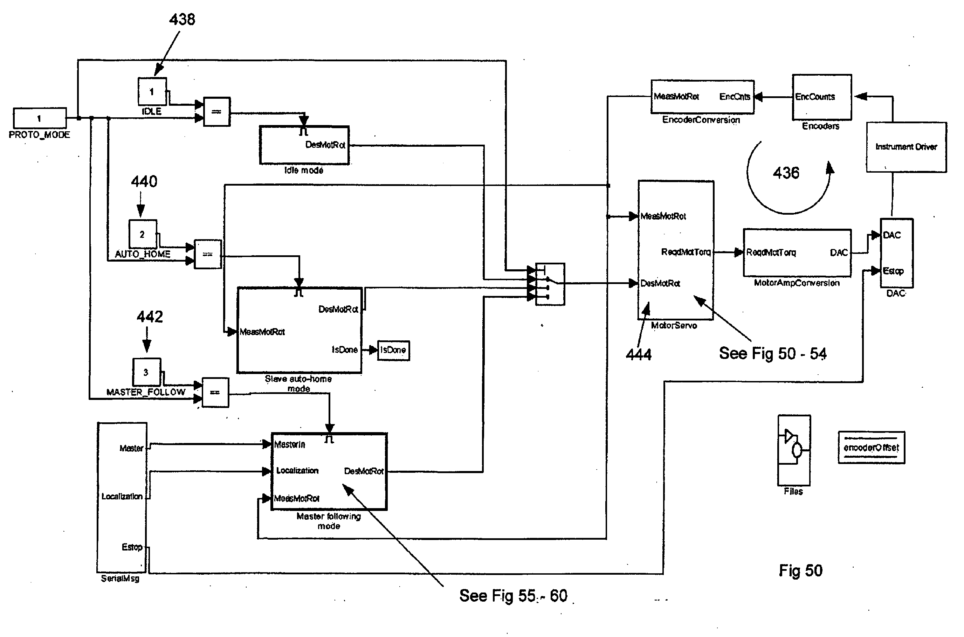

Figures 50-60 depict various aspects of one embodiment of a SimuLink™ software control schema for an embodiment of the physical system, with particular attention to an embodiment of a "master following mode." In this embodiment, an instrument is driven by following instructions from a master input device, and a motor servo loop embodiment, which comprises key operational functionality for executing upon commands delivered from the master following mode to actuate the instrument.

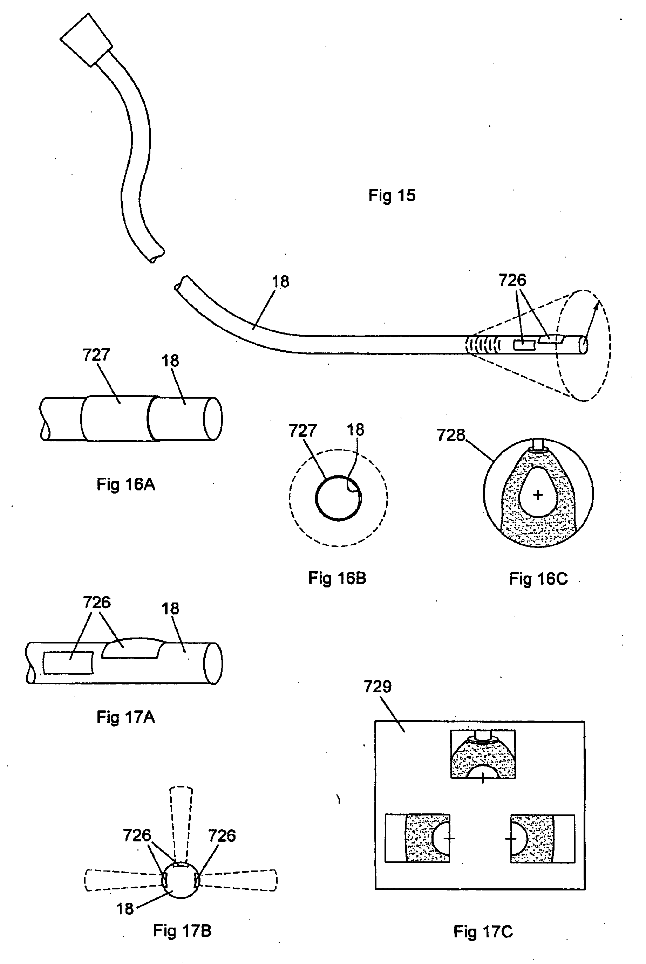

Figure 50 depicts a high-level view of an embodiment wherein any one of three modes may be toggled to operate the primary servo loop (436). In idle mode (438), the default mode when the system is started up, all of the motors are commanded via the motor servo loop (436) to servo about their current positions, their positions being monitored with digital encoders associated with the motors. In other words, idle mode (438) deactivates the motors, while the remaining system stays active. Thus, when the operator leaves idle mode, the system knows the position of the relative components. In auto home mode (440), cable loops within an associated instrument driver, such as that depicted in Figure 32, are centered within their cable loop range to ensure substantially equivalent range of motion of an associated instrument, such as that depicted in Figure 17, in both directions for a various degree of freedom, such as + and - directions of pitch or yaw, when loaded upon the instrument driver. This is a setup mode for preparing an instrument driver before an instrument is engaged.

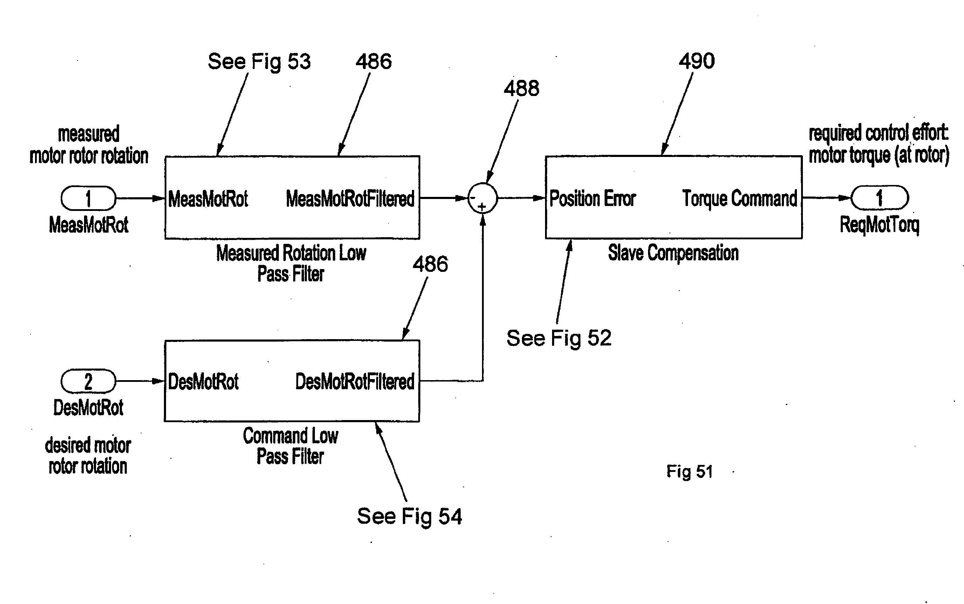

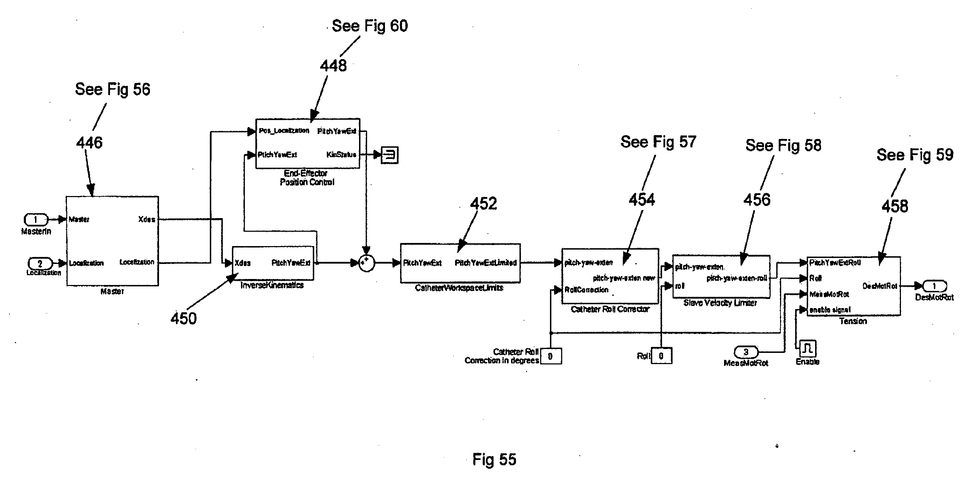

In master following mode (442), the control system receives signals from the master input device, and in a closed loop embodiment from both a master input device and a localization system, and forwards drive signals to the primary servo loop (436) to actuate the instrument in accordance with the forwarded commands. Aspects of this embodiment of the master following mode (442) are depicted in further detail in Figures 55-60. Aspects of the primary servo loop and motor servo block (444) are depicted in further detail in Figures 51-54.

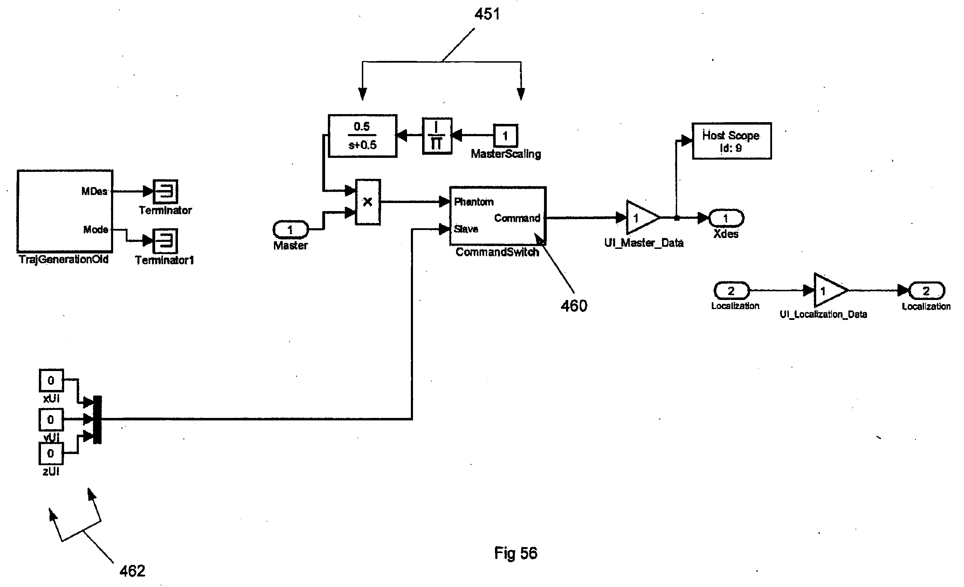

Referring to Figure 55, a more detailed functional diagram of an embodiment of master following mode (442) is depicted. As shown in Figure 55, the inputs to functional block (446) are XYZ position of the master input device in the coordinate system of the master input device which, per a setting in the software of the master input device may be aligned to have the same coordinate system as the catheter, and localization XYZ position of the distal tip of the instrument as measured by the localization system in the same coordinate system as the master input device and catheter. Referring to Figure 56 for a more detailed view of functional block (446) of Figure 55, a switch (460) is provided at block to allow switching between master inputs for desired catheter position, to an input interface (462) through which an operator may command that the instrument go to a particular XYZ location in space. Various controls features may also utilize this interface to provide an operator with, for example, a menu of destinations to which the system should automatically drive an instrument, etc. Also depicted in Figure 56 is a master scaling functional block (451) which is utilized to scale the inputs coming from the master input device with a ratio selectable by the operator. The command switch (460) functionality includes a low pass filter to weight commands switching between the master input device and the input interface (462), to ensure a smooth transition between these modes.

Referring back to Figure 55, desired position data in XYZ terms is passed to the inverse kinematics block (450) for conversion to pitch, yaw, and extension (or "insertion") terms in accordance with the predicted mechanics of materials relationships inherent in the mechanical design of the instrument.

The kinematic relationships for many catheter instrument embodiments may be modeled by applying conventional mechanics relationships. In summary, a control-element-steered catheter instrument is controlled through a set of actuated inputs. In a four-control-element catheter instrument, for example, there are two degrees of motion actuation, pitch and yaw, which both have + and - directions. Other motorized tension relationships may drive other instruments, active tensioning, or insertion or roll of the catheter instrument. The relationship between actuated inputs and the catheter's end point position as a function of the actuated inputs is referred to as the "kinematics" of the catheter.

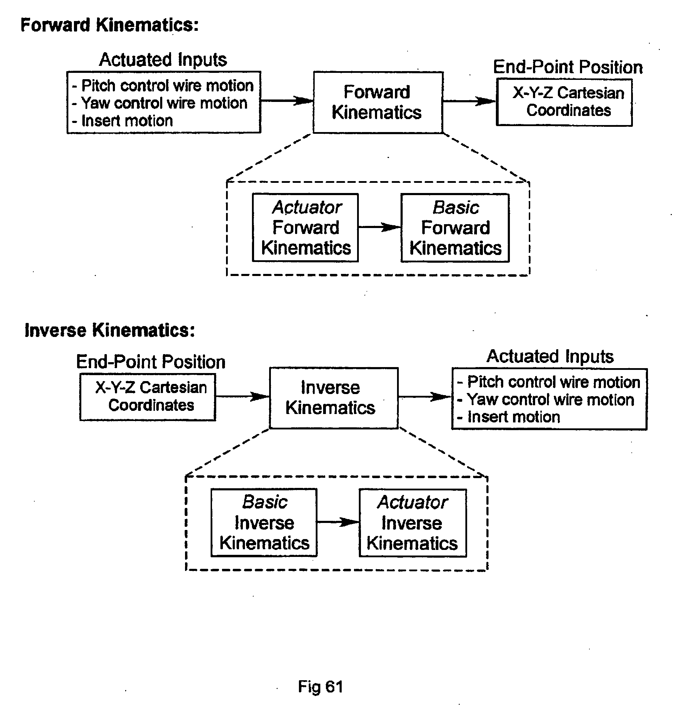

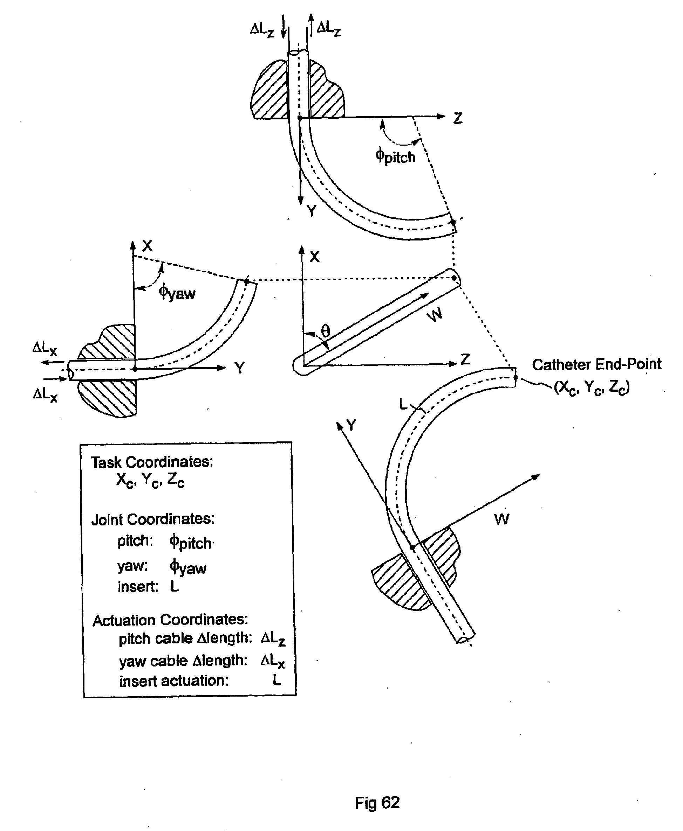

Referring to Figure 61, the "forward kinematics" expresses the catheter's end-point position as a function of the actuated inputs while the "inverse kinematics" expresses the actuated inputs as a function of the desired end-point position. Accurate mathematical models of the forward and inverse kinematics are essential for the control of a robotically controlled catheter system. For clarity, the kinematics equations are further refined to separate out common elements, as shown in Figure 61. The basic kinematics describes the relationship between the task coordinates and the joint coordinates. In such case, the task coordinates refer to the position of the catheter end-point while the joint coordinates refer to the bending (pitch and yaw, for example) and length of the active catheter. The actuator kinematics describes the relationships between the actuation coordinates and the joint coordinates. The task, joint, and bending actuation coordinates for the robotic catheter are illustrated in Figure 62. By describing the kinematics in this way we can separate out the kinematics associated with the catheter structure, namely the basic kinematics, from those associated with the actuation methodology.

The development of the catheter's kinematics model is derived using a few essential assumptions. Included are assumptions that the catheter structure is approximated as a simple beam in bending from a mechanics perspective, and that control elements, such as thin tension wires, remain at a fixed distance from the neutral axis and thus impart a uniform moment along the length of the catheter.

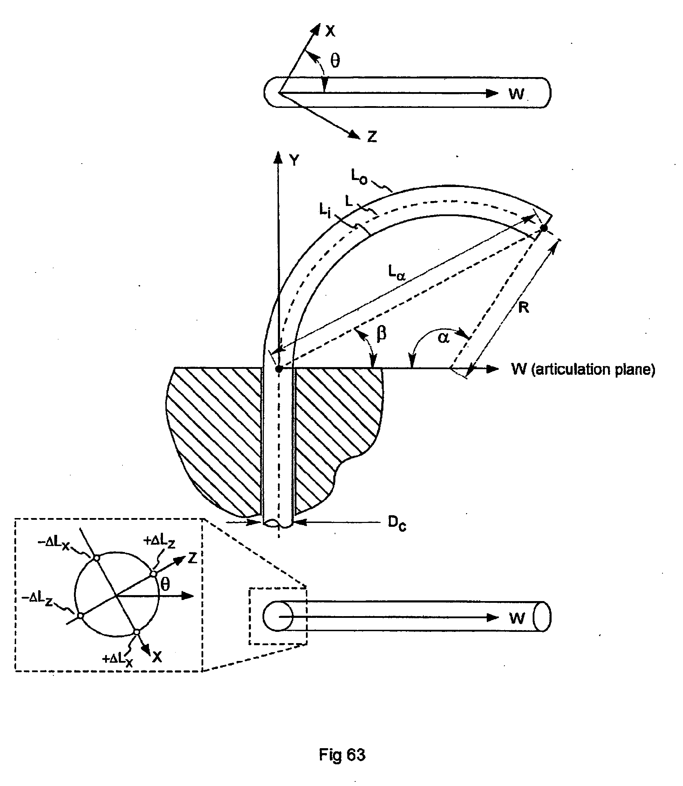

In addition to the above assumptions, the geometry and variables shown in Figure 63 are used in the derivation of the forward and inverse kinematics. The basic forward kinematics, relating the catheter task coordinates ($X_c$, $Y_c$, $Z_c$) to the joint coordinates ($\phi_{pith}$, $\phi_{pitch}$, L), is given as follows:

$$X_c = w \, \cos(\theta)$$

$$Y_c = R \sin(\alpha)$$

$$Z_c = w \sin(\theta)$$

where

$$w = R(1 - \cos(\alpha))$$

$$\alpha = \left[(\phi_{pitch})^2 + (\phi_{yaw})^2\right]^{1/2} \qquad \text{(total bending)}$$

$$R = \frac{L}{\alpha} \qquad \text{(bend radius)}$$

$$\theta = \text{atan2}(\phi_{pitch}, \phi_{yaw}) \qquad \text{(roll angle)}$$

The actuator forward kinematics, relating the joint coordinates ($\phi_{pitch}$, $\phi_{pitch}$, L) to the actuator coordinates ($\Delta L_x$, $\Delta L_z$, L) is given as follows:

$$\phi_{pitch} = \frac{2\Delta L_z}{D_c}$$

$$\phi_{yaw} = \frac{2\Delta L_x}{D_c}$$

As illustrated in Figure 61, the catheter's end-point position can be predicted given the joint or actuation coordinates by using the forward kinematics equations described above.

Calculation of the catheter's actuated inputs as a function of end-point position, referred to as the inverse kinematics, can be performed numerically, using a nonlinear equation solver such as Newton-Raphson. A more desirerable approach, and the one used in this illustrative embodiment, is to develop a closed-form solution which can be used to calculate the required actuated inputs directly from the desired end-point positions.

As with the forward kinematics, we separate the inverse kinematics into the basic inverse kinematics, which relates joint coordinates to the task coordinates, and the actuation inverse kinematics, which relates the actuation coordinates to the joint coordinates. The basic inverse kinematics, relating the joint coordinates ($\phi_{pitch}$, $\phi_{pitch}$, L), to the catheter task coordinates (Xc, Yc, Zc) is given as follows:

$$\phi_{pitch} = \alpha \sin(\theta)$$

$$\phi_{yaw} = \alpha \cos(\theta)$$

$$L = R\alpha$$

$$\rightarrow \quad \text{where} \rightarrow \quad \rightarrow \quad R = \frac{l \sin \beta}{\sin 2\beta} \qquad \rightarrow \quad \begin{array}{l} \overline{\theta = \text{atan2}(Z_c, X_c)} \quad \overline{\beta = \text{atan2}(Y_c, W_c)} \\ W_c = \left( X_c^2 + Z_c^2 \right)^{\frac{1}{2}} \\ l = \left( W_c^2 + Y_c^2 \right)^{\frac{1}{2}} \end{array}$$

$$\alpha = \pi - 2\beta$$

The actuator inverse kinematics, relating the actuator coordinates ($\Delta L_x \Delta L_z L$) to the joint coordinates ($\phi_{pitch}$, $\phi_{pitch}$, L) is given as follows:

$$\Delta L_x = \frac{D_c \phi_{yaw}}{2}$$

$$\Delta L_z = \frac{D_c \phi_{pitch}}{2}$$

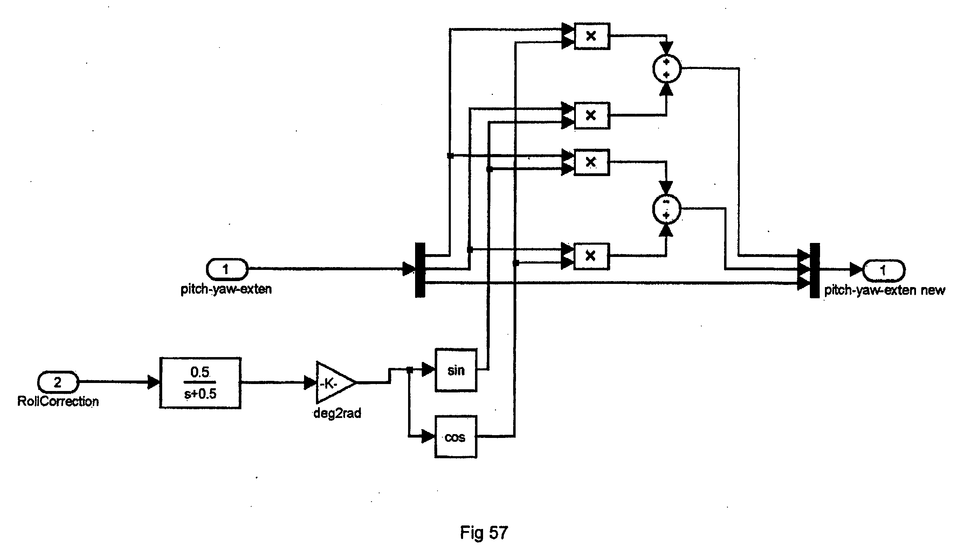

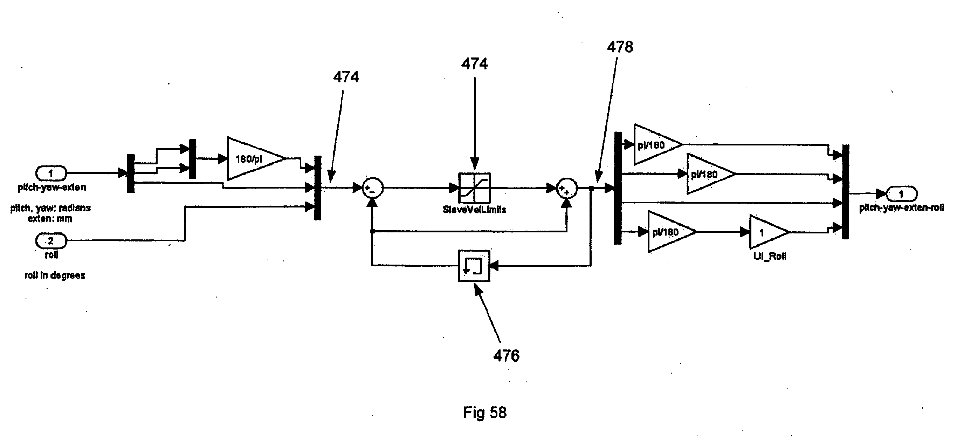

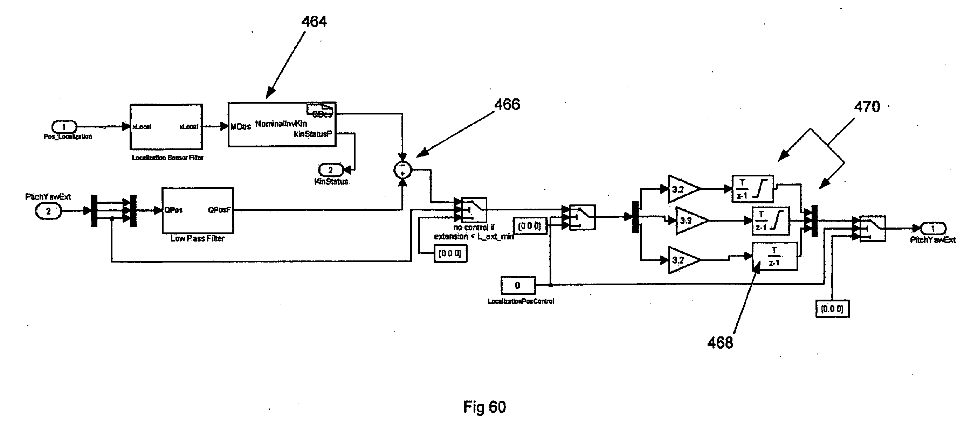

Referring back to Figure 55, pitch, yaw, and extension commands are passed from the inverse kinematics (450) to a position control block (448) along with measured localization data. Figure 60 provides a more detailed view of the position control block (448). After measured , XYZ position data comes in from the localization system, it goes through a inverse kinematics block (464) to calculate the pitch, yaw, and extension the instrument needs to have in order to travel to where it needs to be. Comparing (466) these values with filtered desired pitch, yaw, and extension data from the master input device, integral compensation is then conducted with limits on pitch and yaw to integrate away the error. In this embodiment, the extension variable does not have the same limits (468), as do pitch and yaw (470). As will be apparent to those skilled in the art, having an integrator in a negative feedback loop forces the error to zero. Desired pitch, yaw, and extension commands are next passed through a catheter workspace limitation (452), which may be a function of the experimentally determined physical limits of the instrument beyond which componentry may fail, deform undesirably, or perform unpredictably or undesirably. This workspace limitation essentially defines a volume similar to a cardioid-shaped volume about the distal end of the instrument. Desired pitch, yaw, and extension commands, limited by the workspace limitation block, are then passed to a catheter roll correction block (454).
This functional block is depicted in further detail in Figure 57, and essentially comprises a rotation matrix for transforming the pitch, yaw, and extension commands about the longitudinal, or roll", axis of the instrument - to calibrate the control system for rotational deflection at the distal tip of the catheter that may change the control element steering dynamics. For example, if a catheter has no rotational deflection, pulling on a control element located directly up at twelve o' clock should urge the distal tip of the instrument upward. If, however, the distal tip of the catheter has been rotationally deflected by, say, ninety degrees clockwise, to get an upward response from the catheter, it may be necessary to tension the control element that was originally positioned at a nine o'clock position. The catheter roll correction schema depicted in Figure 57 provides a means for using a rotation matrix to make such a transformation, subject to a roll correction angle, such as the ninety degrees in the above example, which is input, passed through a low pass filter, turned to radians, and put through rotation matrix calculations.
In one embodiment, the roll correction angle is determined through experimental experience with a particular instrument and path of navigation. In another embodiment, the roll correction angle may be determined experimentally in-situ using the accurate orientation data available from the preferred localization systems. In other words, with such an embodiment, a command to, for example, bend straight up can be executed, and a localization system can be utilized to determine at which angle the defection actually went - to simply determine the in-situ roll correction angle.
Referring briefly back to Figure 55, roll corrected pitch and yaw commands, as well as unaffected extension commands, are output from the roll correction block (454) and may optionally be passed to a conventional velocity limitation block (456). Referring to Figure 58, pitch and yaw commands are converted from radians to degrees, and automatically controlled roll may enter the controls picture to complete the current desired position (472) from the last servo cycle. Velocity is calculated by comparing the desired position from the previous servo cycle, as calculated with a conventional memory block (476) calculation, with that of the incoming commanded cycle. A conventional saturation block (474) keeps the calculated velocity within specified values, and the velocity-limited command (478) is converted back to radians and passed to a tension control block (458).

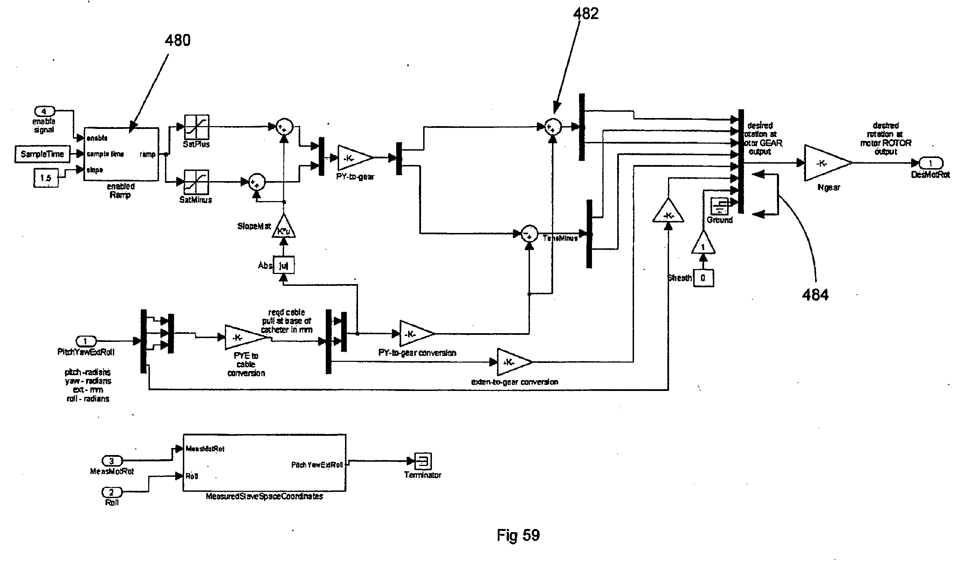

Tension within control elements may be managed depending upon the particular instrument embodiment, as described above in reference to the various instrument embodiments and tension control mechanisms. As an example, Figure 59 depicts a pre-tensioning block (480) with which a given control element tension is ramped to a present value. An adjustment is then added to the original pre-tensioning based upon a preferably experimentally-tuned matrix pertinent to variables, such as the failure limits of the instrument construct and the incoming velocity-limited pitch, yaw, extension, and roll commands. This adjusted value is then added (482) to the original signal for output, via gear ratio adjustment, to calculate desired motor rotation commands for the various motors involved with the instrument movement. In this embodiment, extension, roll, and sheath instrument actuation (484) have no pre-tensioning algorithms associated with their control. The output is then complete from the master following mode functionality, and this output is passed to the primary servo loop (436).

Referring back to Figure 50, incoming desired motor rotation commands from either the master following mode (442), auto home mode (440), or idle mode (438) in the depicted embodiment are fed into a motor servo block (444), which is depicted in greater detail in Figures 51-54.

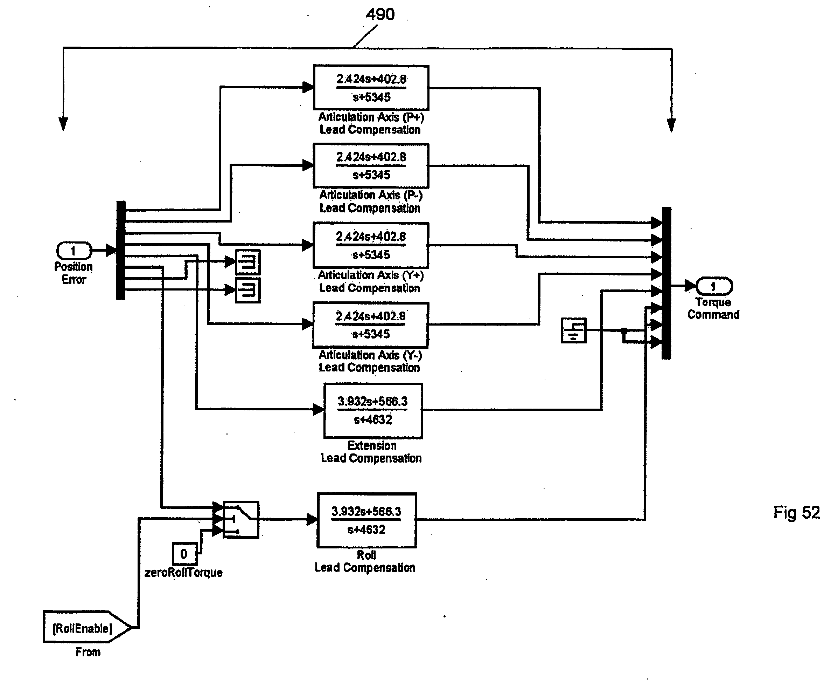

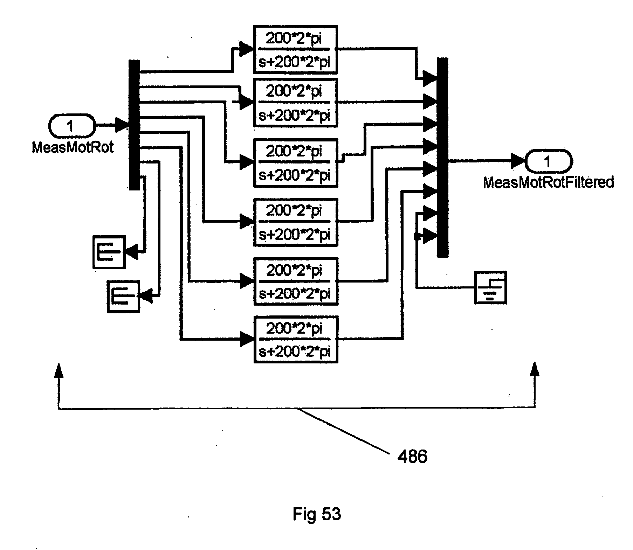

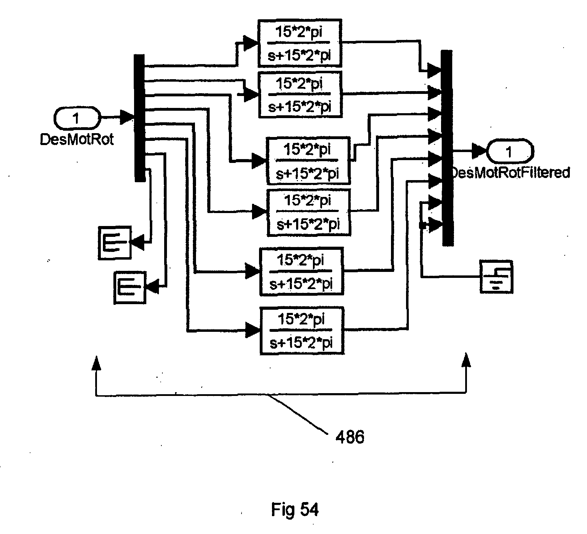

Referring to Figure 51, incoming measured motor rotation data from digital encoders and incoming desired motor rotation commands are filtered using conventional quantization noise filtration at frequencies selected for each of the incoming data streams to reduce noise while not adding undue delays which may affect the stability of the control system. As shown in Figures 53 and 54, conventional quantization filtration is utilized on the measured motor rotation signals at about 200 hertz in this embodiment, and on the desired motor rotation command at about 15 hertz. The difference (488) between the quantization filtered values forms the position error which may be passed through a lead filter, the functional equivalent of a proportional derivative ("PD") + low pass filter. In another embodiment, conventional PID, lead/lag, or state space representation filter may be utilized. The lead filter of the depicted embodiment is shown in further detail in Figure 52.

In particular, the lead filter embodiment in Figure 52 comprises a variety of constants selected to tune the system to achieve desired performance. The depicted filter addresses the needs of one embodiment of a 4-control element guide catheter instrument with independent control of each of four control element interface assemblies for +/- pitch and +/- yaw, and separate roll and extension control. As demonstrated in the depicted embodiment, insertion and roll have different inertia and dynamics as opposed to pitch and yaw controls, and the constants selected to tune them is different. The filter constants may be theoretically calculated using conventional techniques and tuned by experimental techniques, or wholly determined by experimental techniques, such as setting the constants to give a sixty degree or more phase margin for stability and speed of response, a conventional phase margin value for medical control systems.

In an embodiment where a tuned master following mode is paired with a tuned primary servo loop, an instrument and instrument driver, such as those described above, may be "driven" accurately in three-dimensions with a remotely located master input device. Other preferred embodiments incorporate related functionalities, such as haptic feedback to the operator, active tensioning with a split carriage instrument driver, navigation utilizing direct visualization and/or tissue models acquired in-situ and tissue contact sensing, and enhanced navigation logic.

Referring to Figure 64, in one embodiment, the master input device may be a haptic master input device, such as those available from Sensible Devices, Inc., under the trade name Phantom™, and the hardware and software required for operating such a device may at least partially reside on the master computer. The master XYZ positions measured from the master joint rotations and forward kinematics are generally passed to the master computer via a parallel port or similar link and may subsequently be passed to a control and instrument driver computer. With such an embodiment, an internal servo loop for the Phantom™ generally runs at a much higher frequency in the range of 1,000 Hz, or greater, to accurately create forces and torques at the joints of the master.

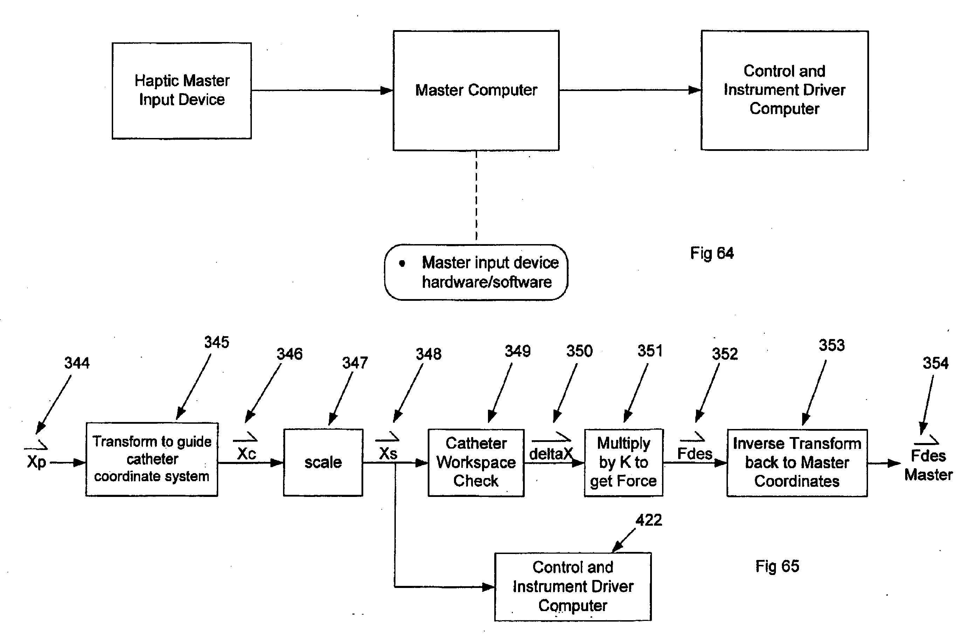

Referring to Figure 65, a sample flowchart of a series of operations leading from a position vector applied at the master input device to a haptic signal applied back at the operator is depicted. A vector (344) associated with a master input device move by an operator may be transformed into an instrument coordinate system, and in particular to a catheter instrument tip coordinate system, using a simple matrix transformation (345). The transformed vector (346) may then be scaled (347) per the preferences of the operator, to produce a scaled-transformed vector (348). The scaled-transformed vector (348) may be sent to both the control and instrument driver computer (422) preferably via a serial wired connection, and to the master computer for a catheter workspace check (349) and any associated vector modification (350). this is followed by a feedback constant multiplication (351) chosen to produce preferred levels of feedback, such as force, in order to produce a desired force vector (352), and an inverse transform (353) back to the master input device coordinate system for associated haptic signaling to the operator in that coordinate system (354).

A conventional Jacobian may be utilized to convert a desired force vector (352) to torques desirably applied at the various motors comprising the master input device, to give the operator a desired signal pattern at the master input device. Given this embodiment of a suitable signal and execution pathway, feedback to the operator in the form of haptics, or touch sensations, may be utilized in various ways to provide added safety and instinctiveness to the navigation features of the system, as discussed in further detail below.

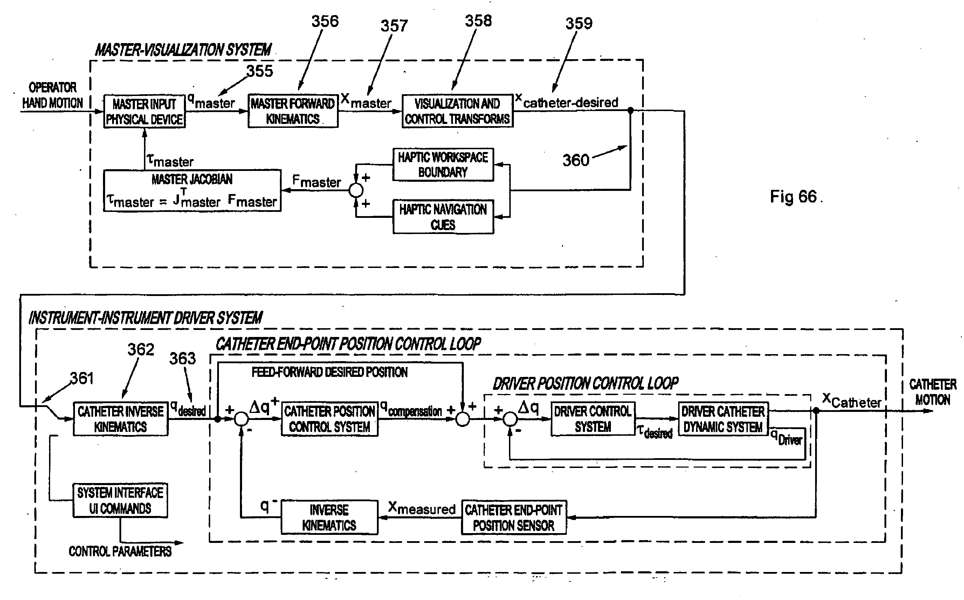

Figure 66 is a system block diagram including haptics capability. As shown in summary form in Figure 66, encoder positions on the master input device, changing in response to motion at the master input device, are measured (355), sent through forward kinematics calculations (356) pertinent to the master input device to get XYZ spatial positions of the device in the master input device coordinate system (357), then transformed (358) to switch into the catheter coordinate system and (perhaps) transform for visualization orientation and preferred controls orientation, to facilitate "instinctive driving."

The transformed desired instrument position (359) may then be sent down one or more controls pathways to, for example, provide haptic feedback (360) regarding workspace boundaries or navigation issues, and provide a catheter instrument position control loop (361) with requisite catheter desired position values, as transformed utilizing inverse kinematics relationships for the particular instrument (362) into yaw, pitch, and extension, or "insertion", terms (363) pertinent to operating the particular catheter instrument with open or closed loop control.

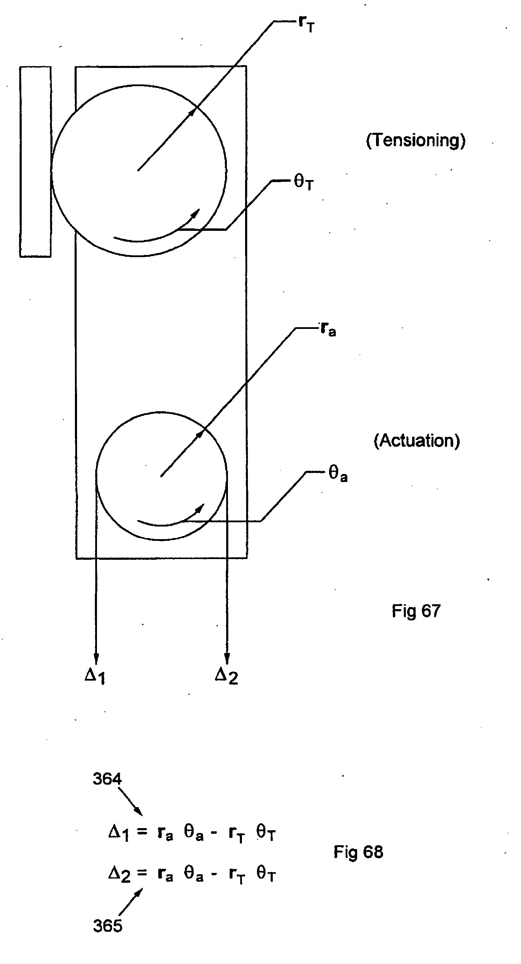

Referring to Figures 67-72, relationships pertinent to tension control via a split carriage design such as that depicted in Figures 37-38 are depicted to illustrate that such a design may isolate tension control from actuation for each associated degree of freedom, such as pitch or yaw of a steerable catheter instrument.

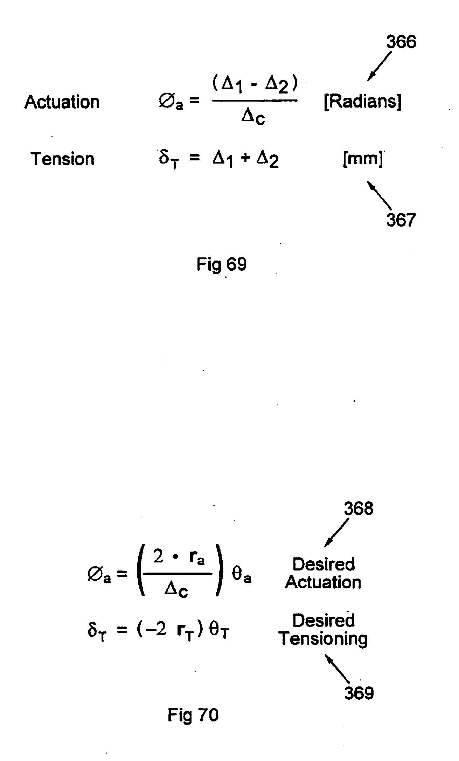

Referring to Figure 67, some of the structures associated with a split carriage design, such as the embodiments depicted in Figures 37 and 38, include a linearly movable portion (302), a guide instrument interface socket (270), a gear (300), and a rack (298). Applying conventional geometric relationships to the physical state of the structures related in Figure 67, the equations (364, 365) of Figure 68 may be generated. Utilizing foreward kinematics of the instrument, such as those described above in reference to a pure cantilever bending model for a catheter instrument, the relationships of Figure 69 may be developed for the amount of bending as a function of cable pull and catheter diameter ("Dc") (366), and for tension (367), defined as the total amount of common pull in the control elements. Combining the equations of Figure 68 and 69, one arrives at the relationships (368, 369) depicted in Figure 70, wherein desired actuation and desired tensioning are decoupled by the mechanics of the involved structures. Desired actuation (368) of the guide instrument interface socket (270) depicted in Figure 67 is a function of the socket's angular rotational position. Desired tensioning (369) of the associated control elements is a function of the position of the tensioning gear (300) versus the rack (298).

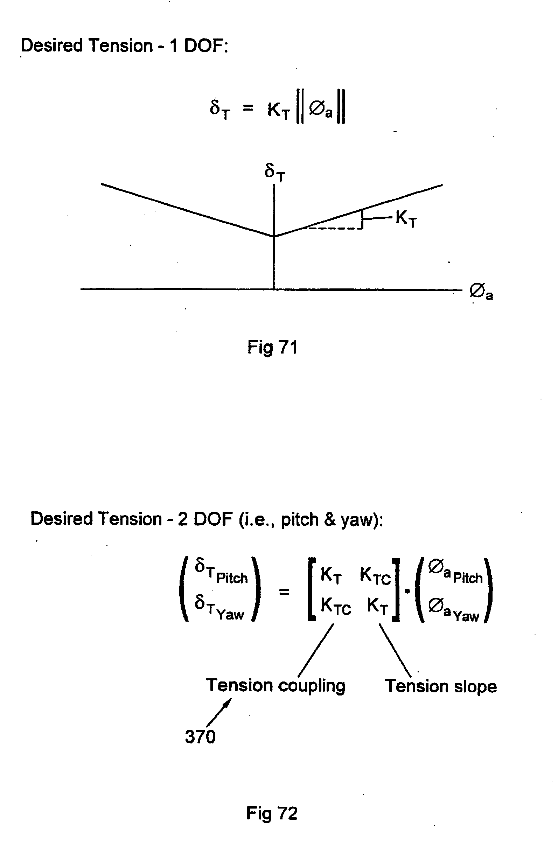

Referring to Figure 71, with a single degree of freedom actuated, such as +/- pitch or +/yaw, and active tensioning via a split carriage mechanism, desired tension is linearly related to the absolute value of the amount of bending, as one would predict per the discussion above in reference to Figures 46A-E. The prescribed system never goes into slack - desired tension is always positive, as shown in Figure 71. Referring to Figure 72, a similar relationship applies for a two degree of freedom system with active tensioning - such as a four-cable system with +/pitch and +/- yaw as the active degrees of freedom and active tensioning via a split carriage design. Since there are two dimensions, coupling terms (370) are incorporated to handle heuristic adjustments to, for example, minimize control element slacking and total instrument compression.

As discussed in reference to Figure 49, in one embodiment, a tissue structure model (414) may be utilized to enhance navigation. It is particularly desirable to utilize actual data, acquired in situ, from the patient onto which a procedure is to be conducted, due to anatomic variation among the patient population which may be significant, depending generally upon the subject tissue structures. For example, the geometry of the left atrium of the human heart varies significantly from patient to patient, according to published reports and experimental verification in animals.

In one embodiment, focused magnetic resonance imaging, gated for heart cycle motion, and preferably gated for respiratory cycle motion, may be utilized along with conventional image cropping and thresholding techniques to produce a three dimensional tissue structure model. One of the challenges with such an imaging modality as applied to modeling active tissue structures such as those of the heart is the gating. In one embodiment, the gating comprises waiting for cardiac resting periods during diastole which are also correlated to substantially limited respiratory-induced motion. Acquiring a three-dimensional image of a left atrium, for example, utilizing gated magnetic resonance, may require an unacceptable amount of acquisition time, not to mention the generally large and expensive instrumentation required to accomplish the acquisition and fusion into a usable tissue structure model. Such a modality, however, may be preferred where cardiac and/or respiratory cyclic motion is negligible, and wherein an image or series of images may be acquired and synthesized into a usable tissue structure model comparatively quickly.

In a generic form, the aforementioned "master following mode" may be logically configured to follow directly each command as it comes through the control system from the master input device. In one closed loop control embodiment, however, a logic layer is configured to interpret data incoming from a master input device and a localization system in light of the integrated tissue structure model and certain system settings information pertinent to the particular procedure at hand, to make modifications to commands forwarded to the master following and subsequent main servo loop controls logic, resulting in movements of the physical instrument.

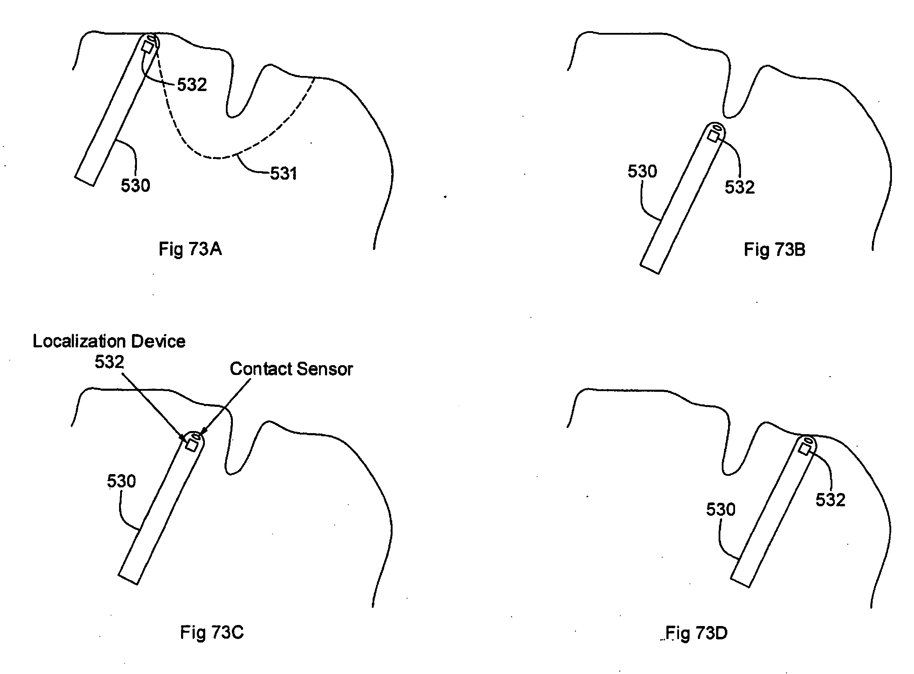

Referring to Figures 73-75, some relatively simplistic examples illustrate challenges addressed by interpreted master following. The exemplary instrument embodiment depicted in each of these figures comprises a localization device and a contact sensing device. Many combinations or instrument components may be utilized with an interpreted master following logic layer to provide an operator with enhanced navigation functionality, depending upon the functional objectives.

As shown in Figure 73, an instrument (530) has a distal end carrying a localization device (532) is positioned adjacent an irregular tissue wall which is represented in the system's visualization and control systems by a preferably three-dimensional tissue structure model acquired utilizing one of the aforementioned modalities. Supposing that the operator's objective is to move the instrument distal tip as indicated in Figure 73, an operator's preferred movement path depends upon his preferred action in between the two locations. For example, if the operator merely wishes to touch the instrument (530) to the tissue wall in each location without contacting any tissue in between, the operator may prefer a path of efficiency around the irregularity in the tissue structure, such as that depicted by a dashed line (531). Following this path, the operator may drive the instrument between the respective positions/locations.

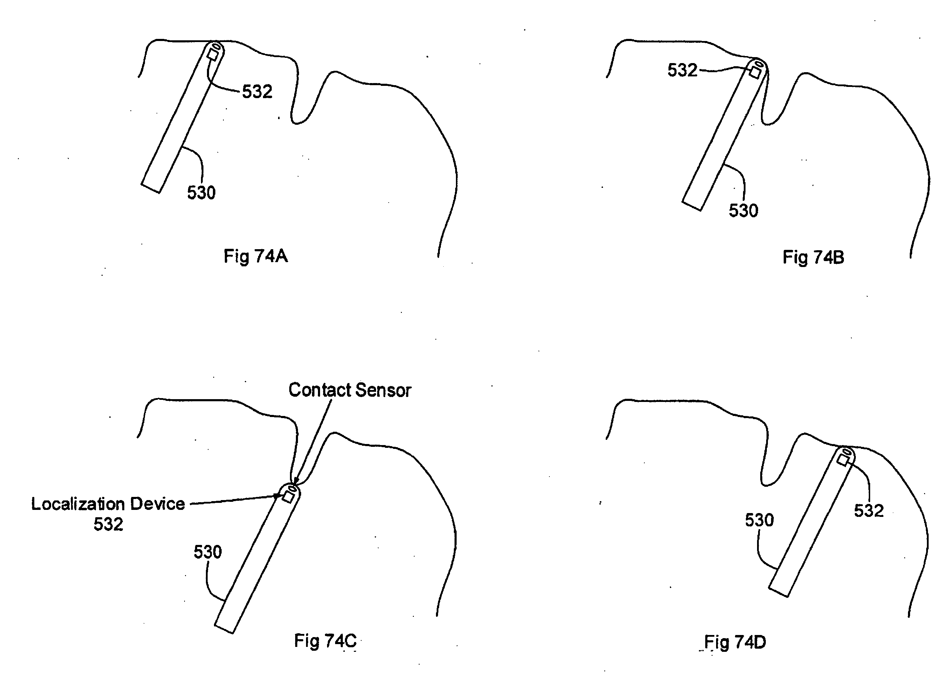

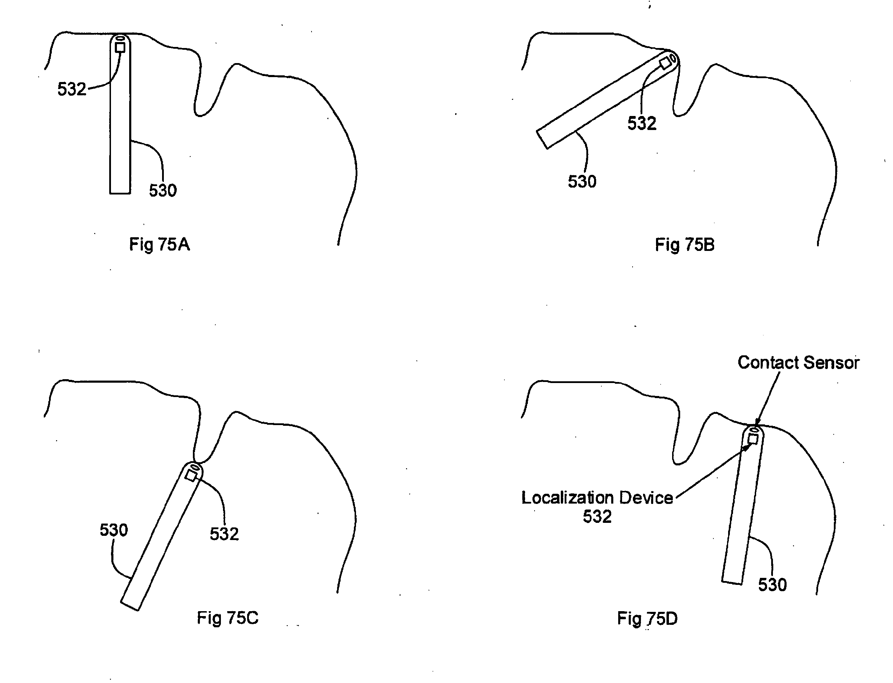

Additionally or alternately, the operator may wish to lightly touch the instrument (530) against the tissue structure and keep the instrument in contact as the instrument is driven between the locations depicted in Figure 74 via a series of hops between the two locations, rather than a constant dragging type of contact as described in the aforementioned embodiment. Further, in another embodiment, as depicted in Figure 75, the operator may wish to move the instrument between positions, while maintaining the instrument substantially normal to the tissue structure wall, perhaps due to the preferred orientation of a distal instrument feature, e.g., an electrode.

In addition, the operator may wish to have safety functionality built into the controls logic to, for example, prevent the instrument from damaging the subject tissue structures by excessively dragging along the tissue with an excessive load, overloading or overstressing a particular portion of a tissue structure with a concentrated load, or occupying a region that may cause tissue damage, such as an active valve entrance.

Such operator objectives are addressed in various embodiments of an interpreted master following logic layer interposed into the controls logic. In one embodiment, interpreted master following interprets commands that would normally lead to dragging along the tissue structure surface as commands to execute a succession of smaller "hops" to and from the tissue structure surface, while logging each contact as a new point to add to the tissue structure surface model. Hops are preferably executed by backing the instrument out the same trajectory it came into contact with the tissue structure, then moving normally along the wall per the tissue structure model, and re-approaching with a similar trajectory. In addition to saving to memory each new XYZ surface point, in one embodiment. the system saves the trajectory of the instrument with which the contact was made by saving the localization orientation data and control element tension commands to allow the operator to re-execute the same trajectory at a later time if so desired. By saving the trajectories and new points of contact confirmation, a more detailed contour map is formed from the tissue structure model, which may be utilized in the procedure and continually enhanced. The length of each hop may be configured, as well as the length of non-contact distance in between each hop contact.

In one embodiment, interpreted master following performs a variety of safety checking steps to ensure that the operator does not accidentally damage the subject tissue structure by driving into it or through it with the instrument. For example, the controls logic may be configured to disallow driving of the instrument beyond or into the subject tissue structure, as determined utilizing a tissue structure model with localization data and/or contact sensing. Such a mode may be manually overridden with an operator command in certain scenarios, for example, in order to purposefully puncture a tissue wall such as the septum at the location of the fossa ovalis. In one embodiment, the controls logic may be configured to prevent instrument electrode activation while the operator is attempting to move the instrument, or may attempt to prevent electrode activation in the same location for more than a predetermined time or amount of energy delivered.

In another embodiment, interpreted master following assists the operator in automating various clinical procedures. For example, where the instrument comprises a distal ablation electrode, the controls may be configured to automatically fit a circular ablation pattern through three contact points selected by the operator. Further, an operator may select a hopping, intermittent electrode burning pattern to automatically apply has he merely moves the master input device linearly. Haptics functionality may be utilized to provide the operator with various feedback to assist in clinical procedures. For example, a haptic "groove" may be created along the insertion axis of the instrument to assist the operator in driving the instrument with the master input device. Further, previously selected points of desired contact may be haptically turned in to "gravity wells" to assist the operator in directing the instrument to such locations.

A control system embodiment, such as described above, facilitates precision steerability of a catheter-based instrument in order to conduct a medical procedure, such as those described herein.

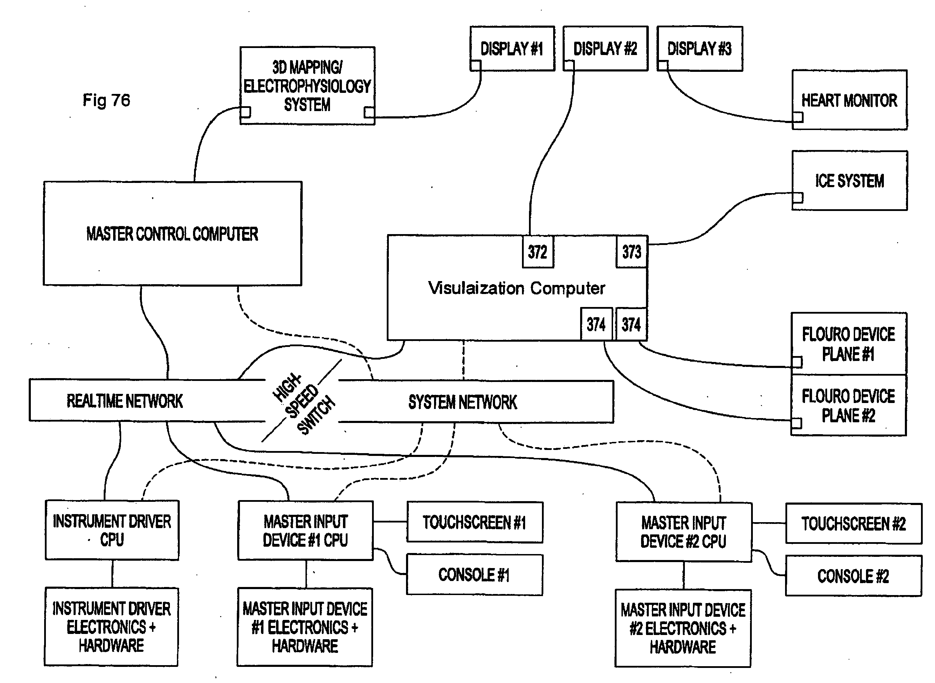

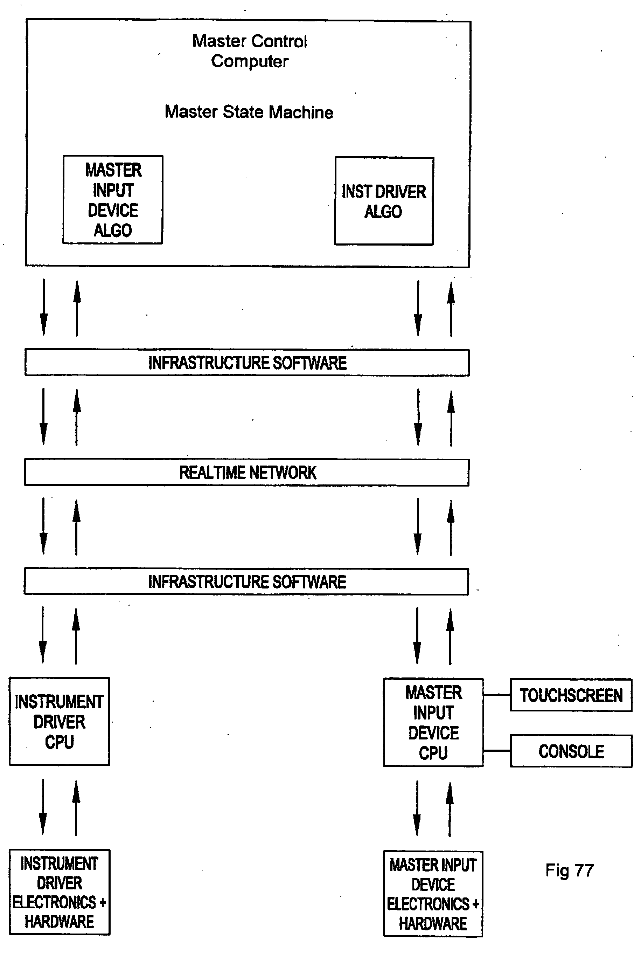

Referring to Figure 76, a distributed system architecture embodiment is depicted. A master control computer running a realtime operating system, such as QNX, is connected to each of the other computers in the system by a 1 gigabit Ethernet "Realtime Network", and also by a 100 megabit Ethernet "System Network", using a conventional high-speed switch. This enables localized custom computing for various devices to be pushed locally near the device, without the need for large cabling or a central computing machine. In one embodiment, the master control computer may be powered by an Intel Xeon dual processor architecture, the visualization computer powered by a high-end X86 Intel architecture PC running Windows XP and having multiple video cards and frame grabbers, the instrument driver and master input device CPUs being PC 104 or "EPIC" standard boards with two Ethernet connections for the two networks. An additional master input device, touchscreen, and console may be configured into an addition operator workstation in a different location relative to the patient. The system is very expandeable - new devices may be plugged into the switch and placed onto either of the two networks. Referring to Figure 76, two high resolution frame grabber boards (374) acquire images from two fluoro devices (or one in the case of single plane fluoro), which a nominal resolution frame grabber board (373) acquires images from an intracardiac echo system. Such image data may be utilized for overlaying, etc, and displayed on a display, such as the #2 display, using a video card (372) of the visualization computer, as depicted. Heart monitor data, from systems such as those distributed by Prucka, may be directly channeled from video out ports on the heart monitor device to one of the displays. Such data may also be acquired by a frame grabber. Similarly, electrophysiological mapping and treatment data and images from systems available from distributors such as Endocardial Solutions, Biosense Webster, etc, may be directed as video to a monitor, or data to a data acquisition board, databus, or frame grabber. Preferably the master control computer has some interface connectivity with the electrophysiology system as well to enable single master input device driving of such device, etc. Referring to Figure 77. a depiction of the software and hardware interaction is depicted. Essentially, the master state machine functionality of the master control system realtime operating system allows for very low latency control of processes used to operate master input device algorithms and instrument driver algorithms, such as those described in reference to the control systems description above. Indeed, XPC may be utilized to develop algorithm code, but preferably a universal modeling language such as rational rose by IBM or Rhapsody by ILogix, is utilized to build code and documentation using a graphical interface. With the gigabit realtime network, in a matter of 200-300 microseconds, the master input device or instrument driver algorithms are able to communicate with FPGA driver code in the electronics and hardware near the pertinent device to exchange new values, etc, and confirm that all is well from a safety perspective. This leaves approximately 700 microseconds for processing if a 1 millisecond motor shutoff time is required if all is not welt - and this is easily achievable with the described architecture. The visualization PC may be configured to cycle data from the master control computer at a lower frequency, about 20 milliseconds.

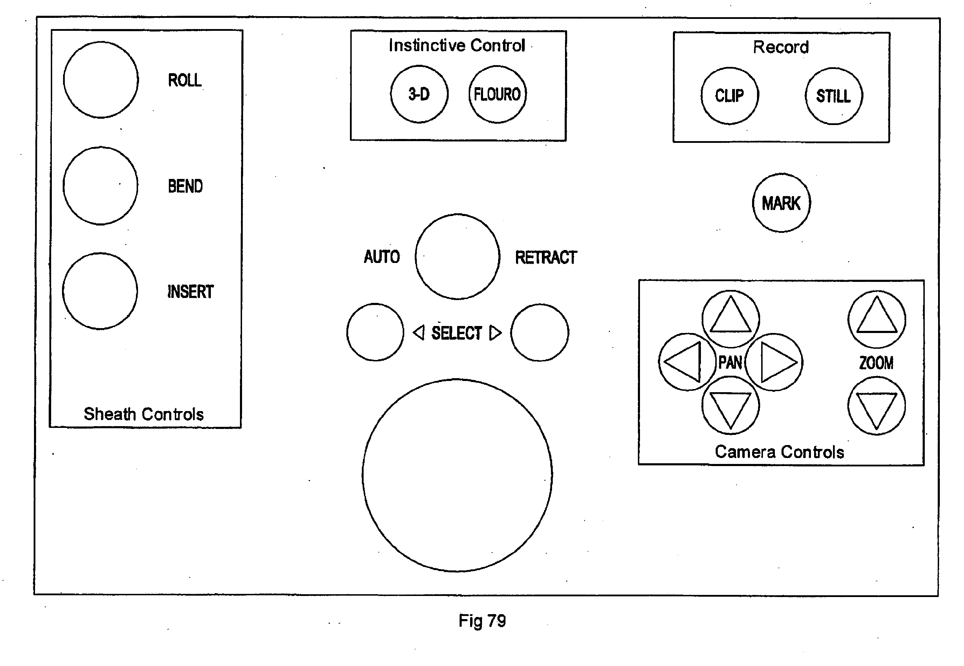

Referring to Figure 79, common features may be accessed by a console. Sheath control buttons for roll, bend, and insert, when depressed one at a time, cause the master input device to control roll of the sheath (in one embodiment, this meaning roll of the entire instrument driver) in one direction or another as directed by the master input device, +/- bending in one direction, and insertion of the sheath relative to the guide instrument. Instinctive control buttons determine whether the main display is to synchronize master input device movement with 3-D images, such as CT images, or fluoro images. An autoretract button pulls in the guide instrument to a zero insertion point along the trajectory that it was bent. A trackball and mouse select buttons may be used for some features not accessed by a touch screen interface. Record features record a digital clip of video on a selected monitor for a preset period of time, or acquire an image of the video on a selected monitor. Camera controls enable the operator to pan or zoom an image featured on a display.

## Claims

1. A robotic catheter system (32), comprising a controller (2) including a master input device (12), an instrument driver (16) in communication with the controller (2), an elongate flexible guide instrument (18) operatively coupled to the instrument driver (16), an elongate flexible member (397) extending through a lumen (716) of the guide instrument (18), the system (32) **characterized in that** it further comprises:

   a fluid injection needle (395) carried by a distal portion of the flexible member (397), wherein the needle (395) may be advanced from, or retracted into, a side of the flexible member (397); and
   an imaging device (398) carried by the flexible member (397) to provide a side-oriented field of view (403) configured to capture images of at least a portion of the needle (395) as it is advanced from the side of the flexible member (397).

2. The system of claim 1, the fluid injection needle (395) comprising an internal lumen in fluid communication with a fluid source (382), and having one or more injection ports in communication with the internal lumen.

3. The system of claim 1, comprising a plurality of fluid injection needles (385) carried on a distal end portion of the flexible member.

4. The system of claims 1, further comprising a helical anchor member (748) that may be rotatably advanced from, and retracted into, a distal end portion of the guide instrument (18).

5. The system of claim 1, further comprising one or more lateral anchor members (732, 733, 734) that may be deployed from a side of the fluid injection needle (395).

6. The system of claim 1, further comprising one or more anchor members (751) that may be deployed from a side of the guide instrument (18).

7. The system of claim 1, further comprising a source of RF energy (379) selectively electrically coupled to the fluid injection needle (395), whereby RF energy may be delivered to tissue proximate the needle (395).

8. The system of claim 1, further comprising a mechanical lever (396) configured for actuating advancement and retraction of the fluid injection needle (395).

9. The system of claim 1, wherein the imaging device (398) comprises one of an ultrasound array, CCD device, and an optical camera.

10. The system of claim 1, wherein the fluid injection needle (395) comprises an electrode.

**Patentansprüche**

1. Roboter-Kathetersystem (32), umfassend eine Steuereinheit (2), die eine Haupteingabevorrichtung (12) aufweist, einen Instrumententreiber (16) in Verbindung mit der Steuereinheit (2), ein längliches, flexibles Führungsinstrument (18), das funktionsfähig an den Instrumententreiber (16) gekoppelt ist, und ein längliches, flexibles Element (397), das sich durch ein Lumen (716) des Führungsinstruments (18) erstreckt, wobei das System (32) **dadurch gekennzeichnet ist, dass** es ferner umfasst:

   eine Flüssigkeitseinspritznadel (395), die durch einen distalen Abschnitt des flexiblen Elements (397) getragen wird, wobei die Nadel (395) aus einer Selte des flexiblen Elements (397) vorgeschoben oder in dieselbe zurückgezogen werden kann; und
   eine Bildgebungsvorrichtung (398), die durch das flexible Element (397) getragen wird, um ein seitlich orientiertes Sichtfeld (403) bereitzustellen, das so konfiguriert ist, dass es Bilder von wenigstens einem Abschnitt der Nadel (395) erfasst, wenn sie aus der Seite des flexiblen Elements (397) vorgeschoben wird.

2. System nach Anspruch 1, wobei die Flüssigkeitseinspritznadel (395) ein Innenlumen in Flüssigkeitsverbindung mit einer Flüssigkeitsquelle (382) umfasst und eine oder mehr Einspritzöffnungen in Verbindung mit dem Innenlumen aufweist.

3. System nach Anspruch 1, umfassend eine Mehrzahl von Flüssigkeitseinspritznadeln (385), die auf einem distalen Endabschnitt des flexiblen Elements getragen werden.

4. System nach Anspruch 1, ferner umfassend ein spiralförmlges Ankerelement (748), das drehbar aus einem distalen Endabschnitt des Führungsinstruments (18) vorgeschoben und in denselben zurückgezogen werden kann.

5. System nach Anspruch 1, ferner umfassend ein oder mehr laterale Ankerelemente (732, 733, 734), die aus einer Seite der Flüssigkeitseinspritznadel (395) ausgefahren werden können.

6. System nach Anspruch 1, ferner umfassend ein oder mehr Ankerelemente (751), die aus einer Seite des Führungsinstruments (18) ausgefahren werden können.

7. System nach Anspruch 1, ferner umfassend eine Quelle von HF-Energie (379), die selektiv elektrisch an die Flüsslgkeitseinspritznadel (395) gekoppelt ist, wobei die HF-Energie dem Gewebe in unmittelbarer Nähe der Nadel (395) zugeführt werden kann.

8. System nach Anspruch 1, ferner umfassend einen mechanischen Hebel (396), der so konfiguriert ist, dass er den Vorschub und den Rückzug der Flüsslgkeitseinspritznadel (395) steuert.

9. System nach Anspruch 1, wobei die Bildgebungsvorrichtung (398) eines von einem Ultraschall-Array, einer CCD-Vorrichtung und einer optischen Kamera umfasst.

10. System nach Anspruch 1, wobei die Flüssigkeitseinspritznadel (395) eine Elektrode umfasst.

**Revendications**

1. Système de cathéter robotisé (32) comprenant un contrôleur (2) comprenant un dispositif d'entrée maître (12), un dispositif d'entraînement d'instrument (16) en communication avec le contrôleur (2), un instrument de guidage souple allongé (18) couplé de manière opérationnelle au dispositif d'entraînement d'instrument (16), un élément souple allongé (397) s'étendant à travers une lumière (716) de l'instrument de guidage (18), le système (32) étant **caractérisé en ce qu'**il comprend en outre :

   une aiguille d'injection de fluide (395) supportée par une partie distale de l'élément souple (397), dans lequel l'aiguille (395) peut être avancée depuis ou rétractée dans un côté de l'élément souple (397) ; et
   un dispositif d'imagerie (398) supporté par l'élément souple (397) pour fournir un champ de vision (403) orienté vers le côté, configuré pour capturer des images d'au moins une partie de aiguille (395) lorsqu'elle est avancée depuis le côté de l'élément souple (397).

2. Système selon la revendication 1, l'aiguille d'injection de fluide (395) comprenant une lumière interne en communication de fluide avec une source de fluide (382) et ayant un ou plusieurs orifices d'injection en communication avec la lumière interne.

3. Système selon la revendication 1, comprenant une pluralité d'aiguilles d'injection de fluide (385) supportées sur une partie d'extrémité distale de l'élément souple.

4. Système selon la revendication 1, comprenant en outre un élément d'ancrage hélicoïdal (748) qui peut être avancé en rotation depuis et rétracté dans une partie d'extrémité distale de l'instrument de guidage (18).

5. Système selon la revendication 1, comprenant en outre un ou plusieurs éléments d'ancrage latéraux (732, 733, 734) qui peuvent être déployés d'un côté de l'aiguille d'injection de fluide (395).

6. Système selon la revendication 1, comprenant en outre un ou plusieurs éléments d'ancrage (751) qui peuvent être déployés d'un côté de l'instrument de guidage (18).

7. Système selon la revendication 1, comprenant en outre une source d'énergie RF (379) sélectivement couplée électriquement à l'aiguille d'injection de fluide (395), moyennant quoi l'énergie RF peut être délivrée au tissu à proximité de l'aiguille (395).

8. Système selon la revendication 1, comprenant en outre un levier mécanique (396) configuré pour actionner l'avancement et la rétraction de l'aiguille d'injection de fluide (395).

9. Système selon la revendication 1, dans lequel le dispositif d'imagerie (398) comprend un ensemble d'ultrasons, un dispositif CCD et une caméra optique.

10. Système selon la revendication 1, dans lequel l'aiguille d'injection de fluide (395) comprend une électrode.

Fig 1

Fig 2

Fig 3

Fig 4

Fig 5

EP 1 776 057 B1

Fig 6

717

716

28

30

18

717

717

716

Fig 7

2

378

18

16

6

379

RF
Energy
Control
Unit

Injection
System

382

Fig 8A

382

Injection
System

379

Energy
Control
Unit

30

18

380

381

16

30

28

18

Fig 8B

Fig 9A

383

384

Fig 9B

383

385

Fig 9C

383

386

Fig 9D

387

Fig 9E

383

388

389

Fig 9F

390

391

393

394

Fig 9H

393

392

Fig 9G

Fig 10C

Fig 10B

Fig 10A

Fig 11A

Fig 11B

Fig 11C

Fig 11D

30

Fig 12A

720

30

748

Fig 13A

30

721

Fig 12B

30

748

18

Fig 13B

718

EP 1 776 057 B1

35

Fig 14A

722

Fig 14B

723

Fig 14C

724

Fig 14D

725

Fig 15

726

18

727

18

Fig 16A

727    18

Fig 16B

728

Fig 16C

726

18

Fig 17A

729

726    726

18

Fig 17B

Fig 17C

Fig 18A

Fig 18B

Fig 18C

Fig 18D

18

718

763

731

Fig 19

Fig 20

718

732

718

Fig 21A

733

718

Fig 21B

718

736

Fig 21E

734

718

Fig 21C

718

724

Fig 21F

718

735

Fig 21D

718

Fig 22A

718

736

Fig 22B

718

737

Fig 22C

718

738

Fig 22D

Fig 23A

742

741

743

740

742

741

Fig 23B

Fig 24A

Fig 24B

Fig 24C

Fig 25

Fig 26A

Fig 26B

Fig 27A

Fig 27B

Fig 28A

Fig 28B

Fig 29A

Fig 29B

Fig 29C

Fig 29D

Fig 29G

Fig 29E

Fig 29H

Fig 29F

Fig 29I

Fig 29J

Fig 29K

Fig 29L

30

Fig 29M

18

751    750

EP 1 776 057 B1

30

Fig 29N

18

751

Fig 29O

Fig 29P

48

Fig 29Q

Fig 29R

Fig 30A

Fig 30B

Fig 30C

753

18

30

718

Fig 30D

Fig 30E

Fig 30F

Fig 30G

741

754

30

741

Fig 30H

741

749

759

741

30

Fig 30l

Fig 30K

Fig 30J

Fig 30M

Fig 30L

Fig 31A

Fig 31B

Fig 31C

Fig 31D

16

18

30

Fig 32

Fig 33

Fig 34

Fig 35

Fig 36

Fig 37

Fig 38

Fig 39A

145

143

145

141

Fig 39B

145

143

147

219

147

141

219

145

Fig 39C

143

145

145

141

Fig 39D

151

149

153

155

157

Fig 39E

147

151

149

155

155

153

159

157

Fig 39F

Fig 39G

Fig 39H

Fig 39l

Fig 39J

Fig 39K

Fig 40

Fig 41A

EP 1 776 057 B1

Fig 41B

Fig 42A

Fig 42B

308 310 312 314

Fig 43B

Fig 43B

90

Fig 43A

Fig 44B

90

Fig 44A

308

310

314

312

Fig 44B

EP 1 776 057 B1

Fig 45B

Fig 45B

Fig 45A

Fig 46A

Fig 46B

Fig 46C

Fig 46D

Fig 46E

434

Measured Instrument Position Data

400 426 422 430 424

| Master Computer | ←→ | Control & Instrument Driver Computer | ←→ | Instrument Driver Motors, Encoders, Amplifiers; Instrument position detection |

428 432

- Master input device hardware/software

- Instrument localization hardware/software

- Visualization software

- Operator control station buttons/switches

- Controls software

- Encoder boards

Fig 47

EP 1 776 057 B1

Fig 48B

Fig 48A

Fig 49

Fig 50

See Fig 53    486

490

488

measured
motor rotor rotation

required control effort:
motor torque (at rotor)

| 1 |

| MeasMotRot | MeasMotRotFiltered |

MeasMotRot

Measured Rotation Low
Pass Filter

| Position Error | Torque Command |

| 1 |

ReqMotTorq

Slave Compensation

486

| 2 |

| DesMotRot | DesMotRotFiltered |

DesMotRot

Command Low
Pass Filter

See Fig 52

desired motor
rotor rotation

Fig 51

See Fig 54

Fig 52

Fig 53

486

MeasMotRotFiltered

MeasMotRot

$$\frac{200*2*pi}{s+200*2*pi}$$

Fig 54

See Fig 59

458

DesMotRot

PitchYawExtRoll
Roll
MeasMotRot
enable signal
Tension
DesMotRot

See Fig 58

456

Enable

MeasMotRot

pitch-yaw-exten
pitch-yaw-exten-roll
roll
Slave Velocity Limiter

See Fig 57

454

pitch-yaw-exten
pitch-yaw-exten-new
RollCorrection
Catheter Roll Corrector

Roll

See Fig 60

448

End-Effector
Position Control
Pos_Localization   PitchYawExt
PitchYawExt          KinStatus

452

PitchYawExt   PitchYawExtLimited
CatheterWorkspaceLimits

Catheter Roll
Correction in degrees

Fig 55

See Fig 56

446

Master
MasterIn
Localization

Master   Localization
Localization   Localization
Xdes

450

Xdes   PitchYawExt
InverseKinematics

PitchYawExt

Fig 56

EP 1 776 057 B1

pitch-yaw-exten new

pitch-yaw-exten

RollCorrection

$\dfrac{0.5}{s+0.5}$

-K-

deg2rad

sin

cos

Fig 57

Fig 58

Fig 59

Fig 60

**Forward Kinematics:**

Actuated Inputs

| - Pitch control wire motion |
| - Yaw control wire motion |
| - Insert motion |

→ Forward Kinematics →

End-Point Position

| X-Y-Z Cartesian Coordinates |

Actuator Forward Kinematics → Basic Forward Kinematics

**Inverse Kinematics:**

End-Point Position

| X-Y-Z Cartesian Coordinates |

→ Inverse Kinematics →

Actuated Inputs

| - Pitch control wire motion |
| - Yaw control wire motion |
| - Insert motion |

Basic Inverse Kinematics → Actuator Inverse Kinematics

Fig 61

Fig 62

Fig 63

```
┌─────────────┐      ┌─────────────┐      ┌─────────────┐
│ Haptic Master│      │             │      │  Control and │
│ Input Device │ ───▶ │Master Computer│ ──▶ │Instrument Driver│
│             │      │             │      │  Computer    │
└─────────────┘      └─────────────┘      └─────────────┘
                            ┆
                            ┆
                     ╭──────────────────╮
                     │ • Master input device│
                     │   hardware/software │
                     ╰──────────────────╯
```

Fig 64

```
              345      346     347    348          349   350    351    352         353        354
        344    │        │       │      │            │     │      │      │           │          │
         │     ▼        ▼       ▼      ▼            ▼     ▼      ▼      ▼           ▼          ▼
         │  ┌────────┐      ┌─────┐     ┌──────────┐   ┌───────┐    ┌──────────┐      ──▶
    ──▶  │  │Transform to│ ──▶ │scale│ ──▶│ Catheter │──▶│Multiply│──▶│Inverse Transform│ ──▶ Fdes
   ──▶ Xp   │guide      │ Xc  │     │ Xs │Workspace │deltaX│by K to │Fdes│back to Master │     Master
         │  │catheter    │     └─────┘    │  Check   │   │get Force│    │Coordinates│
         │  │coordinate system│           └──────────┘   └───────┘    └──────────┘
         │  └────────┘
                                    │
                                    │         422
                                    │          │
                                    ▼          ▼
                              ┌──────────────┐
                              │  Control and  │
                              │Instrument Driver│         Fig 65
                              │   Computer    │
                              └──────────────┘
```

Fig 65

Fig 66.

**MASTER-VISUALIZATION SYSTEM**

OPERATOR HAND MOTION → MASTER INPUT PHYSICAL DEVICE

355 — MASTER FORWARD KINEMATICS

$q_{master}$

$\tau_{master}$

MASTER JACOBIAN
$\tau_{master} = J_{master}^{T} \, F_{master}$

356 — $X_{master}$

357 — VISUALIZATION AND CONTROL TRANSFORMS

358

$F_{master}$

HAPTIC WORKSPACE BOUNDARY

HAPTIC NAVIGATION CUES

360

$X_{catheter-desired}$

359

**INSTRUMENT-INSTRUMENT DRIVER SYSTEM**

361 — CATHETER INVERSE KINEMATICS

362 / 363 — $q_{desired}$

SYSTEM INTERFACE UI COMMANDS

CONTROL PARAMETERS

$\Delta q$ + − CATHETER POSITION CONTROL SYSTEM

FEED-FORWARD DESIRED POSITION

$q_{compensation}$ +

INVERSE KINEMATICS

$q$

$X_{measured}$

CATHETER END-POINT POSITION SENSOR

**CATHETER END-POINT POSITION CONTROL LOOP**

**DRIVER POSITION CONTROL LOOP**

$\Delta q$ + − DRIVER CONTROL SYSTEM

$\tau_{desired}$

DRIVER CATHETER DYNAMIC SYSTEM

$q_{Driver}$

$X_{Catheter}$

CATHETER MOTION

(Tensioning)

(Actuation)

Fig 67

$\Delta_1$

$\Delta_2$

364

$$\Delta_1 = r_a \; \theta_a - r_T \; \theta_T$$

Fig 68

$$\Delta_2 = r_a \; \theta_a - r_T \; \theta_T$$

365

Actuation $\quad \emptyset_a = \dfrac{(\Delta_1 - \Delta_2)}{\Delta_c} \quad$ [Radians] $\quad\nearrow^{366}$

Tension $\quad \delta_T = \Delta_1 + \Delta_2 \quad$ [mm]

$\nwarrow_{367}$

Fig 69

$$\emptyset_a = \left(\dfrac{2 \cdot r_a}{\Delta_c}\right) \theta_a \qquad \begin{array}{c}\text{Desired} \\ \text{Actuation}\end{array} \nearrow^{368}$$

$$\delta_T = (-2\ r_T)\ \theta_T \qquad \begin{array}{c}\text{Desired} \\ \text{Tensioning}\end{array}$$

$\nwarrow_{369}$

Fig 70

Desired Tension - 1 DOF:

$$\delta_T = K_T \left\| \varnothing_a \right\|$$

Fig 71

Desired Tension - 2 DOF (i.e., pitch & yaw):

$$\begin{pmatrix} \delta_{T_{Pitch}} \\ \delta_{T_{Yaw}} \end{pmatrix} = \begin{bmatrix} K_T & K_{TC} \\ K_{TC} & K_T \end{bmatrix} \cdot \begin{pmatrix} \varnothing_{a_{Pitch}} \\ \varnothing_{a_{Yaw}} \end{pmatrix}$$

Tension coupling     Tension slope

370

Fig 72

532

530

Fig 73B

532

530

Fig 73D

531

532

530

Fig 73A

Contact Sensor

Localization Device
532

530

Fig 73C

Fig 74B

532

530

Fig 74D

532

530

Fig 74A

532

530

Contact Sensor

Localization Device
532

530

Fig 74C

Fig 75A

Fig 75B

Fig 75C

Fig 75D

Fig 76

3D MAPPING/ ELECTROPHYSIOLOGY SYSTEM

DISPLAY #1  DISPLAY #2  DISPLAY #3

HEART MONITOR

ICE SYSTEM

MASTER CONTROL COMPUTER

Visulaization Computer

372    373

374    374

FLOURO DEVICE PLANE #1

FLOURO DEVICE PLANE #2

REALTIME NETWORK

HIGH-SPEED SWITCH

SYSTEM NETWORK

INSTRUMENT DRIVER CPU

MASTER INPUT DEVICE #1 CPU

TOUCHSCREEN #1

CONSOLE #1

MASTER INPUT DEVICE #2 CPU

TOUCHSCREEN #2

CONSOLE #2

INSTRUMENT DRIVER ELECTRONICS + HARDWARE

MASTER INPUT DEVICE #1 ELECTRONICS + HARDWARE

MASTER INPUT DEVICE #2 ELECTRONICS + HARDWARE

Master Control
Computer

Master State Machine

MASTER
INPUT
DEVICE
ALGO

INST DRIVER
ALGO

INFRASTRUCTURE SOFTWARE

REALTIME NETWORK

INFRASTRUCTURE SOFTWARE

INSTRUMENT
DRIVER
CPU

MASTER
INPUT
DEVICE
CPU

TOUCHSCREEN

CONSOLE

INSTRUMENT
DRIVER
ELECTRONICS +
HARDWARE

MASTER INPUT
DEVICE
ELECTRONICS +
HARDWARE

Fig 77

## Master Control Computer

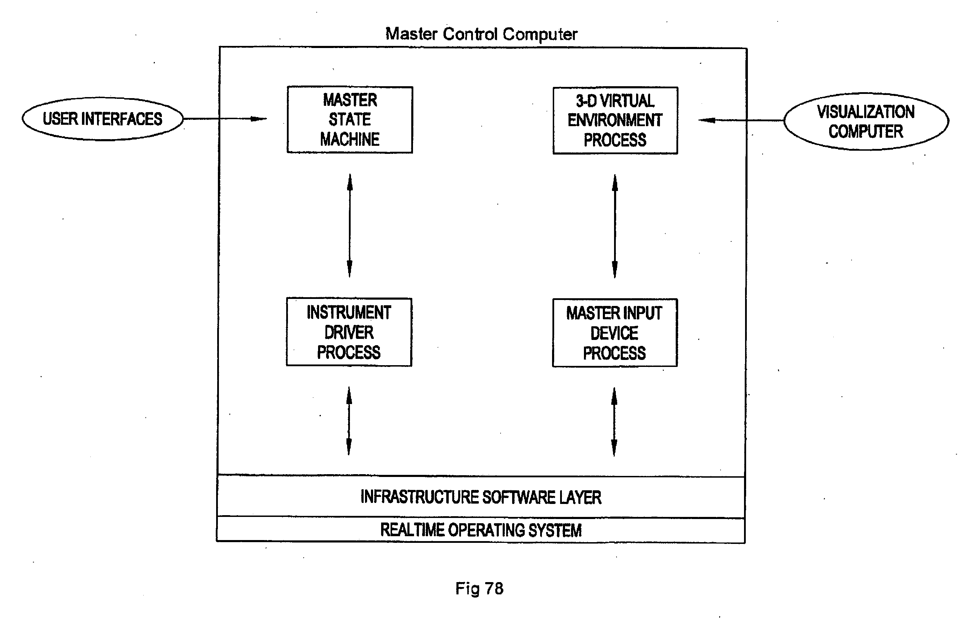

Fig 78

EP 1 776 057 B1

**Sheath Controls**
- ROLL
- BEND
- INSERT

**Instinctive Control**
- 3-D
- FLOURO

**Record**
- CLIP
- STILL

MARK

AUTO — RETRACT

◁ SELECT ▷

**Camera Controls**
- PAN
- ZOOM

Fig 79

EP 1 776 057 B1

**EP 1 776 057 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02065933 A